(19) Europäisches Patentamt
European Patent Office
Office européen des brevets

(11) EP 1 627 635 B1

(12) **EUROPEAN PATENT SPECIFICATION**

(45) Date of publication and mention of the grant of the patent:
**07.10.2009 Bulletin 2009/41**

(51) Int Cl.:
***A61K 31/428*** $^{(2006.01)}$ ***A61P 31/12*** $^{(2006.01)}$
***C07D 277/62*** $^{(2006.01)}$

(21) Application number: **05291733.3**

(22) Date of filing: **16.08.2005**

(54) **Benzothiazolium compounds for use in methods of inhibiting NO production and TNF alpha and treating coronaviral infection**

Benzothiazolium Verbindungen zur Verwendung in Methoden zur Hemmung der NO Produktion und von TNF alpha und zur Behandlung von Coronavirus Infektionen

Composés de benzothiazolium utilisés dans des méthodes pour l'inhibition de la production de NO et du TNF alpha et pour le traitement des infections à coronavirus

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **13.08.2004 US 601390 P**

(43) Date of publication of application:
**22.02.2006 Bulletin 2006/08**

(73) Proprietor: **National Health Research Institutes**
**Zhunan Town, Miaoli Country (TW)**

(72) Inventors:
- **Lee, Shiow-Ju**
**Tainan (TW)**
- **Yang, Yung-Ning**
**Sindian City, Taipei (TW)**

(74) Representative: **Nargolwalla, Cyra**
**Cabinet Plasseraud**
**52, rue de la Victoire**
**75440 Paris Cedex 09 (FR)**

(56) References cited:
**WO-A-01/58897**

- **CHEN H-F ET AL: "DISCOVERY OF ANTI-SARS CORONAVIRUS DRUG BASED ON MOLECULAR DOCKING AND DATABASE SCREENING" CHINESE JOURNAL OF CHEMISTRY, vol. 22, no. 8, 2004, pages 882-887, XP008035036**
- **NG PAK C ET AL: "Inflammatory cytokine profile in children with severe acute respiratory syndrome." PEDIATRICS. JAN 2004, vol. 113, no. 1 Pt 1, January 2004 (2004-01), pages e7-14, XP002364277 ISSN: 1098-4275**
- **TSENG H-Y ET AL: "Benzothiazolium compounds: novel classes of inhibitors that suppress the nitric oxide production in RAW264.7 cells stimulated by LPS/IFNgamma" BIOORGANIC & MEDICINAL CHEMISTRY LETTERS, OXFORD, GB, vol. 15, no. 8, 15 April 2005 (2005-04-15), pages 2027-2032, XP004829167 ISSN: 0960-894X**
- **BLANCHARD J E ET AL: "High-Throughput Screening Identifies Inhibitors of the SARS Coronavirus Main Proteinase" CHEMISTRY AND BIOLOGY, CURRENT BIOLOGY, LONDON, GB, vol. 11, no. 10, October 2004 (2004-10), pages 1445-1453, XP004601878 ISSN: 1074-5521**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 627 635 B1

## Description

### BACKGROUND

Nitric oxide (NO) is an important pleiotropic molecule mediating a wide range of physiological and pathophysiological processes. Overproduction of NO has been implicated in various pathological processes including septic shock, virus infection, tissue damage following inflammation, cancer, atherosclerosis, and arthritis.

NO is produced from L-arginine and molecular oxygen by three distincf isoforms of nitric oxide synthase (NOS), i.e., neural NOS (nNOS), endothelial NOS (eNOS), and inducible NOS (iNOS). Among the three NOSs, iNOS can be induced by endotoxins or cytokines (e.g., TNF $\alpha$ and IL-6) to produce a high level of NO. Inhibiting expression or activity of iNOS is a major task of preventing and eliminating NO overproduction.

CHEN H-F ET AL: "DISCOVERY OF ANTI-SARS CORONAVIRUS DRUG BASED ON MOLECULAR DOCKING AND DATABASE SCREENING" CHINESE JOURNAL OF CHEMISTRY, vol. 22, no. 8, 2004, pages 882-887, describes a strategy of anti-SARS drug design based on molecular docking and database screening.

NG PAK C ET AL: "Inflammatory cytokine profile in children with severe acute respiratory syndrome." PEDIATRICS. JAN 2004, vol. 113, no. 1 Pt 1, January 2004 (2004-01), pages e7-14, studies the inflammatory cytokine profile in children with severe acute respiratory syndrome (SARS) and investigates whether monoclonal antibody to tumor necrosis factor-alpha (TNF-alpha) could be considered for treatment of these patients.

WO0158897 discloses benzothiazoles as TNF and PDE-IV inhibitors.

### SUMMARY

This invention is based on a discovery that a group of benzothiazolium compounds, unexpectedly, suppress NO production and possess inhibitory activities against coronavirus.

One aspect of this invention relates to a compound of the following formula:

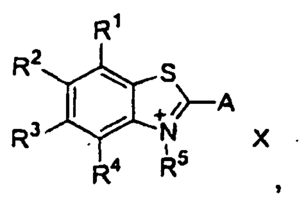,

1

wherein each of $R^1$, $R^2$, $R^3$, and $R^4$, independently, is H, halo, alkyl, alkenyl, akynyl, alkoxyl, aryl, heteroaryl, cylcyl, heterocyclyl, nitro, cyano, amino, amido, or alkoxycarbonyl, or $R^1$, $R^2$, and the two carbon atoms to which they are attached, together form a 3-8 membered ring containing 0, 1, 2, or 3 heteroatoms, or $R^2$, $R^3$, and the two carbon atoms to which they are attached, together form a 3-8 membered ring containing 0, 1, 2, or 3 heteroatoms, or $R^3$, $R^4$, and the two carbon atoms to which they are attached, together form a 3-8 membered ring containing 0, 1, 2, or 3 heteroatoms; $R^5$ is alkyl, aryl, heteroaryl, cyclyl, or heterocyclyl; A is H, alkyl, aryl, heteroaryl, cyclyl, heterocyclyl, alkoxy, amino, halo, mercapto, thioalkoxy,

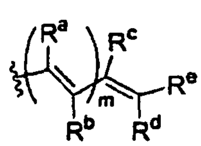, or 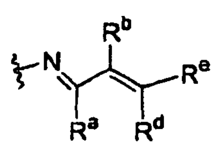;

in which m is 0, 1, 2, or 3; each of $R^a$, $R^b$, $R^c$, $R^d$, and $R^e$ independently is H, alkyl, akenyl, akynyl, halo, aryl, heteroaryl, cyclyl, heterocylyl, alkoxy, mercapto, thioalkoxy, amino, amido, or $R^d$, $R^c$, and the carbon atom to which they are attached, together form a 3-8 membered ring containing 0, 1, 2, or 3 heteroatoms, which is optionally fused with aryl, heteroaryl, cyclyl, or heterocyclyl; optionally A, $R^5$, the N atom to which $R^5$ is attached, and the carbon atom to which A is attached, together form a 4-8 membered ring containing 1, 2, or 3 heteroatoms; and X is an anion or a negatively charged group attached to an atom of $R^1$, $R^2$, $R^3$, $R^4$, $R^5$, or A for use in treating coronaviral infection.

Referring to the above formula, one subset of the compounds feature that X is $I^-$, $Br^-$, $Cl^-$, $ClO_4^-$, $C_2H_5SO_4^-$, or

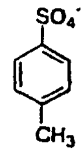;

or a negatively charged group attached to an atom of $R^1$, $R^2$, $R^3$, $R^4$, $R^5$, or A, the negatively charged group being selected from - $CO_2^-$, $-SO_3^-$, or $-PO_3^{2-}$.

Another subset of the compounds feature that A is alkyl or $R^5$ is alkyl.

Still another subset of the compounds feature that A is

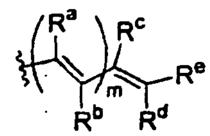

and $R^5$ is alkyl. In some of these compounds, one of $R^d$ and $R^c$ is substituted or unsubstituted phenyl, thioalkoxy, amino, or amido. In some other compounds, $R^d$, $R^c$, and the carbon atom to which they are attached, together form a 3-8 membered ring containing 0, 1, or 2 heteroatoms. As an example, A can be

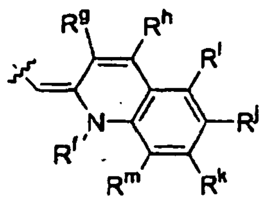,

$R^f$ being H, alkyl, alkenyl, aryl, cyclyl, heteroaryl, or heterocyclyl; and each of $R^g$, $R^h$, $R^i$, $R^j$, $R^k$, and $R^m$, independently being H, alkyl, alkenyl, aryl, cyclyl, heteroaryl, heterocyclyl, alkoxyl, halogen, nitro, or cyano. As another example, A can be

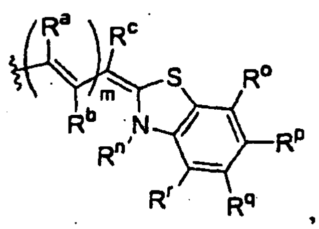,

$R^n$ being H, alkyl, alkenyl, aryl, cyclyl, heteroaryl, heterocyclyl, and each of $R^o$, $R^p$, $R^q$, and $R^r$, independently, being H, alkyl, alkenyl, aryl, cyclyl, heteroaryl, heterocyclyl, alkoxyl, nitro, amino, cyano, or halogen.

Shown in Table 1 are exemplary compounds:

Table 1. Benzothiazolium compounds

| Compound Number | Structure |
|---|---|
| 1 | 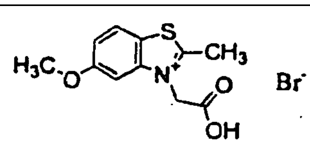 |

(continued)

| Compound Number | Structure |
|---|---|
| 2 | 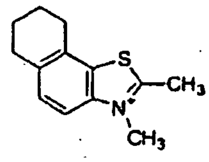 $p$-$CH_3C_6H_4SO_3^-$ |
| 3 | 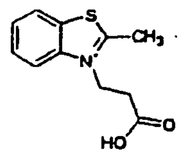 $Br^-$ |
| 4 | 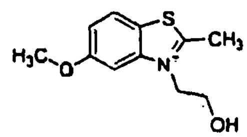 $I^-$ |
| 5 | 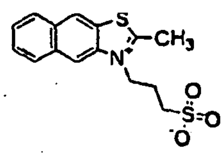 |
| 6 | 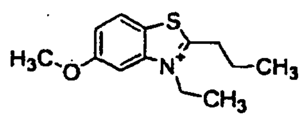 $I^-$ |
| 7 | 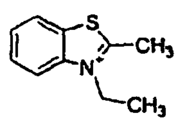 $p$-$CH_3C_6H_4SO_3^-$ |
| 8 | 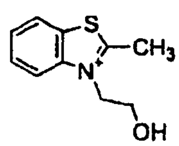 $Cl^-$ |
| 9 | 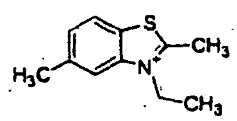 $I^-$ |
| 10 | 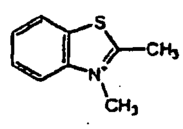 $I^-$ |
| 11 | 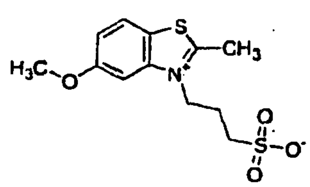 |

(continued)

| Compound Number | Structure |
|---|---|
| 12 | 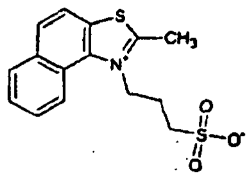 |
| 13 | 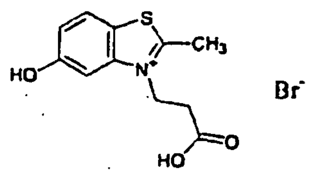 |
| 14 | 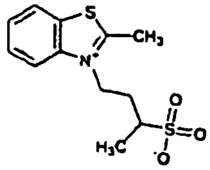 |
| 15 | 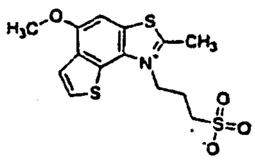 |
| 16 | 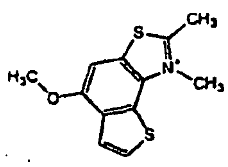 |
| 17 | 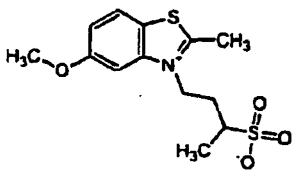 |
| 18 | 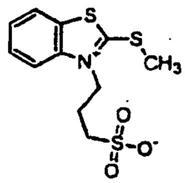 |

(continued)

| Compound Number | Structure |
|---|---|
| 19 | 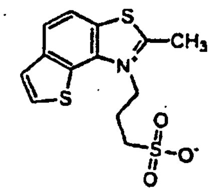 |
| 20 | 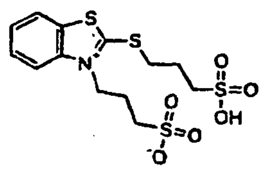 |
| 21 | 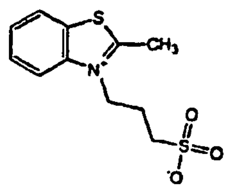 |
| 22 | 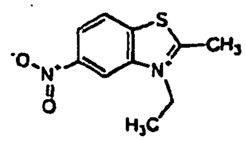 $I^-$ |
| 23 | 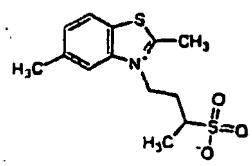 |
| 24 | 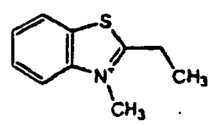 $I^-$ |
| 25 | 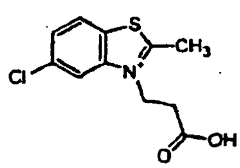 $p$-$CH_3C_6H_4SO_3^-$ |
| 26 | 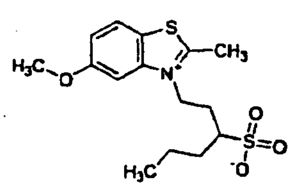 |

(continued)

| Compound Number | Structure |
|---|---|
| 27 | 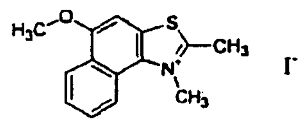 |
| 28 | 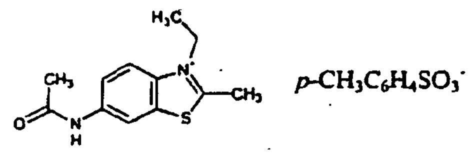 |
| 29 | 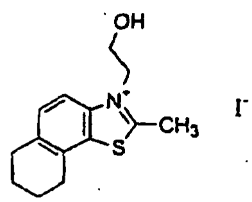 |
| 32 | 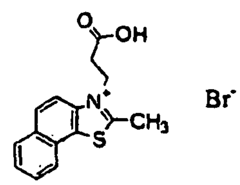 |
| 33 | 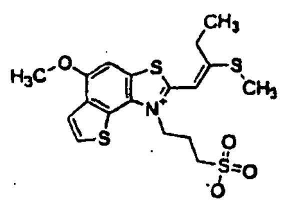 |
| 34 | 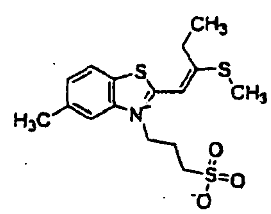 |
| 35 | 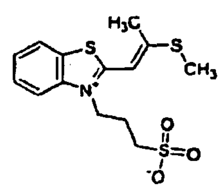 |
| 36 | 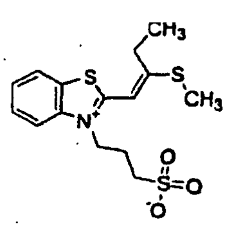 |

(continued)

| Compound Number | Structure |
|---|---|
| 37 | 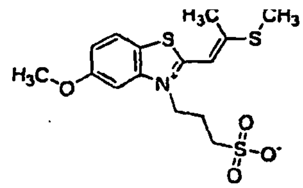 |
| 38 | 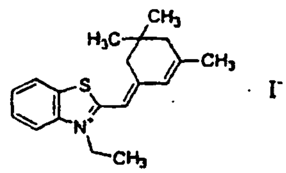 |
| 39 | 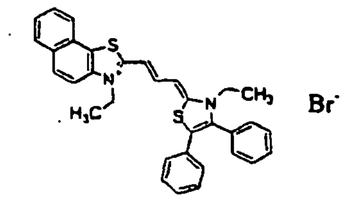 |
| 40 | 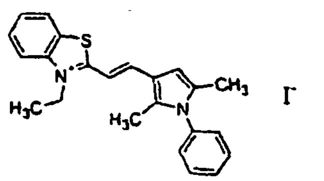 |
| 41 | 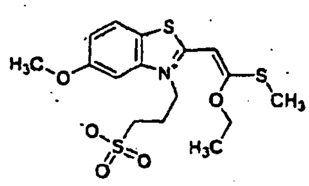 |
| 42 | 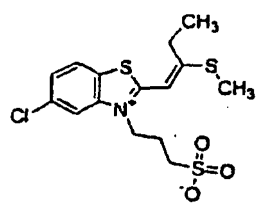 |
| 43 | 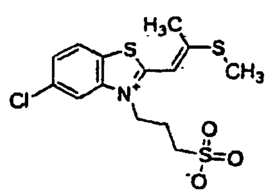 |
| 44 | 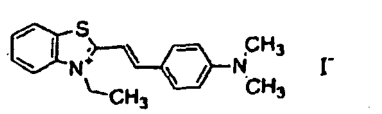 |

(continued)

| Compound Number | Structure |
|---|---|
| 45 | 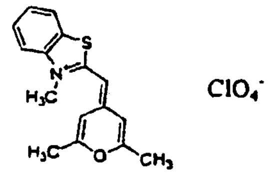 |
| 46 | 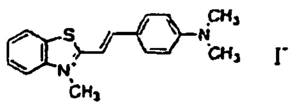 |
| 47 | 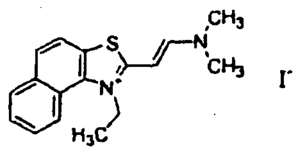 |
| 48 | 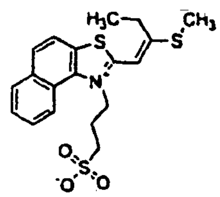 |
| 49 | 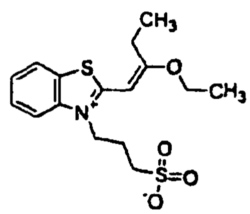 |
| 50 | 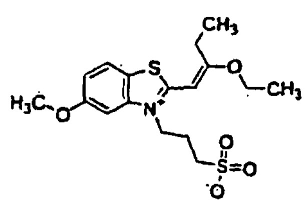 |
| 51 | 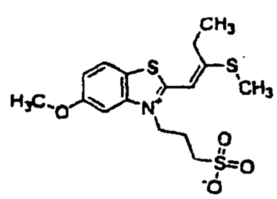 |
| 52 | 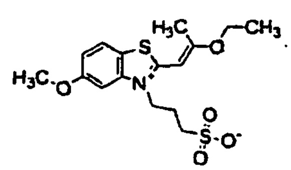 |

(continued)

| Compound Number | Structure |
|---|---|
| 53 | 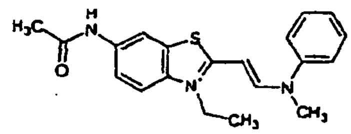 $I^-$ |
| 54 | 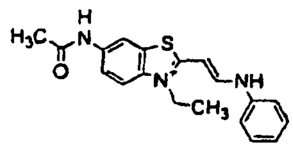 $ClO_4^-$ |
| 55 | 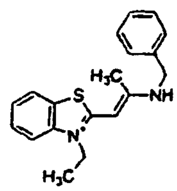 $I^-$ |
| 56 | 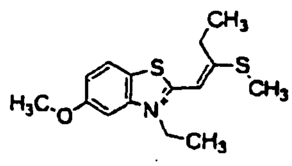 $CH_3SO_4^-$ |
| 57 | 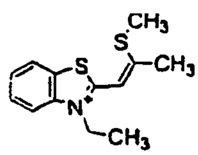 $CH_3SO_4^-$ |
| 58 | 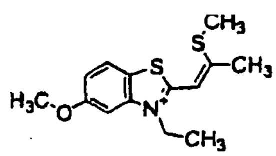 $CH_3SO_4^-$ |
| 59 | 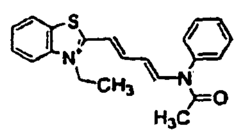 $I^-$ |
| 60 | 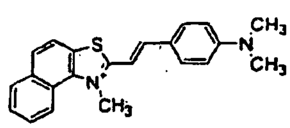 $p$-$CH_3C_6H_4SO_3^-$ |
| 61 | 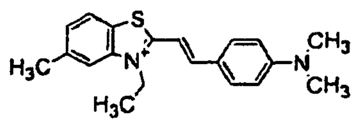 $I^-$ |

(continued)

| Compound Number | Structure |
|---|---|
| 62 | 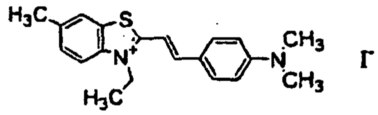 |
| 63 | 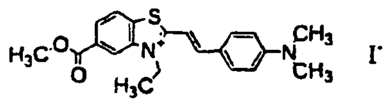 |
| 64 | 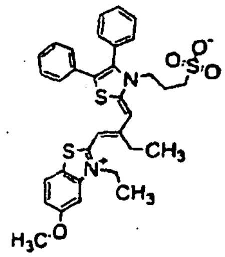 |
| 65 | 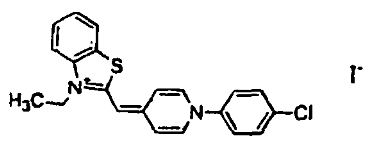 |
| 66 | 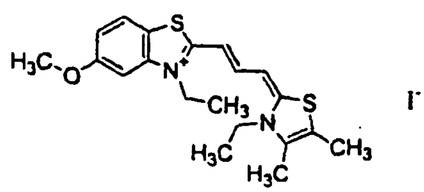 |
| 67 | 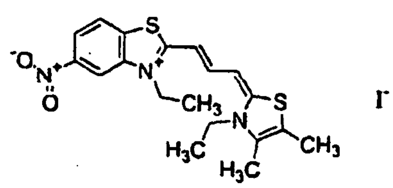 |
| 68 | 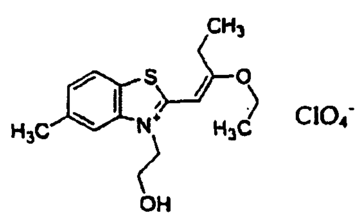 |
| 69 | 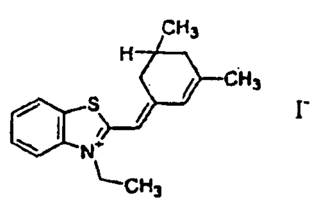 |

(continued)

| Compound Number | Structure |
|---|---|
| 70 | 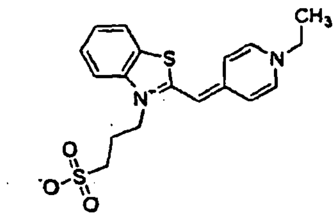 $I^-$ |
| 71 | 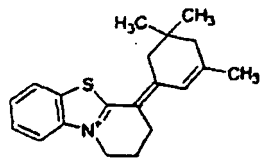 $I^-$ |
| 72 | 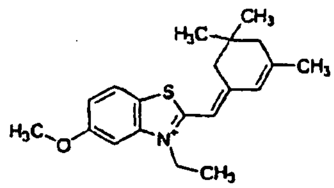 $p$-$CH_3C_6H_4SO_3^-$ |
| 73 | 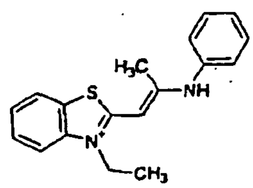 $I^-$ |
| 74 | 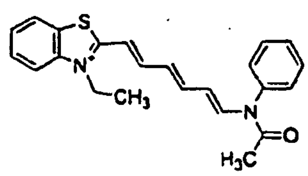 $I^-$ |
| 75 | 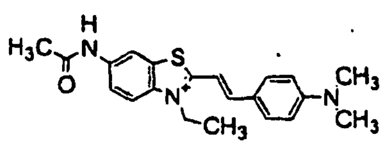 $I^-$ |
| 76 | 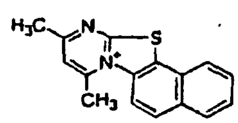 $ClO_4^-$ |
| 77 | 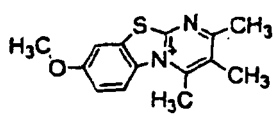 $ClO_4^-$ |
| 78 | 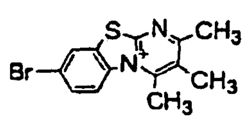 $ClO_4^-$ |

(continued)

| Compound Number | Structure |
|---|---|
| 79 | 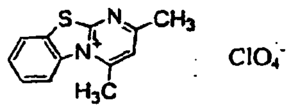 |
| 80 | 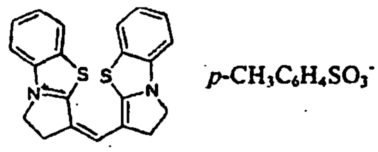 |
| 81 | 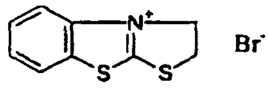 |
| 82 | 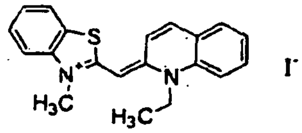 |
| 83 | 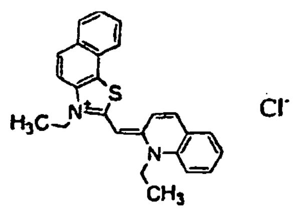 |
| 84 | 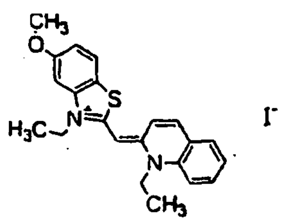 |
| 85 | 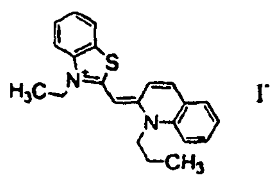 |
| 86 | 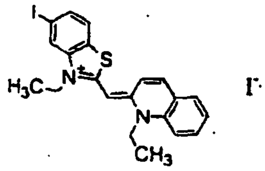 |

(continued)

| Compound Number | Structure |
|---|---|
| 87 | 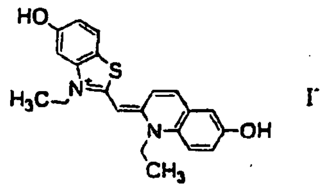 |
| 88 | 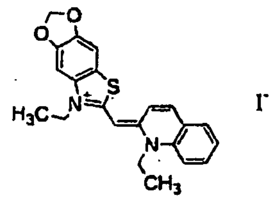 |
| 89 | 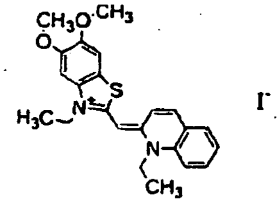 |
| 90 | 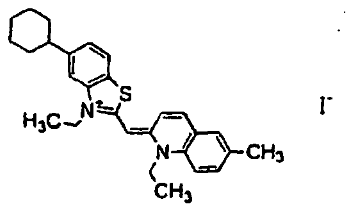 |
| 91 | 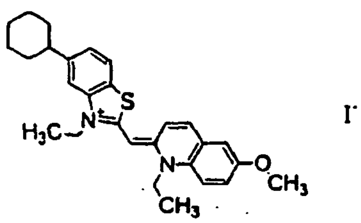 |
| 92 | 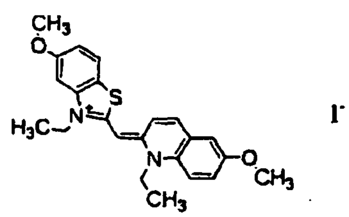 |
| 93 | 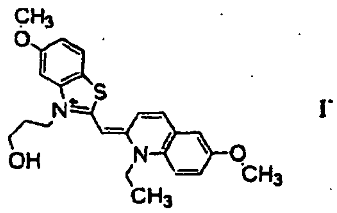 |

(continued)

| Compound Number | Structure |
|---|---|
| 94 | 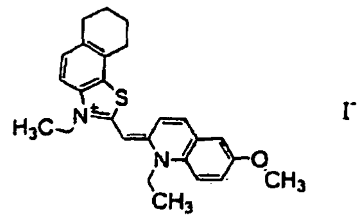 |
| 95 | 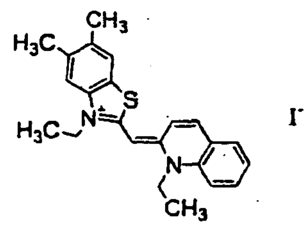 |
| 96 | 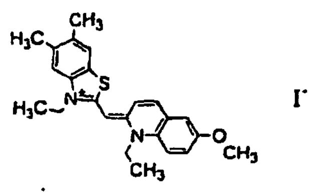 |
| 97 | 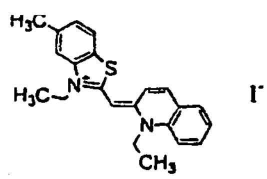 |
| 98 | 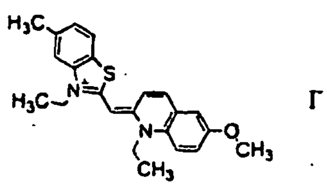 |
| 99 | 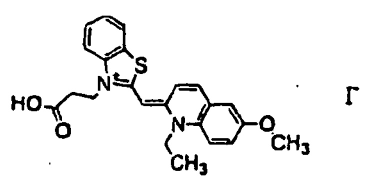 |
| 100 | 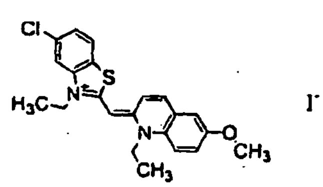 |

(continued)

| Compound Number | Structure |
|---|---|
| 101 | 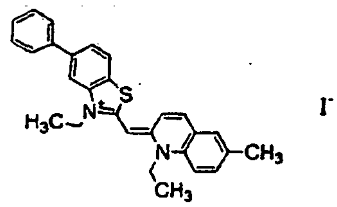 |
| 102 | 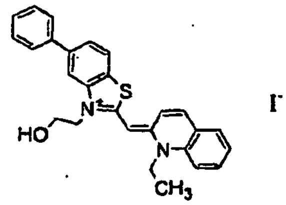 |
| 103 | 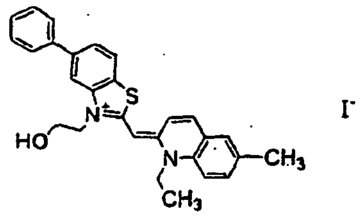 |
| 104 | 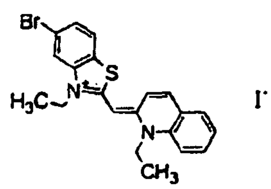 |
| 105 | 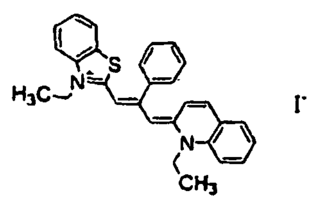 |
| 106 | 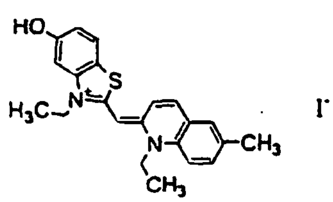 |
| 107 | 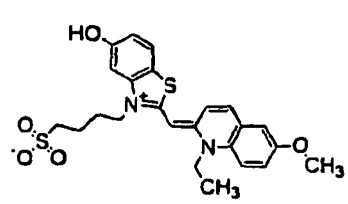 |

(continued)

| Compound Number | Structure |
|---|---|
| 108 | 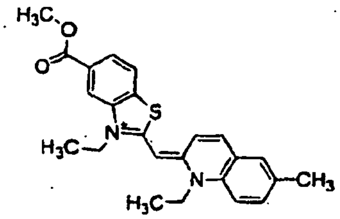 $Br^-$ |
| 109 | 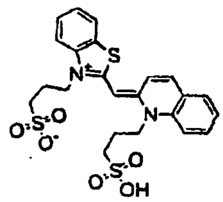 |
| 110 | 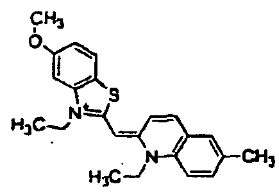 $p$-$CH_3C_6H_4SO_3^-$ |
| 111 | 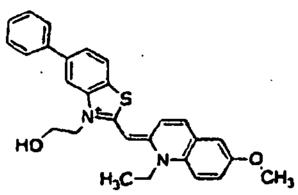 $I^-$ |
| 112 | 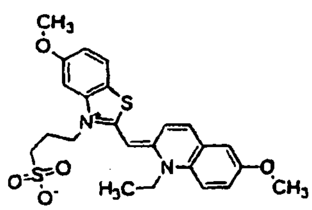 |
| 113 | 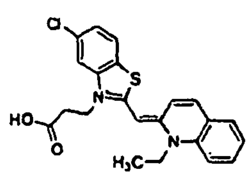 $Cl^-$ |
| 114 | 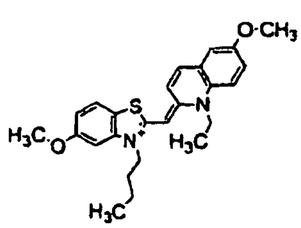 $I^-$ |

(continued)

| Compound Number | Structure |
|---|---|
| 115 | 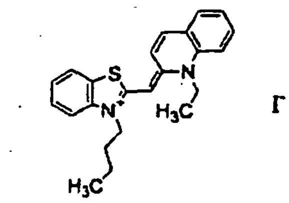 |
| 116 | 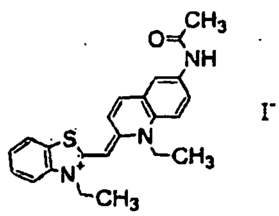 |
| 117 | 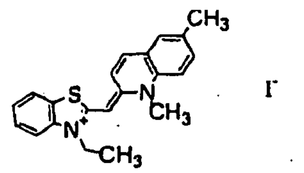 |
| 118 | 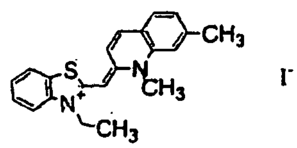 |
| 119 | 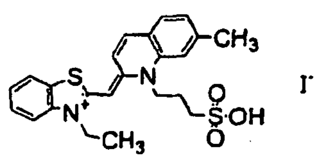 |
| 120 | 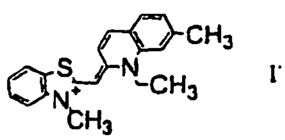 |
| 121 | 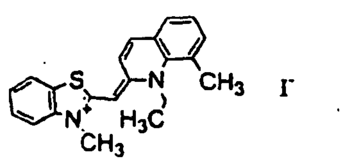 |
| 122 | 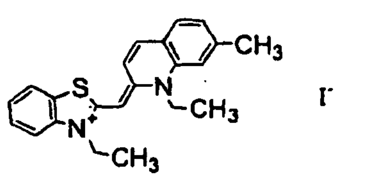 |

(continued)

| Compound Number | Structure |
| --- | --- |
| 123 | 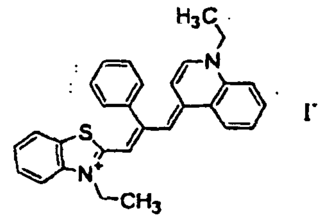 |
| 124 | 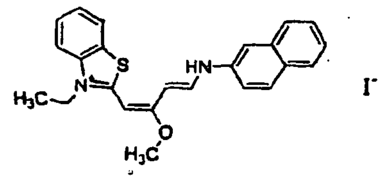 |
| 125 | 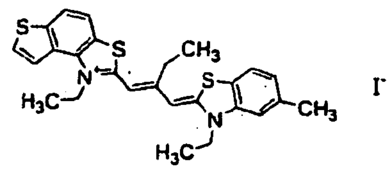 |
| 126 | 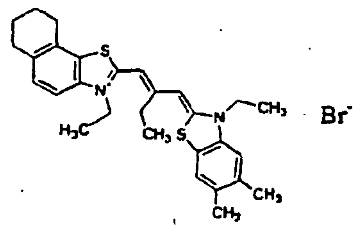 |
| 127 | 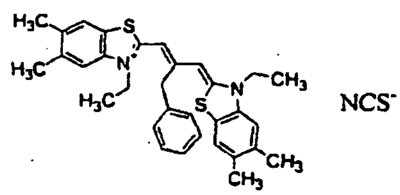 |
| 128 | 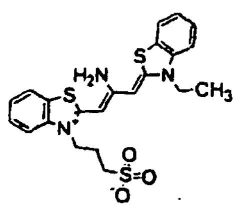 |
| 129 | 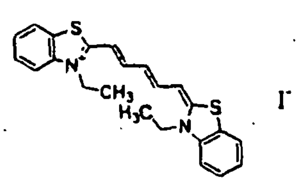 |

(continued)

| Compound Number | Structure |
|---|---|
| 130 | 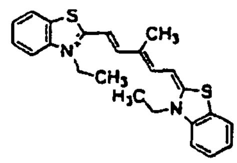 $I^-$ |
| 131 | 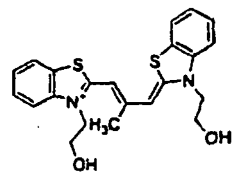 $Cl^-$ |
| 132 | 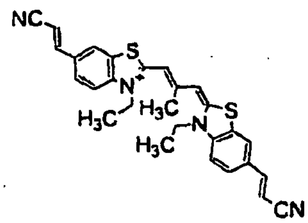 $p$-$CH_3C_6H_4SO_3^-$ |
| 133 | 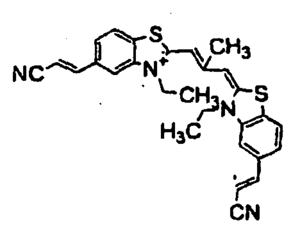 $p$-$CH_3C_6H_4SO_3^-$ |
| 134 | 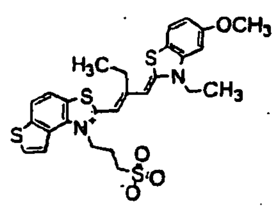 |
| 135 | 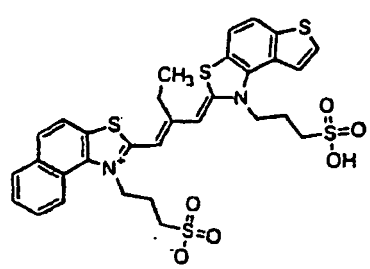 |

(continued)

| Compound Number | Structure |
|---|---|
| 136 | 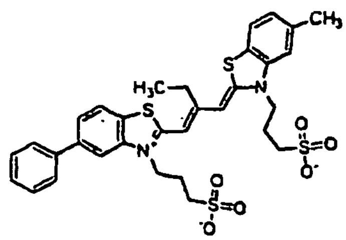 $(C_4H_9)_3NH^+$ |
| 137 | 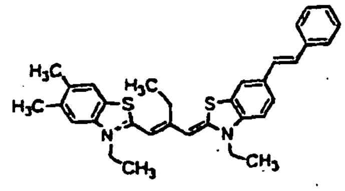 $I^-$ |
| 138 | 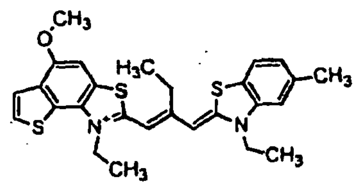 $Br^-$ |
| 139 | 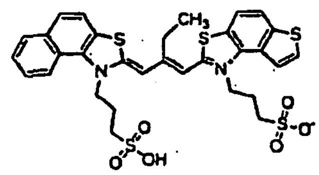 |
| 140 | 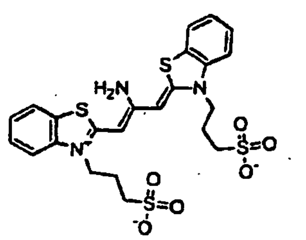 $NH_4^+$ |
| 141 | 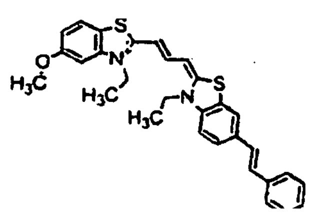 $I^-$ |
| 142 | 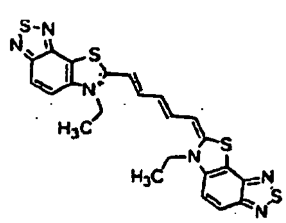 $p$-$CH_3C_6H_4SO_3^-$ |

(continued)

| Compound Number | Structure |
|---|---|
| 143 | 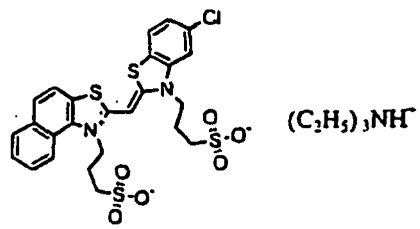 |
| 144 | 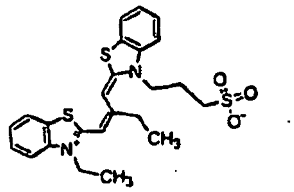 |
| 145 | 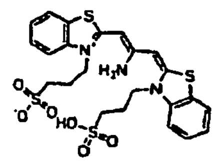 |
| 146 | 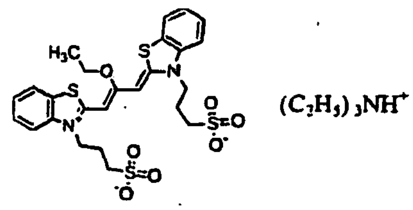 |
| 147 | 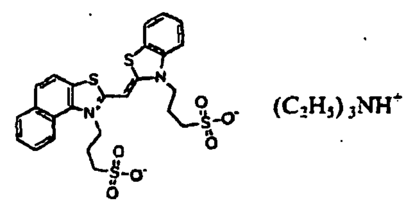 |
| 148 | 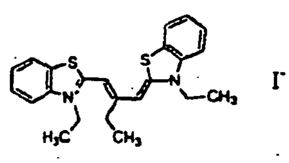 |
| 149 | 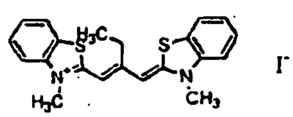 |

(continued)

| Compound Number | Structure |
|---|---|
| 150 | 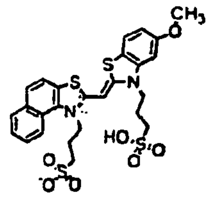 |
| 151 | 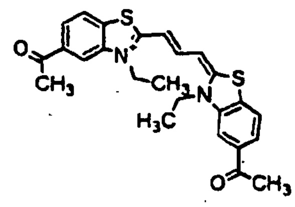 $C_2H_5SO_4^-$ |
| 152 | 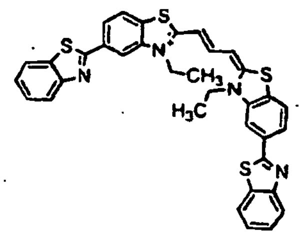 $p\text{-}CH_3C_6H_4SO_3^-$ |
| 153 | 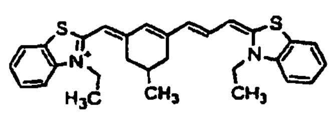 $I^-$ |
| 154 | 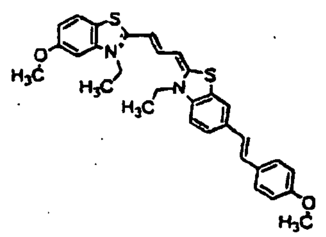 $I^-$ |
| 155 | 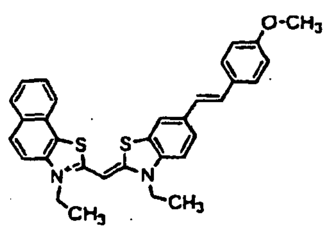 $p\text{-}CH_3C_6H_4SO_3^-$ |
| 156 | 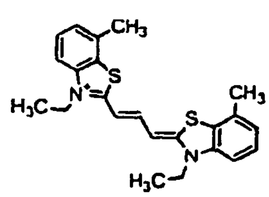 $I^-$ |

(continued)

| Compound Number | Structure |
|---|---|
| 157 | 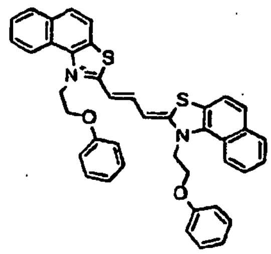 $p$-$CH_3C_6H_4SO_3^-$ |
| 158 | 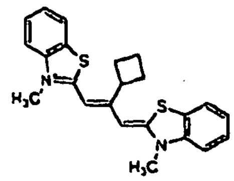 $I^-$ |
| 159 | 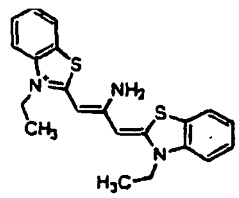 $I^-$ |
| 160 | 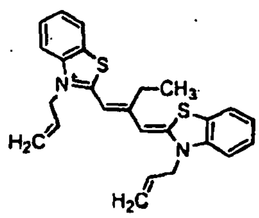 $I^-$ |
| 161 | 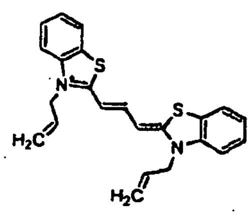 $I^-$ |
| 162 | 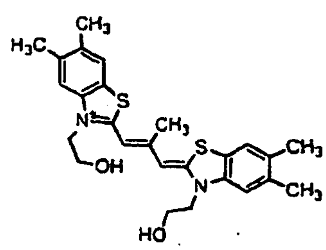 $I^-$ |

(continued)

| Compound Number | Structure |
|---|---|
| 163 | 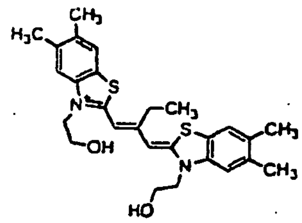 $I^-$ |
| 164 | 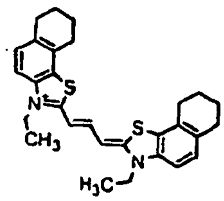 $I^-$ |
| 165 | 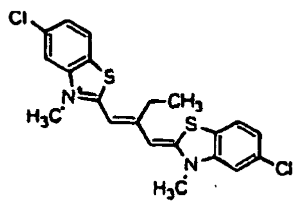 $p\text{-}CH_3C_6H_4SO_3^-$ |
| 166 | 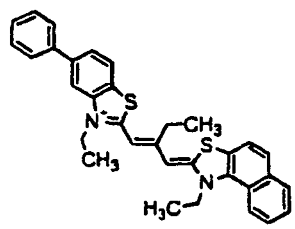 $Br^-$ |
| 167 | 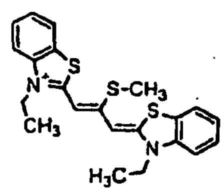 $CH_3SO_4^-$ |
| 168 | 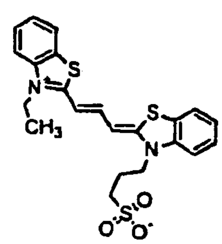 |

(continued)

| Compound Number | Structure |
|---|---|
| 169 | 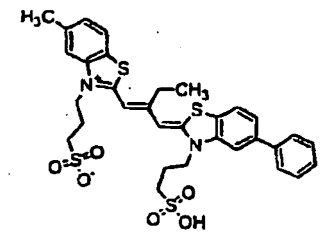 |
| 170 | 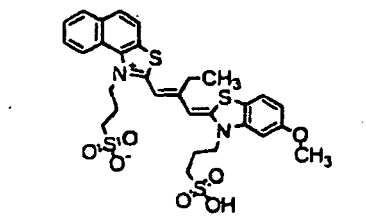 |
| 171 | 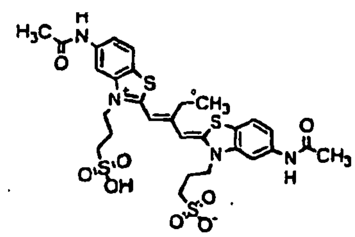 |
| 172 | 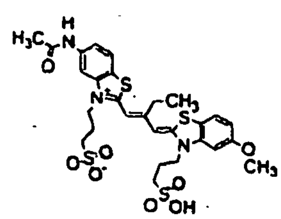 |
| 173 | 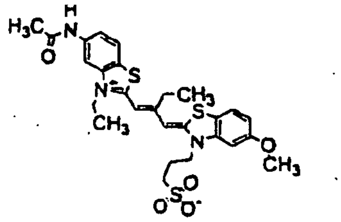 |
| 174 | 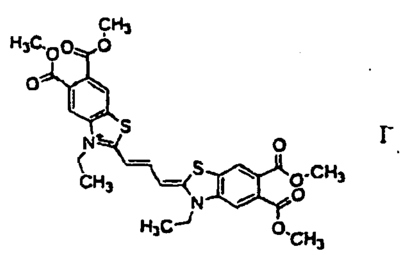 |

(continued)

| Compound Number | Structure |
|---|---|
| 175 | 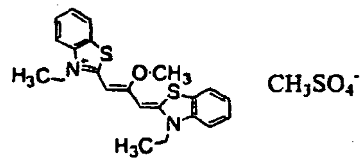 |
| 176 | 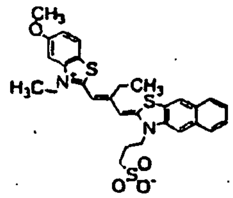 |
| 177 | 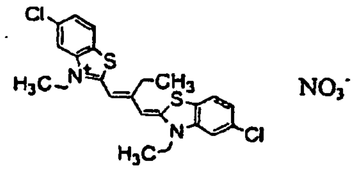 |
| 178 | 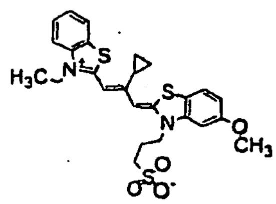 |
| 179 | 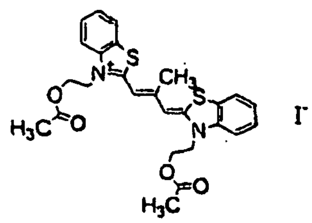 |
| 180 | 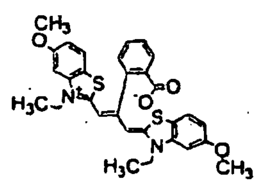 |
| 181 | 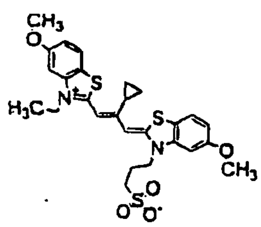 |

(continued)

| Compound Number | Structure |
|---|---|
| 182 | 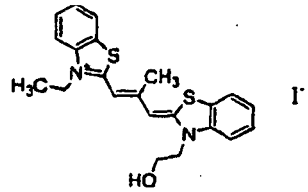 |
| 183 | 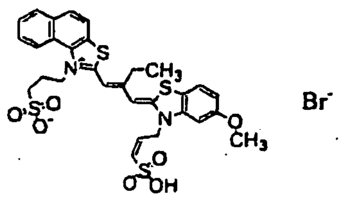 |
| 184 | 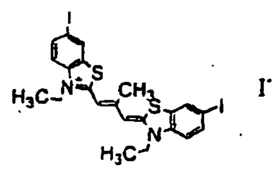 |
| 185 | 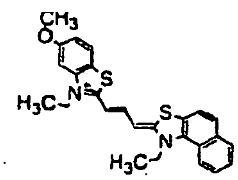 |
| 186 | 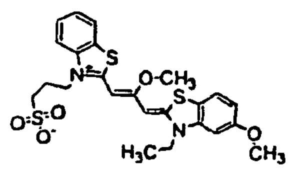 |
| 187 | 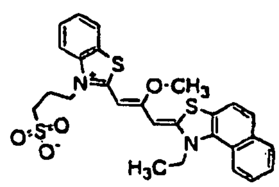 |
| 188 | 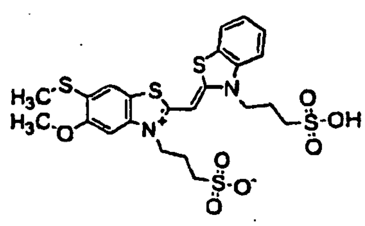 |

(continued)

| Compound Number | Structure |
|---|---|
| 189 | 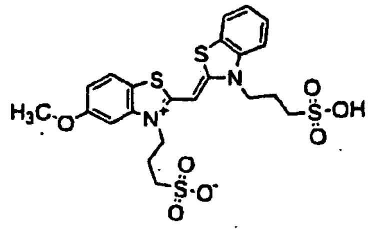 |
| 190 | 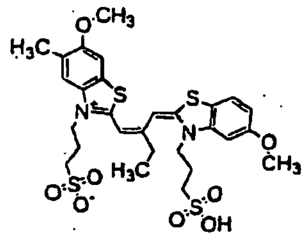 |
| 191 | 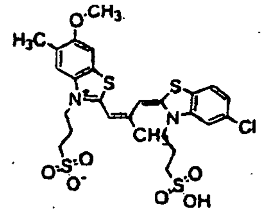 |
| 192 | 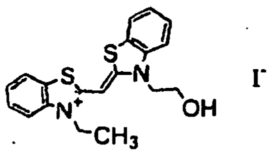 |
| 193 | 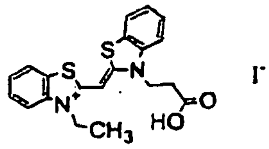 |
| 194 | 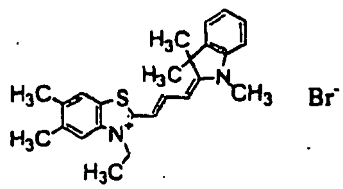 |
| 195 | 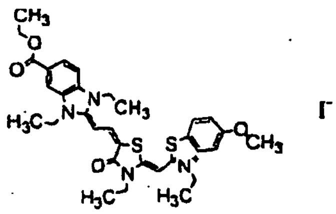 |

(continued)

| Compound Number | Structure |
|---|---|
| 196 | 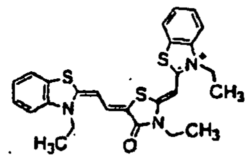 $I^-$ |
| 197 | 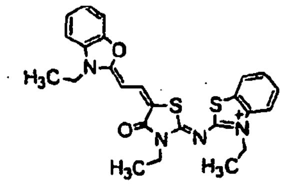 $p$-$CH_3C_6H_4SO_3^-$ |
| 198 | 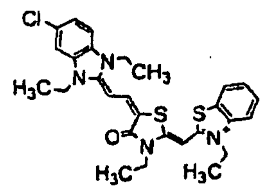 $I^-$ |
| 199 | 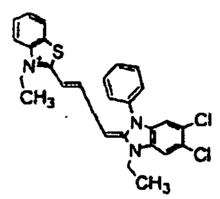 $I^-$ |
| 200 | 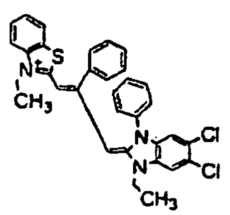 $I^-$ |
| 201 | 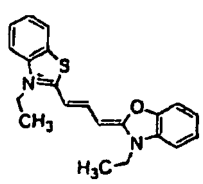 $I^-$ |
| 202 | 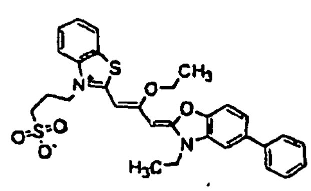 |

(continued)

| Compound Number | Structure |
|---|---|
| 204 | 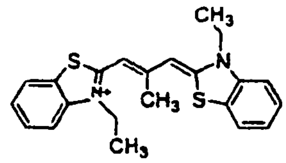 $I^-$ |
| 205 | 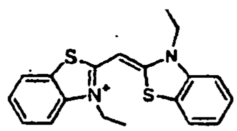 $I^-$ |
| 206 | 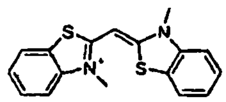 $p\text{-}CH_3C_6H_4SO_3^-$ |

The term "alkyl" refers to a straight or branched hydrocarbon, containing 1-10 carbon atoms. Examples of alkyl groups include, but are not limited to, methyl, ethyl, n-propyl, i-propyl, n-butyl, i-butyl, and t-butyl. The term "alkenyl" referes to a straight or branched hydrocarbon, containing 1-10 carbon atoms and one or more double bonds. The term "alkynyl" referes to a straight or branched hydrocarbon, containing 1-10 carbon atoms and one or more triple bonds. The term "alkoxy" refers to an -O-alkyl. The term "alkoxyalkyl" refers to an alkyl group substituted with one or more alkoxy groups. The term "haloalkyl" refers to an alkyl group substituted with one or more halo groups. The term "hydroxyalkyl" refers to an alkyl group substituted with one or more hydroxy groups.

The term "aryl" refers to a 6-carbon monocyclic, 10-carbon bicyclic, 14-carbon tricyclic aromatic ring system wherein each ring may have 1 to 4 substituents. Examples of aryl groups include, but are not limited to, phenyl, naphthyl, and anthracenyl. The term "aryloxy" refers to an -O-aryl. The term "aralkyl" refers to an alkyl group substituted with an aryl group.

The term "cyclyl" refers to a saturated and partially unsaturated cyclic hydrocarbon group having 3 to 12 carbons. Examples of cyclyl groups include, but are not limited to, cyclopropyl, cyclobutyl, cyclopentyl, cyclopentenyl, cyclohexyl, cyclohexenyl, cycloheptyl, and cyclooctyl.

The term "heteroaryl" refers to an aromatic 5-8 membered monocyclic, 8-12 membered bicyclic, or 11-14 membered tricyclic ring system having one or more heteroatoms (such as O, N, or S). Examples of heteroaryl groups include pyridyl, furyl, imidazolyl, benzimidazolyl, pyrimidinyl, thienyl, quinolinyl, indolyl, and thiazolyl. The term "heteroaralkyl" refers to an alkyl group substituted with a heteroaryl group.

The term "heterocyclyl" refers to a nonaromatic 5-8 membered monocyclic, 8-12 membered bicyclic, or 11-14 membered tricyclic ring system having one or more heteroatoms (such as O, N, or S). Examples of heterocyclyl groups include, but are not limited to, piperazinyl, pyrrolidinyl, dioxanyl, morpholinyl, and tetrahydrofuranyl.

The term "negatively charged group" refers to a functional group having one or more negative charges. Examples of negatively charged groups include, but are not limited to, $-CO_2^-$ or $-SO_3^-$, and $-PO_3^{2-}$.

Alkyl, alkenyl, alkynyl, cyclyl, heterocyclyl, aryl, heteroaryl, aralkyl, heteroaralkyl, alkoxy, and aryloxy mentioned herein include both substituted and unsubstituted moieties. Examples of substituents include, but are not limited to, halo, hydroxyl, amino, cyano, nitro, mercapto, alkoxycarbonyl, amido, carboxy, alkanesulfonyl, alkylcarbonyl, carbamido, carbamyl, carboxyl, thioureido, thiocyanato, sulfonamido, alkyl, alkenyl, alkynyl, alkyloxy, aryl, heteroaryl, cyclyl, heterocyclyl, in which alkyl, alkenyl, alkynyl, alkyloxy, aryl, heteroaryl, cyclyl, and heterocyclyl are optionally further substituted with alkyl, aryl, heteroaryl, halogen, hydroxyl, amino, mercapto, cyano, or nitro.

The compounds described above include their pharmaceutically acceptable salts and prodrugs, if applicable. Such a salt can be formed between a negatively charged ionic group in a benzothiazolium compound (e.g., carboxylate) and a positively charged counterion (e.g., sodium, potassium, calcium, or magnesium). Likewise, a positively charged ionic group in a benzothiazolium compound (e.g., ammonium) can also form a salt with a negatively charged counterion (e.g., chloride, bromide, or iodide). Examples of prodrugs include esters and other pharmaceutically acceptable compounds, which, upon administration to a subject, are each capable of providing one of the benzothiazolium compounds described above..

Another aspect of this invention relates to a method of inhibiting production of nitric oxide, by administering to

a subject in need thereof an effective amount of one of the compounds described above.

Still another aspect of this invention relates to a method of treating coronaviral infection by administering to a subject in need thereof an effective amount of one of the compounds described above.

Within the scope of this invention is one or more of the benzothiazolium compounds described above for use in treating coronaviral infection, as well as the use of such a compound for the manufacture of a medicament for treating coronaviral infection.

The details of many embodiments of the invention are set forth in the description below. Other features, objects, and advantages of the invention will be apparent from the description and the claims.

## DETAILED DESCRIPTION

The above-described benzothiazolium compounds are commercially available. They can also be prepared by methods well known in the art. For example, benzothiazole is first substituted with an alkyl, alkenyl, or aryl group by a conventional reaction, such as a substitution reaction, or a coupling reaction. The substituted benzothiazole compound reacts with an alkylating agent, e.g., methyl iodide, or methyl *p*-toluenesulfonate, to afford an alkylated benzothiazolium compound.

The methods described above may also additionally include steps, either before or after the steps described specifically herein, to add or remove suitable protecting groups in order to ultimately allow synthesis of the benzothiazolium compounds. In addition, various synthetic steps may be performed in an alternate sequence to give the desired compounds. Synthetic chemistry transformations and protecting group methodologies (protection and deprotection) useful in synthesizing applicable benzothiazolium compounds are known in the art and include, for example, those described in R. Larock, Comprehensive Organic Transformations, VCH Publishers (1989); T.W. Greene and P.G.M. Wuts, Protective Groups in Organic Synthesis, 3rd Ed., John Wiley and Sons (1999); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

A benzothiazolium compound thus obtained can be further purified by column chromatography, high performance liquid chromatography, or crystallization.

The above described benzothiazolium compounds may contain a non-aromatic double bond and one or more asymmetric centers. Thus, they can occur as racemic mixtures, single enantiomers, individual diastereomers, diastereomeric mixtures, and cis- or trans- isomeric forms. All such isomeric forms are contemplated.

The benzothiazolium compounds suppressing NO production. Thus, this invention relates to methods of suppressing NO production by administering to a subject in need thereof an effective amount of one of the above-described compounds. The term "an effective amount" refers to the amount of the compound which is required to confer one of the above-described effects in the subject. Effective amounts may vary, as recognized by those skilled in the art, depending on route of administration, excipient usage, and the possibility of co-usage with other agents.

Also within the context of this invention is a method of inhibiting expression of iNOS or lowering production of TNF $\alpha$ and IL-6. The method includes administering to a subject in need of inhibiting NO production an effective amount of one of the benzothiazolium compounds described above.

This invention also relates to a method for treating coronavirus infection. The method includes administering to a subject in need thereof an effective amount of one of the benzothiazolium compounds described above and a pharmaceutically acceptable carrier. The term "coronavirus" is well known in the art. It refers to a genus of pleomorphic viruses that resemble coronas when viewed with a microscope. Examples of coronavirus include, but are not limited to, human CoV 229E, transmissible gastroenteritis virus (TGEV), mouse hepatitis virus, bovine CoV, infectious bronchitis virus, and severe acute respiratory syndrome virus. The term "treating" refers to administering the extract to a subject that is infected with coronavirus, or has a symptom of the infection, or has a predisposition toward the infection, with the purpose to cure, heal, alleviate, relieve, alter, remedy, ameliorate, improve, or affect the infection, the symptoms of the infection, or the predisposition toward the infection.

To practice the method of the present disclosure, a composition having one of the benzothiazolium compounds describe above can be administered parenterally, orally, nasally, rectally, topically, or buccally. The term "parenteral" as used herein refers to subcutaneous, intracutaneous, intravenous, intramuscular, intraarticular, intraarterial, intrasynovial, intrasternal, intrathecal, intralesional, or intracranial injection, as well as any suitable infusion technique.

A sterile injectable composition can be a solution or suspension in a non-toxic parenterally acceptable diluent or solvent, such as a solution in 1,3-butanediol. Among the acceptable vehicles and solvents that can be employed are mannitol and water. In addition, fixed oils are conventionally employed as a solvent or suspending medium (e.g., synthetic mono- or diglycerides). Fatty acid, such as oleic acid and its glyceride derivatives are useful in the preparation of injectables, as are natural pharmaceutically acceptable oils, such as olive oil or castor oil, especially in their polyoxyethylated versions. These oil solutions or suspensions can also contain a long chain alcohol diluent or dispersant, carboxymethyl cellulose, or similar dispersing agents. Other commonly used surfactants such as Tweens or Spans or

other similar emulsifying agents or bioavailability enhancers which are commonly used in the manufacture of pharmaceutically acceptable solid, liquid, or other dosage forms can also be used for the purpose of formulation.

A composition for oral administration can be any orally acceptable dosage form including capsules, tablets, emulsions and aqueous suspensions, dispersions, and solutions. In the case of tablets, commonly used carriers include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

A nasal aerosol or inhalation composition can be prepared according to techniques well known in the art of pharmaceutical formulation. For example, such a composition can be prepared as a solution in saline, employing benzyl alcohol or other suitable preservatives, absorption promoters to enhance bioavailability, fluorocarbons, and/or other solubilizing or dispersing agents known in the art. A composition having an active benzothiazolium compounds can also be administered in the form of suppositories for rectal administration.

The carrier in the pharmaceutical composition must be "acceptable" in the sense that it is compatible with the active ingredient of the composition (and preferably, capable of stabilizing the active ingredient) and not deleterious to the subject to be treated. One or more solubilizing agents can be utilized as pharmaceutical excipients for delivery of an active benzothiazolium compound. Examples of other carriers include colloidal silicon oxide, magnesium stearate, cellulose, sodium lauryl sulfate, and D&C Yellow # 10.

The benzothiazolium compounds described above can be preliminarily screened by an *in vitro* assay for their activity, e.g., inhibiting NO production. Compounds that demonstrate high activity in the preliminary screening can further be screened for their efficacy in treating coronaviral infection by *in vivo* assays. For example, a test compound can be administered to an animal (e.g., a mouse model) affected with coronavirus and its therapeutic effects are then accessed. Based on the results, an appropriate dosage range and administration route can also be determined.

The specific examples below are to be construed as merely illustrative, and not limitative of the remainder of the disclosure in any way whatsoever. Without further elaboration, it is believed that one skilled in the art can, based on the description herein, utilize the present invention to its fullest extent.

PREPARATION OF BENZOTHIAZOLIUM COMPOUNDS

Compounds 1-204 were purchased from Chemical Diversity Labs, Inc. (San Diego). Compound 205 was purchased from Sigma Aldrich Co. (U.S.A.).
Compound 206 was synthesized as follows:

Synthesis of 3-methyl-2-(3-methyl-3H-benzothiazol-2-ylidenemethyl)-benzothiazol-3-ium*p*-toluenesulfonate (Compound 206)

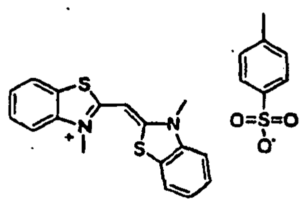

A mixture of 2,2-methylenebisbenzothiazole (0.565 g, 2 mmol) and methyl *p*-toluenesulfonate (0.466 g, 2.5 mmol) was heated by microwave (160˚C, min) to give a gum. The gum was washed with acetone repeatedly to yield the desired compound as a yellow crystal (0.65 g, 67%).

BIOLOGICAL ASSAYS

RAW 264.7 macrophage cells were maintained in sodium pyruvate-free Dubelcco's modified Eagle medium (Hyclone) with 4 mM glutamine containing 1 % non-essential amino acids (Biological Industries, Israel) and 10% heat-inactivated fetal bovine serum (Hyclone) or 10% fetal calf serum (Biological Industries, Israel) in culture plates. The cells were scraped off the culture plates for passage without any trypsin or EDTA treatment, and grown in an incubator at 37˚C and 5% $CO_2$. FuGene6™ was obtained from Roche (German) and lipopolysaccharide of *E. coli* O111:B4 from Chemicon International (California, U.S.A.).

A murine iNOS promoter-Luc, a human iNOS promoter-Luc (pGL3-8296), and a murine cyclooxygenase II promoter-Luc plasmid were generously provided by Drs. Charles J. Lowenstein (John Hopkines University), Joel Moss

(National Institute of Health) and Yu-Chih Liang (Taipei Medical University), respectively. CMX-β-gal plasmid containing the *E. coli* β-galactosidase coding sequence was used for transfection efficiency control.

Inhibition of iNOS promoter activity

RAW 264.7 cells (or A549 cells) were seeded in 24-well plates ($9x10^4$ cells/well). The cells reached to 90-95% confluence in the above-described medium with antibiotics within 24 h and transfected with murine or human iNOS promoter-luciferase reporter plasmids (100 ng/well) using FuGene6 (Roche, Co.) following the protocol provided by the manufacturer. Transfection efficiencies were normalized by co-transfection with 100 ng/well of the β-galactosidase expression plasmid. After 24 h incubation, the medium was replaced with the above-described medium containing LPS (5 μg/ml)/IFNγ (20 ng/ml) and test compounds were added at 10 μM. After 24 h incubation, the medium was removed, cell lysis buffer was added (50 μl/well) and the lysates were subjected to the luciferase assay according to the manufacturer's instructions (Tropix).

The luciferase assay was performed using a luciferase assay system according to the manufacturer's instructions. Luminescence was measured in a TopCount.NXT™ Microplate Scintillation and Luminescence Counter (Packard, Inc.). The results were normalized to β-galactosidase activity derived from co-transfected LacZ gene under the control of a constitutive promoter.

The results show that all test compounds inhibited iNOS promoter activity, thereby suppressing iNOS expression.

Inhibition of nitric oxide production

RAW 264.7 cells were seeded (70,000 cells/well) and cultured in 96-well culture plate. After 24 h incubation, the medium was replaced with a medium containing stimuli of LPS (5 ug/ml)/IFNγ (20 ng/ml) and the test compounds were added at different concentrations. After 18-34 h, the supernatants were subjected to the measurement of nitric oxide production using the Nitrate/Nitrite assay kit (Cayman Chemical). Nitric oxide levels were measured as the accumulation of nitrite and nitrate in the incubation medium. Nitrate was reduced to nitrite with nitrate reductase and determined spectrophotometrically with Griess reagent at $OD_{405}$. The attached cells were subjected to cytotoxicity measurement using a MTS assay. The results show that a number of benzothiazolium compounds effectively inhibited production of NO.

Cytokine measurement

TNF α and IL-6 proteins were measured in cell culture supernatants using an ELISA kit from R & D Systems Inc. (U.S.A.) according to the manufacturer's instructions. The results show that several test compounds effectively inhibited production of TNF a and production of IL-6.

Inhibition of iNOS and cyclooxygenase II expression

Levels of iNOS, cyclooxygenase II, and β-actin (control) were measured by immunoblotting with anti-iNOS antibody (Biomol), anti-cyclooxygenase II antibody (Upstate), and anti-β-actin antibody (Chemicon), respectively. The cell lysates were subjected to SDS-PAGE and the separated proteins were electrophoretically transferred to nitrocellulose membranes. The membranes were incubated, respectively, with blocking solution for 1 h, primary antibody for 2 h, and secondary antibody for 1h, and wash procedures were carried out. Antigen-antibody complexes were detected using ECL detection reagents (Perkin Elmer, Western Blot Chemiluminescence Reagent Plus) according to the manufacturer's instructions. The results show that several benzothazolium compounds lowered both iNOS and cyclooxygenase II levels.

Anti-SARS CoV assay

Fluorogenic peptide substrate Dabcyl-KTSAVLQSGFRKME-Edans was obtained from Biogenesis (Taiwan). Expression and purification of SARS CoV main protease were performed as described in Kuo, et al. Biochemical and Biophysical Research Communications, 2004, 318: 862-867.

A mixture containing 50 nM SARS protease, 6 μM fluorogenic peptide substrate in a buffer of 12 mM Tris-HCl (pH 7.5), 120 mM NaCl, 0.1 mM EDTA, and 1 mM DTT plus 7.5 mM b-ME was prepared. From this mixture, a series of solutions having different concentrations of a test compound (ranging from 0 to 50 μM) were obtained. The fluorescence change of the solutions was measured using a 96-well fluorescence plate reader. The $IC_{50}$ values were calculated using the following equation:

$$A(I) = A(0) \times \{1 - [I/(I + IC_{50})]\}$$

where A(I) is the enzyme activity at compound concentration I; A(0) is the enzyme activity in the absence of the compound; and I is the compound concentration.

A number of compounds inhibited the activity of SARS coronavirus. Unexpectedly, some of the compounds have $IC_{50}$ values lower than 50 μM.

Anti-human CoV 229E assay

Human fibroblast MRC-5 cells were seeded to 96-well plates (70,000 cells/well) and incubated at 37°C for 48 hours. The culture medium was replaced with a medium containing 229E virus. After 1 hour, a test compound was added to the plate and incubated at 37°C for 64 hours. After washed with PBS three times, the cells were fixed and stained with 0.1 % crystal violet and analyzed in an automatic microtiter plate reader at $OD_{570}$ to measure relative cell numbers with respect to control, in which no test compound was added. The results show that several benzothiazolium compounds effectively inhibited human CoV 229E.

Anti-TGEV assay

ST cells were seeded into a 96-well plate (50,000 cells/well) and incubated at 37°C overnight. The culture medium was replaced with 2% fetal bovine serum containing a test compound (final concentration of 10 uM) and incubated at 37°C for 2hrs before TGEV infection (>5 m.o.i.). The culture plates were subjected to indirect fluorescent antibody (IFA) 7 hours after infection, washed with PBS three times, fixed by 80% acetone, and then stored at -20°C. The cells were subjected to IF A staining using antibody against TGEV S and N proteins as the primary antibody and anti-mouse Ig conjugate FITC as the secondary antibody. Fluorescence intensities were measured on a Victor II plate reader. The results show that a number of benzothiazolium compounds effectively inhibited TGEV.

**OTHER EMBODIMENTS**

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the scope of the claims, can make various changes and modifications of the invention to adapt it to various usages and conditions. For example, compounds structurally analogous to above-described compounds also can be made, screened for the above-described activities and used to practice this invention. Thus, other embodiments are also within the claims.

**Claims**

1. Compound of the following formula:

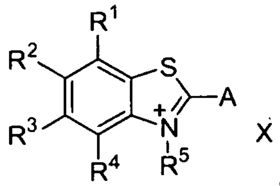

,

wherein

each of $R^1$, $R^2$, $R^3$, and $R^4$, independently, is H, halo, alkyl, alkenyl, alkynyl, alkoxyl, aryl, heteroaryl, cyclyl, heterocyclyl, nitro, cyano, amino, amido, or alkoxycarbonyl, or $R^1$, $R^2$, and the two carbon atoms to which they are attached, together form a 3-8 membered ring containing 0, 1, 2, or 3 heteroatoms, or $R^2$, $R^3$, and the two carbon

atoms to which they are attached, together form a 3-8 membered ring containing 0, 1, 2, or 3 heteroatoms, or $R^3$, $R^4$, and the two carbon atoms to which they are attached, together form a 3-8 membered ring containing 0, 1, 2, or 3 heteroatoms;

$R^5$ is alkyl, aryl, heteroaryl, cyclyl, or heterocyclyl;

A is H, alkyl, aryl, heteroaryl, cyclyl, heterocyclyl, alkoxy, amino, halo, mercapto, thioalkoxy,

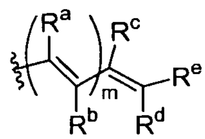

,

or

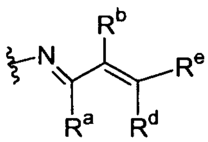

;

in which m is 0, 1,2,or3; each of $R^a$, $R^b$, $R^c$, $R^d$, and $R^e$ independently is H, alkyl, alkenyl, alkynyl, halo, aryl, heteroaryl, cyclyl, heterocyclyl, alkoxy, mercapto, thioalkoxy, amino, amido, or $R^d$, $R^e$, and the carbon atom to which they are attached, together form a 3-8 membered ring containing 0, 1, 2, or 3 heteroatoms, which is optionally fused with aryl, heteroaryl, cyclyl, or heterocyclyl;

optionally A, $R^5$, the N atom to which $R^5$ is attached, and the carbon atom to which A is attached, together form a 4-8 membered ring containing 1, 2, or 3 heteroatoms; and

X is an anion or a negatively charged group attached to an atom of $R^1$, $R^2$, $R^3$, $R^4$, $R^5$, or A,

for use in treating coronaviral infection.

**2.** The compound of claim 1, wherein X is a negatively charged group attached to an atom of $R^1$, $R^2$, $R^3$, $R^4$, $R^5$, or A, the negatively charged group being selected from $-CO_2^-$, $-SO_3^-$, or $-PO_3^{2-}$.

**3.** The compound of claim 1, wherein X is $I^-$, $Br^-$, $Cl^-$, $ClO_4^-$, $C_2H_5SO_4^-$, or

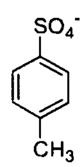

.

**4.** The compound of claim 1, wherein A is H or alkyl.

**5.** The compound of claim 4, wherein $R^5$ is alkyl.

**6.** The compound of claim 1, wherein $R^5$ is alkyl.

**7.** The compound of claim 1, wherein A is

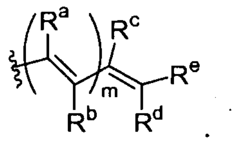

8. The compound of claim 7, wherein one of $R^d$ and $R^e$ is substituted or unsubstituted phenyl.

9. The compound of claim 7, wherein one of $R^d$ and $R^e$ is thioalkoxy, amino, or amido.

10. The compound of claim 7, wherein $R^d$, $R^e$, and the carbon atom to which they are attached, together form a 3-8 membered ring containing 0, 1, or 2 heteroatoms.

11. The compound of claim 10, wherein A is

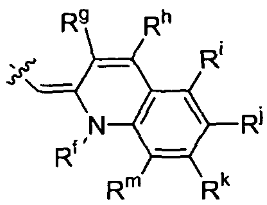

,

$R^f$ being H, alkyl, alkenyl, aryl, cyclyl, heteroaryl, or heterocyclyl; and each of $R^g$, $R^h$, $R^i$, $R^j$, $R^k$, and $R^m$, independently being H, alkyl, alkenyl, aryl, cyclyl, heteroaryl, heterocyclyl, alkoxyl, halogen, nitro, or cyano.

12. The compound of claim 11, wherein each of $R^f$ and $R^5$ is alkyl.

13. The compound of claim 10, wherein A is

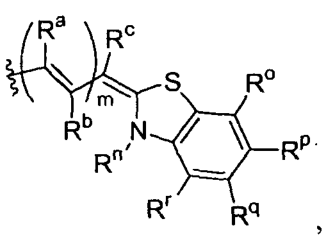

,

$R^n$ being H, alkyl, alkenyl, aryl, cyclyl, heteroaryl, heterocyclyl, and each of $R^o$, $R^p$, $R^q$, and $R^r$, independently, being H, alkyl, alkenyl, aryl, cyclyl, heteroaryl, heterocyclyl, alkoxyl, nitro, amino, cyano, or halogen.

14. The compound of claim 13, wherein each of $R^n$ and $R^5$ is alkyl.

15. The compound of claim 1, wherein the compound is selected from those listed in the Table below:

| Compound Number | Structure |
|---|---|
| 1 | 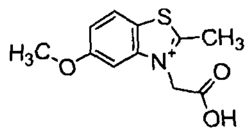 $Br^-$ |
| 2 | 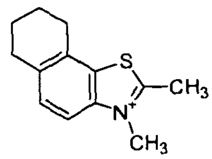 $p$-$CH_3C_6H_4SO_3^-$ |
| 3 | 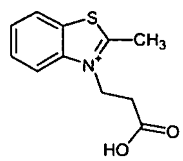 $Br^-$ |
| 4 | 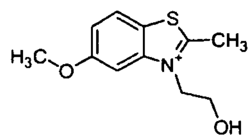 $I^-$ |
| 5 | 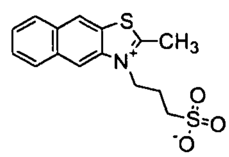 |
| 6 | 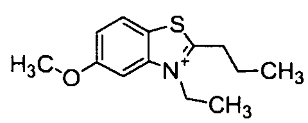 $I^-$ |
| 7 | 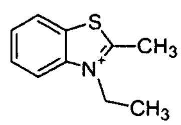 $p$-$CH_3C_6H_4SO_3^-$ |
| 8 | 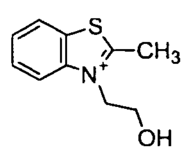 $Cl^-$ |
| 9 | 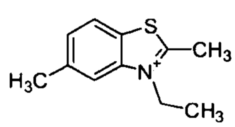 $I^-$ |
| 10 | 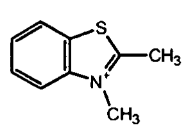 $I^-$ |

(continued)

| Compound Number | Structure |
|---|---|
| 11 | 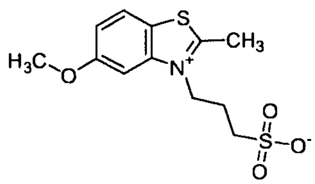 |
| 12 | 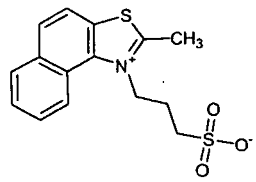 |
| 13 | 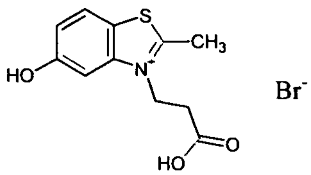 |
| 14 | 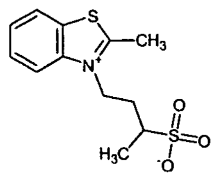 |
| 15 | 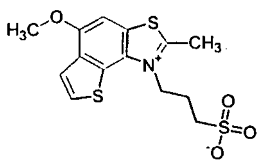 |
| 16 | 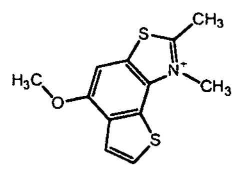 |
| 17 | 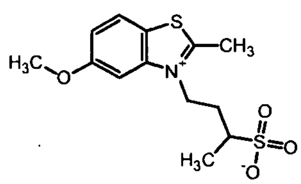 |

(continued)

| Compound Number | Structure |
|---|---|
| 18 | 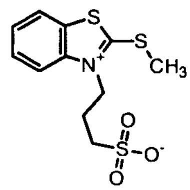 |
| 19 | 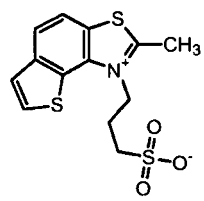 |
| 20 | 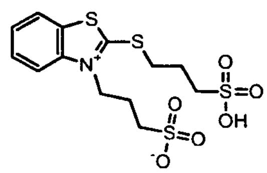 |
| 21 | 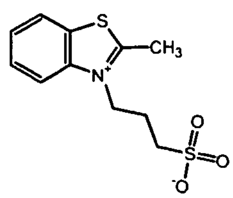 |
| 22 | 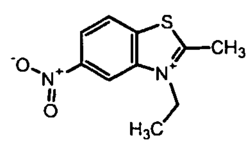 $I^-$ |
| 23 | 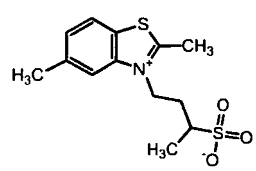 |
| 24 | 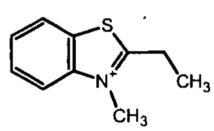 $I^-$ |
| 25 | 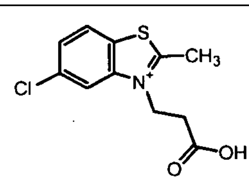 *p*-$CH_3C_6H_4SO_3^-$ |

(continued)

| Compound Number | Structure |
|---|---|
| 26 | 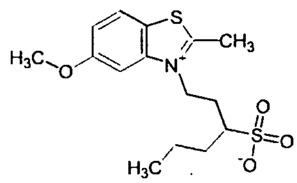 |
| 27 | 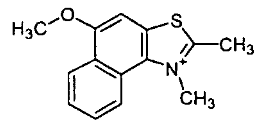 $I^-$ |
| 28 | 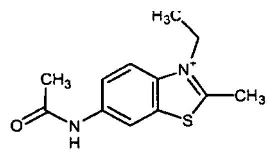 *p*-$CH_3C_6H_4SO_3^-$ |
| 29 | 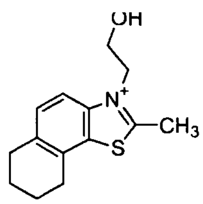 $I^-$ |
| 32 | 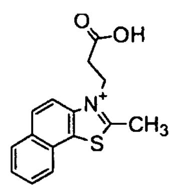 $Br^-$ |
| 33 | 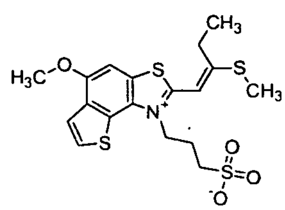 |
| 34 | 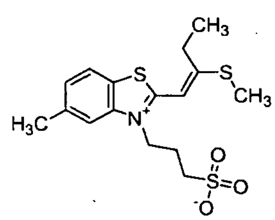 |

(continued)

| Compound Number | Structure |
|---|---|
| 35 | 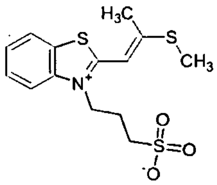 |
| 36 | 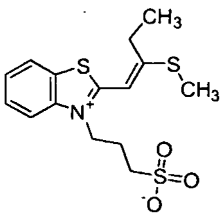 |
| 37 | 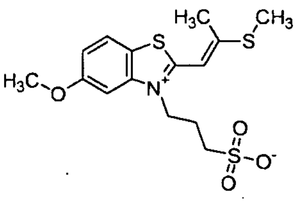 |
| 38 | 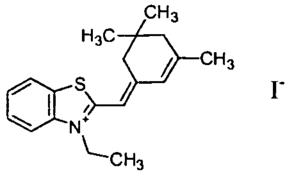 |
| 39 | 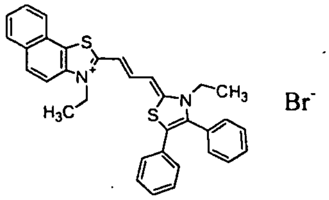 |
| 40 | 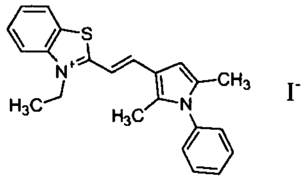 |
| 41 | 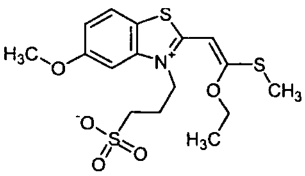 |

(continued)

| Compound Number | Structure |
|---|---|
| 42 | 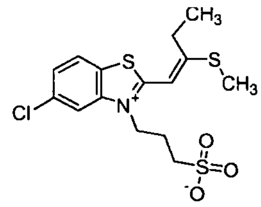 |
| 43 | 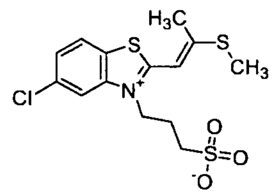 |
| 44 | 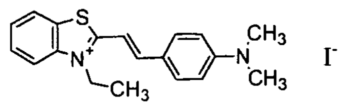 |
| 45 | 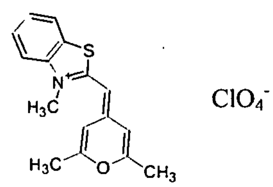 |
| 46 | 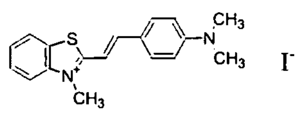 |
| 47 | 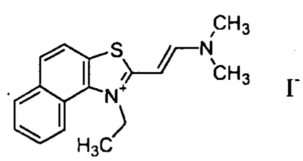 |
| 48 | 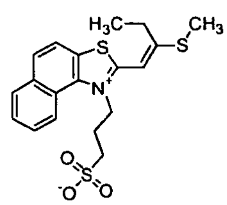 |
| 49 | 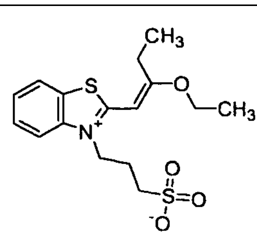 |

(continued)

| Compound Number | Structure |
|---|---|
| 50 | 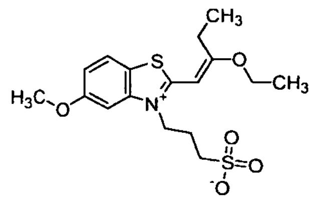 |
| 51 | 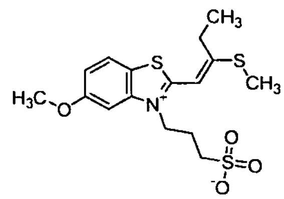 |
| 52 | 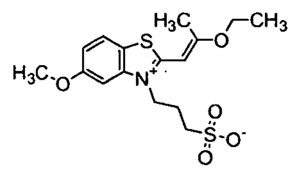 |
| 53 | 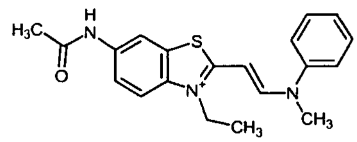 $I^-$ |
| 54 | 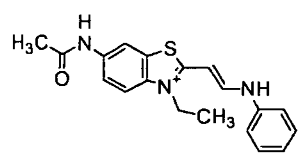 $ClO_4^-$ |
| 55 | 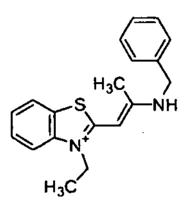 $I^-$ |
| 56 | 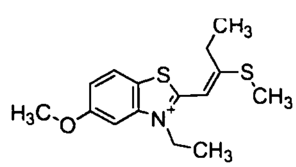 $CH_3SO_4^-$ |
| 57 | 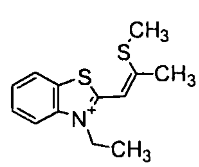 $CH_3SO_4^-$ |

(continued)

| Compound Number | Structure |
|---|---|
| 58 | 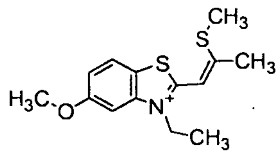 $CH_3SO_4^-$ |
| 59 | 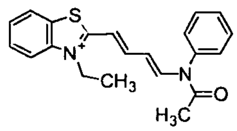 $I^-$ |
| 60 | 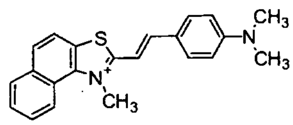 *p*-$CH_3C_6H_4SO_3^-$ |
| 61 | 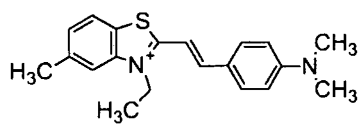 $I^-$ |
| 62 | 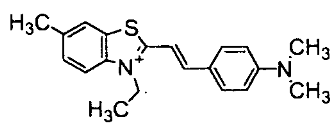 $I^-$ |
| 63 | 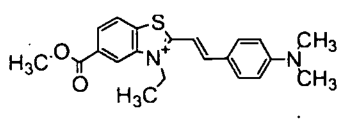 $I^-$ |
| 64 | 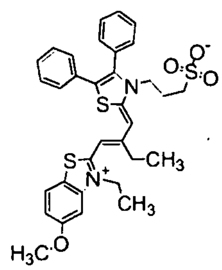 |
| 65 | 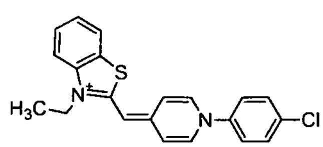 $I^-$ |
| 66 | 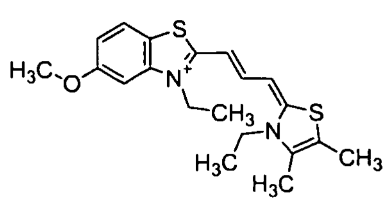 $I^-$ |

(continued)

| Compound Number | Structure |
|---|---|
| 67 | 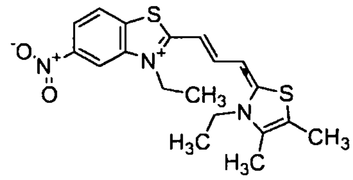 $I^-$ |
| 68 | 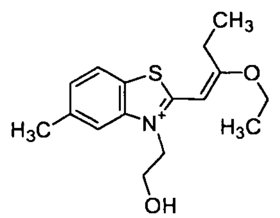 $ClO_4^-$ |
| 69 | 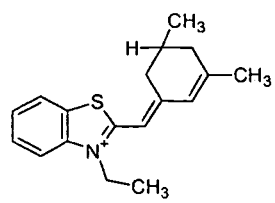 $I^-$ |
| 70 | 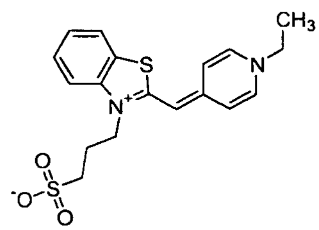 $I^-$ |
| 71 | 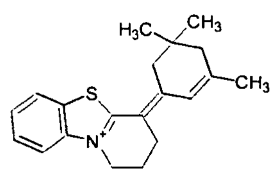 $I^-$ |
| 72 | 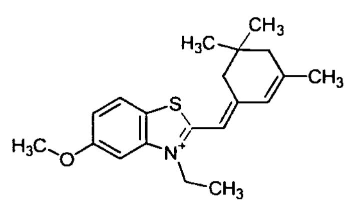 $p$-$CH_3C_6H_4SO_3^-$ |
| 73 | 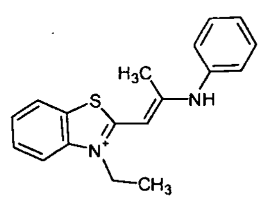 $I^-$ |

(continued)

| Compound Number | Structure |
|---|---|
| 74 | 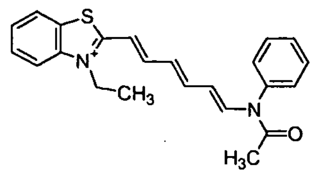 $I^-$ |
| 75 | 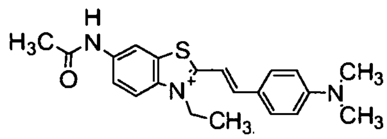 $I^-$ |
| 76 | 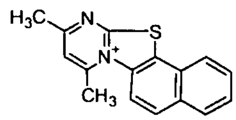 $ClO_4^-$ |
| 77 | 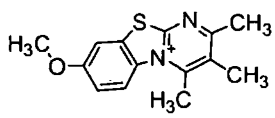 $ClO_4^-$ |
| 78 | 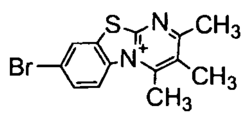 $ClO_4^-$ |
| 79 | 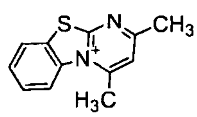 $ClO_4^-$ |
| 80 | 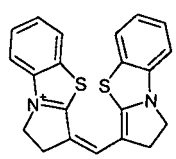 *p*-$CH_3C_6H_4SO_3^-$ |
| 81 | 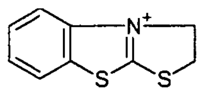 $Br^-$ |
| 82 | 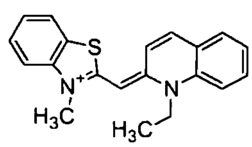 $I^-$ |

(continued)

| Compound Number | Structure |
|---|---|
| 83 | 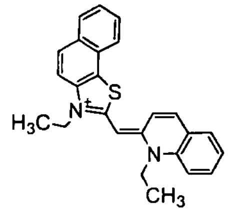 Cl⁻ |
| 84 | 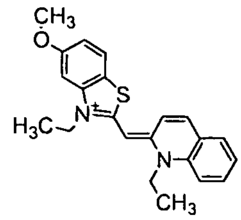 I⁻ |
| 85 | 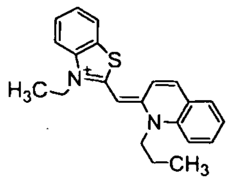 I⁻ |
| 86 | 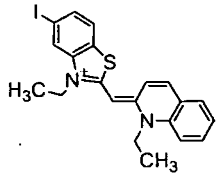 I⁻ |
| 87 | 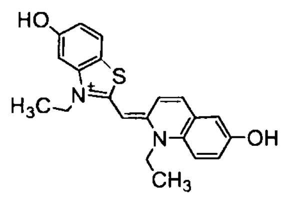 I⁻ |
| 88 | 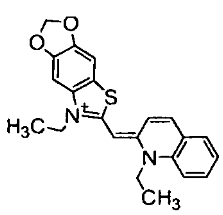 I⁻ |
| 89 | 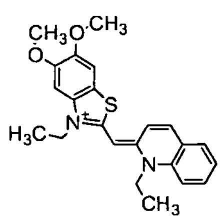 I⁻ |

(continued)

| Compound Number | Structure |
|---|---|
| 90 | 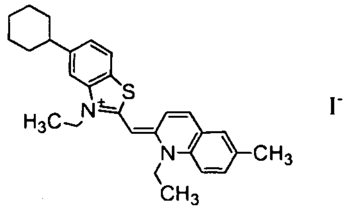 |
| 91 | 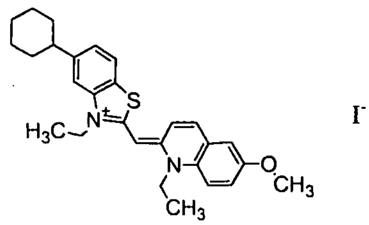 |
| 92 | 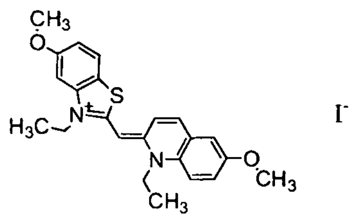 |
| 93 | 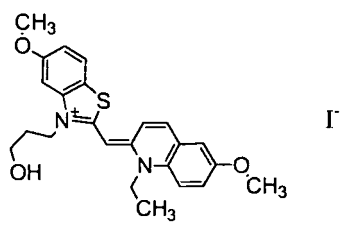 |
| 94 | 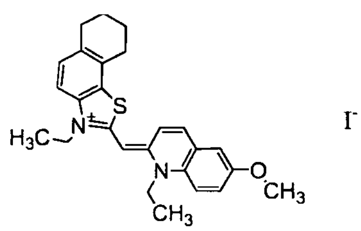 |
| 95 | 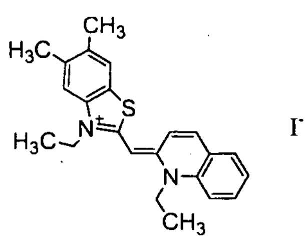 |

(continued)

| Compound Number | Structure |
|---|---|
| 96 | 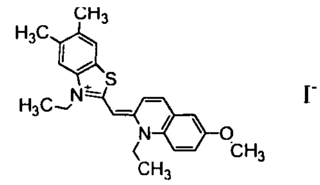 |
| 97 | 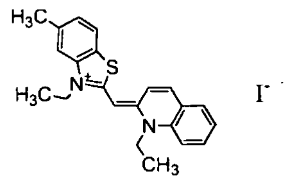 |
| 98 | 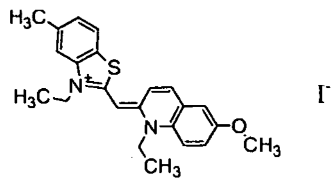 |
| 99 | 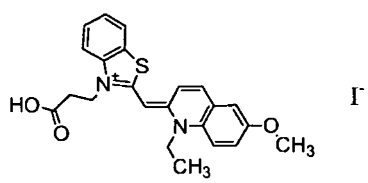 |
| 100 | 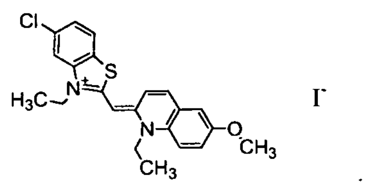 |
| 101 | 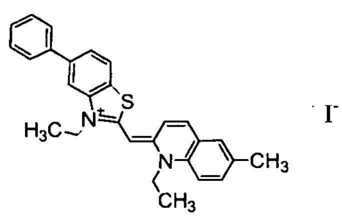 |
| 102 | 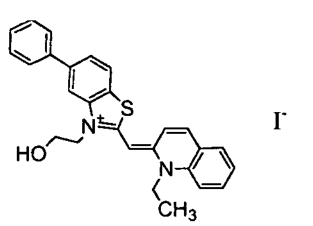 |

(continued)

| Compound Number | Structure |
|---|---|
| 103 | 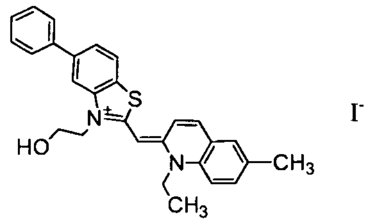 |
| 104 | 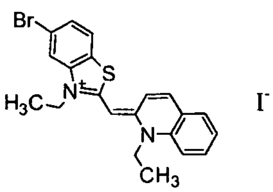 |
| 105 | 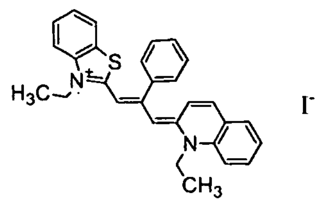 |
| 106 | 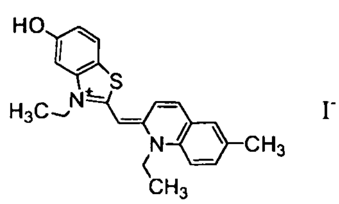 |
| 107 | 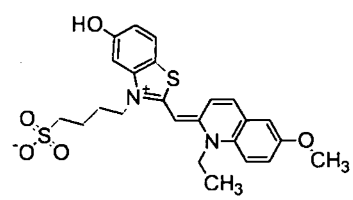 |
| 108 | 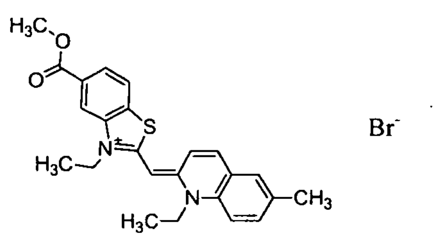 |

(continued)

| Compound Number | Structure |
|---|---|
| 109 | 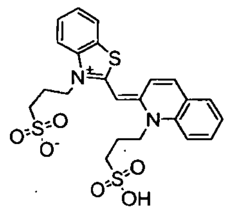 |
| 110 | 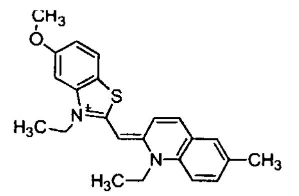 $p$-$CH_3C_6H_4SO_3^-$ |
| 111 | 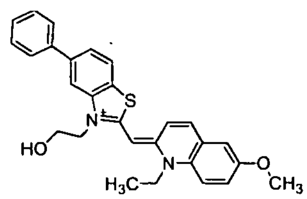 $I^-$ |
| 112 | 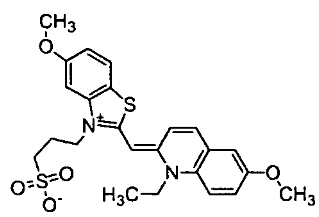 |
| 113 | 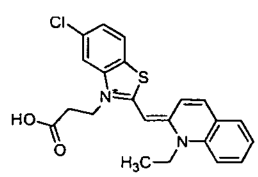 $Cl^-$ |
| 114 | 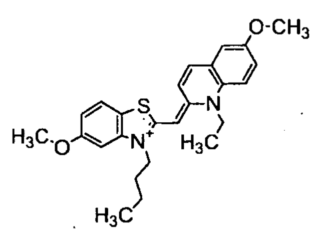 $I^-$ |
| 115 | 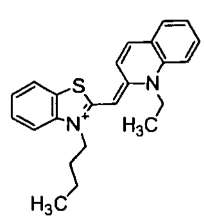 $I^-$ |

(continued)

| Compound Number | Structure |
|---|---|
| 116 | 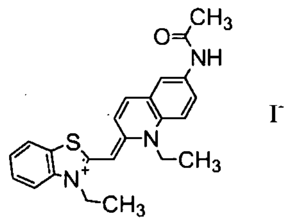 |
| 117 | 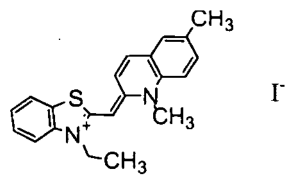 |
| 118 | 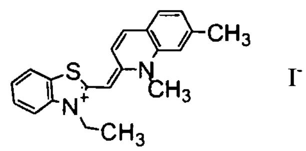 |
| 119 | 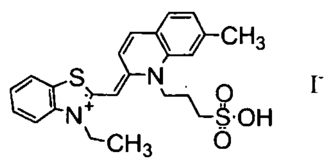 |
| 120 | 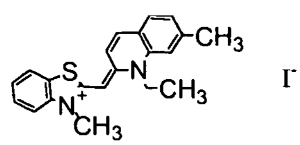 |
| 121 | 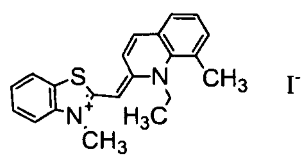 |
| 122 | 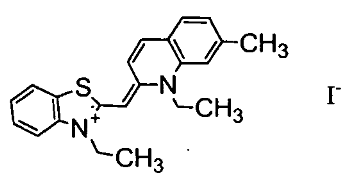 |

(continued)

| Compound Number | Structure |
| --- | --- |
| 123 | 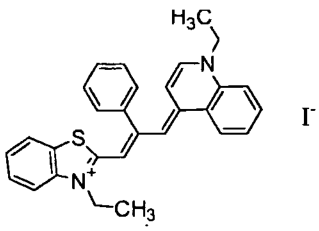 |
| 124 | 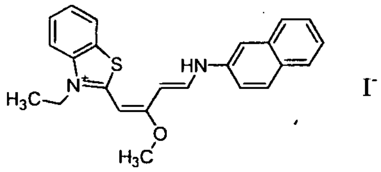 |
| 125 | 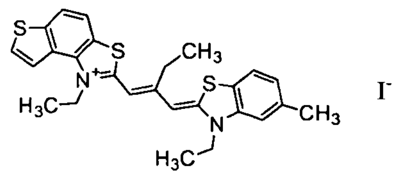 |
| 126 | 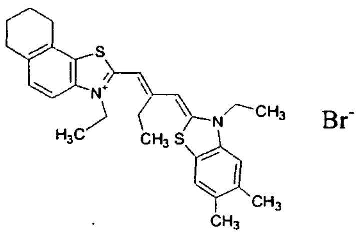 |
| 127 | 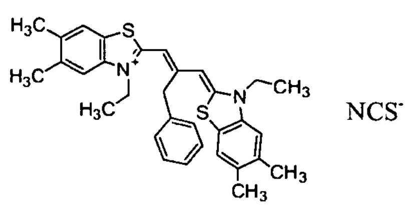 |
| 128 | 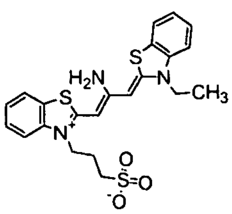 |
| 129 | 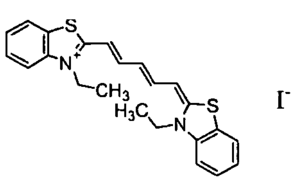 |

(continued)

| Compound Number | Structure |
|---|---|
| 130 | 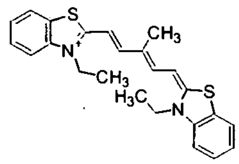 $I^-$ |
| 131 | 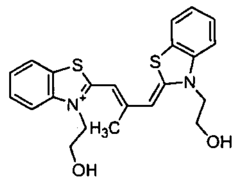 $Cl^-$ |
| 132 | 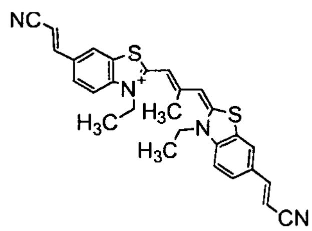 *p*-$CH_3C_6H_4SO_3^-$ |
| 133 | 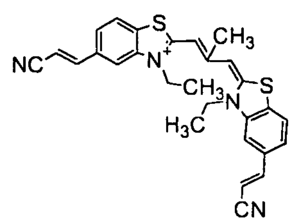 *p*-$CH_3C_6H_4SO_3^-$ |
| 134 | 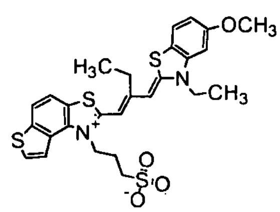 |
| 135 | 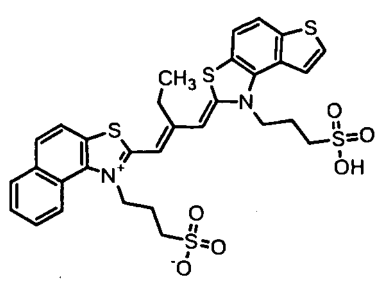 |

(continued)

| Compound Number | Structure |
|---|---|
| 136 | 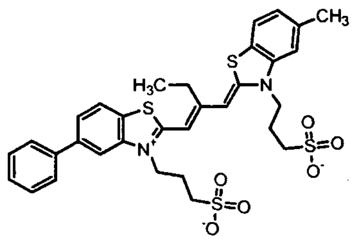 $(C_4H_9)_3NH^+$ |
| 137 | 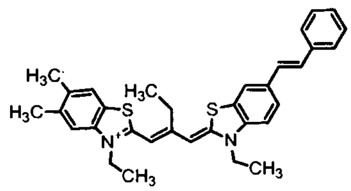 $I^-$ |
| 138 | 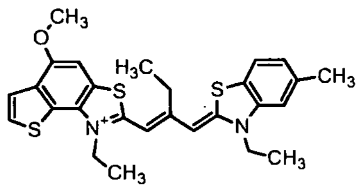 $Br^-$ |
| 139 | 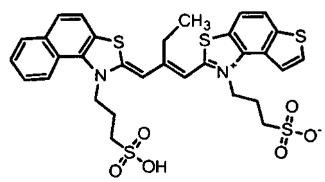 |
| 140 | 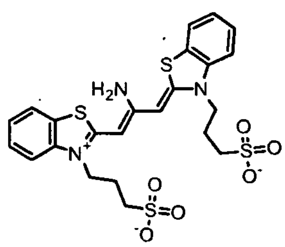 $NH_4^+$ |
| 141 | 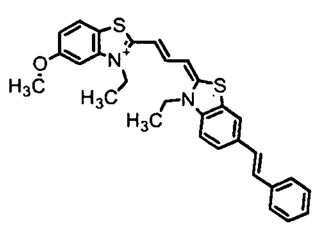 $I^-$ |

(continued)

| Compound Number | Structure |
|---|---|
| 142 | 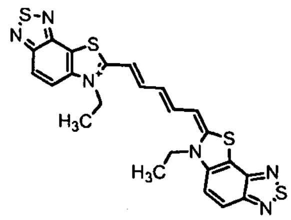 $p$-$CH_3C_6H_4SO_3^-$ |
| 143 | 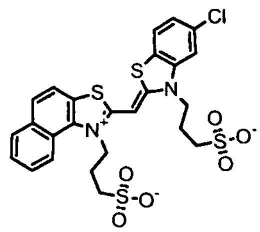 $(C_2H_5)_3NH^+$ |
| 144 | 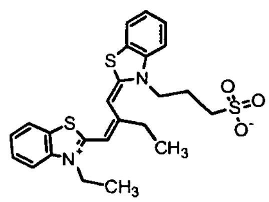 |
| 145 | 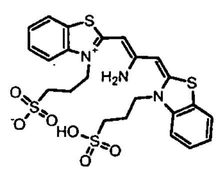 |
| 146 | 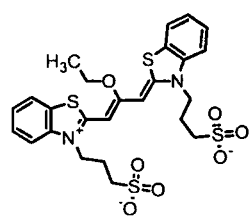 $(C_2H_5)_3NH^+$ |
| 147 | 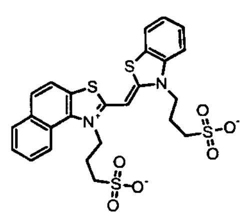 $(C_2H_5)_3NH^+$ |
| 148 | 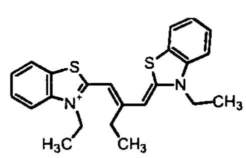 $I^-$ |

(continued)

| Compound Number | Structure |
|---|---|
| 149 | 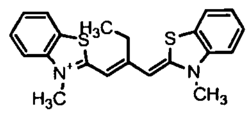 $I^-$ |
| 150 | 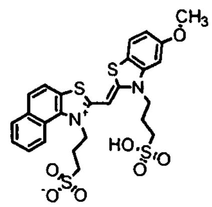 |
| 151 | 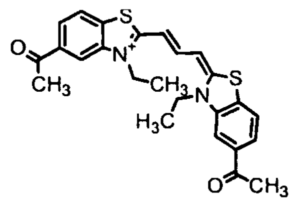 $C_2H_5SO_4^-$ |
| 152 | 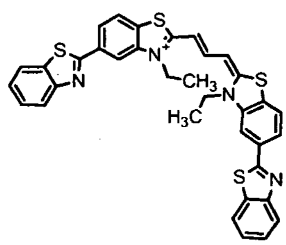 $p$-$CH_3C_6H_4SO_3^-$ |
| 153 | 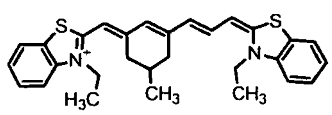 $I^-$ |
| 154 | 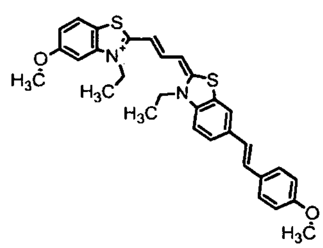 $I^-$ |
| 155 | 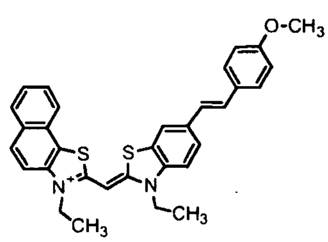 $p$-$CH_3C_6H_4SO_3^-$ |

(continued)

| Compound Number | Structure |
|---|---|
| 156 | 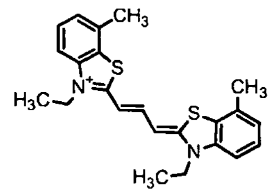 $I^-$ |
| 157 | 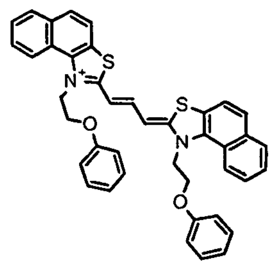 $p$-$CH_3C_6H_4SO_3^-$ |
| 158 | 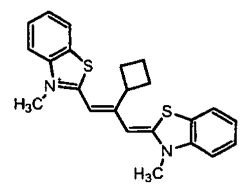 $I^-$ |
| 159 | 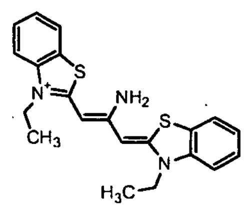 $I^-$ |
| 160 | 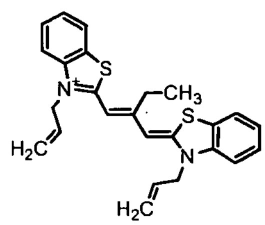 $I^-$ |
| 161 | 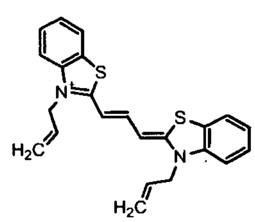 $I^-$ |

(continued)

| Compound Number | Structure |
|---|---|
| 162 | 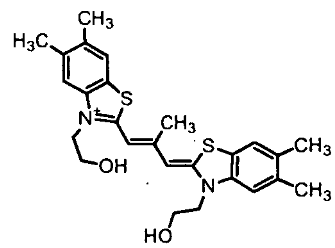 $I^-$ |
| 163 | 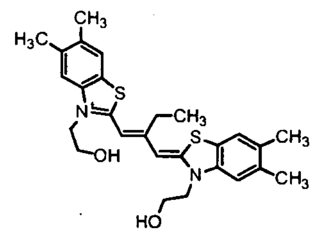 $I^-$ |
| 164 | 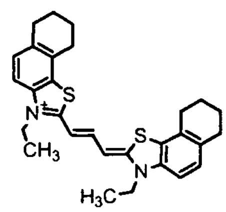 $I^-$ |
| 165 | 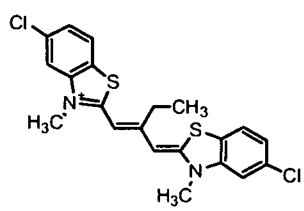 $p\text{-}CH_3C_6H_4SO_3^-$ |
| 166 | 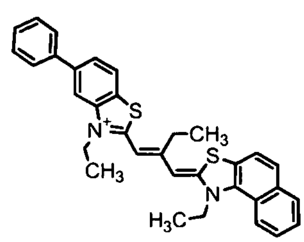 $Br^-$ |
| 167 | 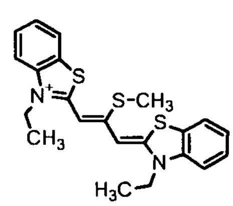 $CH_3SO_4^-$ |

(continued)

| Compound Number | Structure |
|---|---|
| 168 | 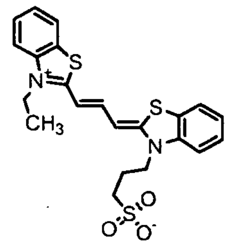 |
| 169 | 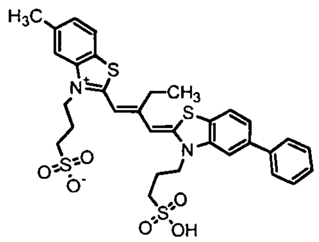 |
| 170 | 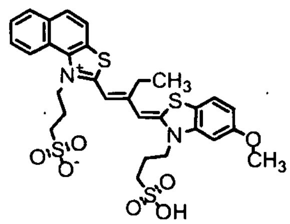 |
| 171 | 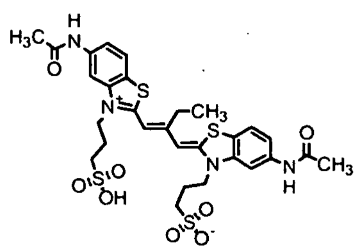 |
| 172 | 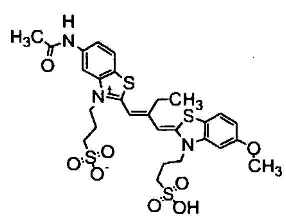 |
| 173 | 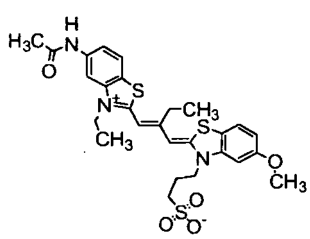 |

(continued)

| Compound Number | Structure |
|---|---|
| 174 | 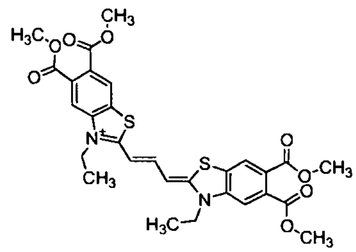 $I^-$ |
| 175 | 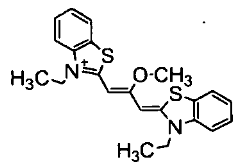 $CH_3SO_4^-$ |
| 176 | 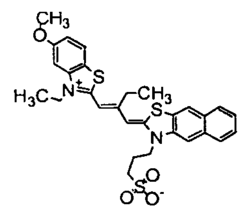 |
| 177 | 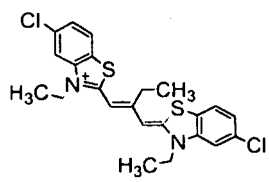 $NO_3^-$ |
| 178 | 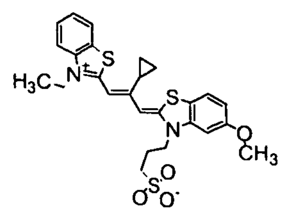 |
| 179 | 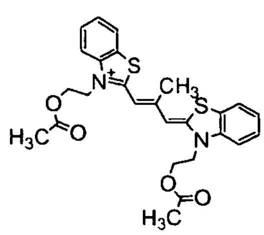 $I^-$ |
| 180 | 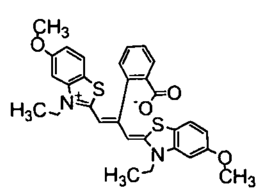 |

(continued)

| Compound Number | Structure |
|---|---|
| 181 | 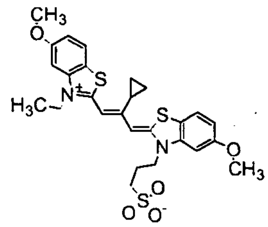 |
| 182 | 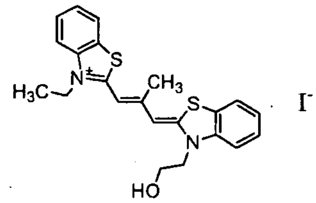 |
| 183 | 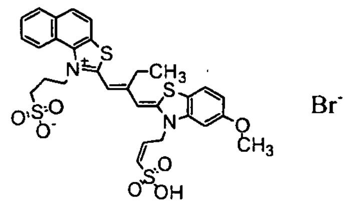 |
| 184 | 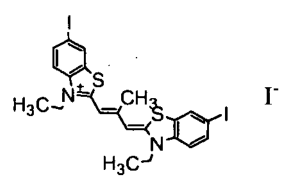 |
| 185 | 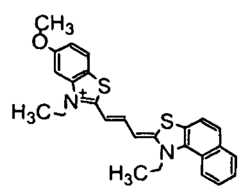 |
| 186 | 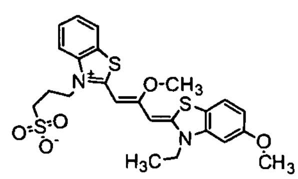 |
| 187 | 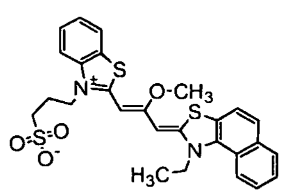 |

(continued)

| Compound Number | Structure |
|---|---|
| 188 | 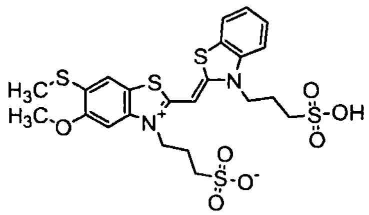 |
| 189 | 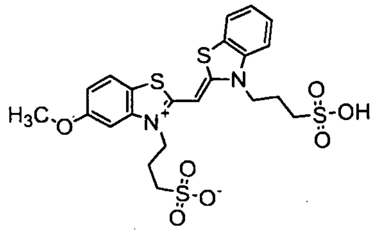 |
| 190 | 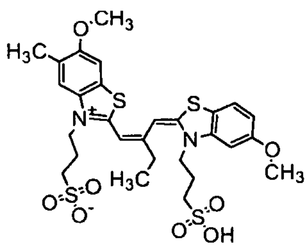 |
| 191 | 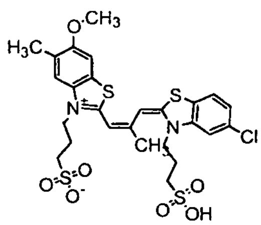 |
| 192 | 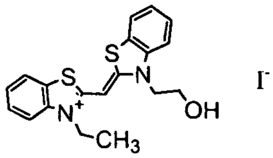 |
| 193 | 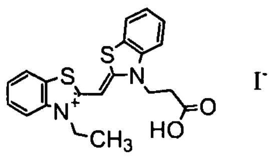 |
| 194 | 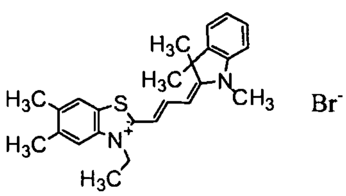 |

(continued)

| Compound Number | Structure |
|---|---|
| 195 | 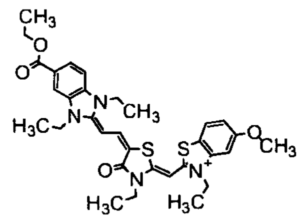 $I^-$ |
| 196 | 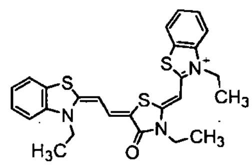 $I^-$ |
| 197 | 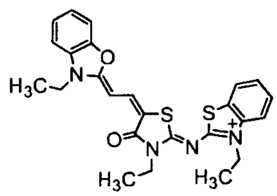 $p$-$CH_3C_6H_4SO_3^-$ |
| 198 | 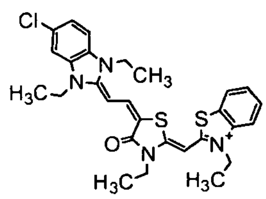 $I^-$ |
| 199 | 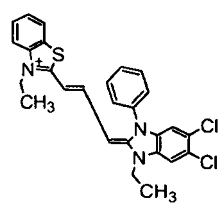 $I^-$ |
| 200 | 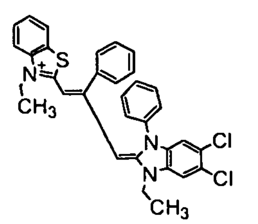 $I^-$ |
| 201 | 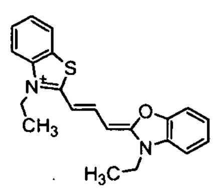 $I^-$ |

(continued)

| Compound Number | Structure |
|---|---|
| 202 | 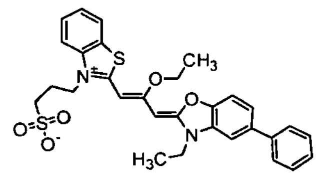 |
| 204 | 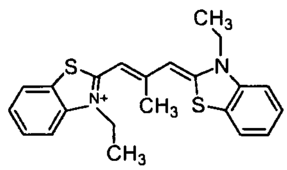 $I^-$ |
| 205 | 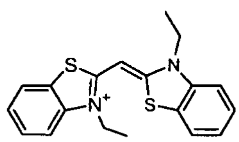 $I^-$ |
| 206 | 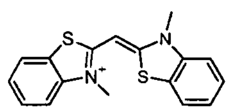 $p$-$CH_3C_6H_4SO_3^-$ |

**16.** The compound of claim 1, wherein said compound inhibits production of nitric oxide by suppressing expression of inducible nitric oxide synthase.

**17.** Use of a compound as defined in any one of claims 1-16 in the manufacture of a medicament for treating coronaviral infections.

## Patentansprüche

**1.** Verbindung der folgenden Formel:

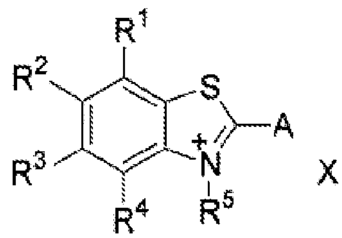

,

wobei
jedes aus $R^1$, $R^2$, $R^3$ und $R^4$ unabhängig voneinander H, Halogen, Alkyl, Alkenyl, Alkynyl, Alkoxyl, Aryl, Heteroaryl, Cyclyl, Heterocyclyl, Nitro, Cyano, Amino, Amido oder Alkoxycarbonyl ist, oder $R^1$, $R^2$ und die zwei Kohlenstoffatome, an welchen sie gebunden sind, zusammen einen 3-8 gliedrigen Ring bilden, der 0, 1, 2 oder 3 Heteroatome enthält, oder $R^2$, $R^3$ und die zwei Kohlenstoffatome, an welchen sie gebunden sind, zusammen einen 3-8 gliedrigen Ring bilden, der 0, 1, 2 oder 3 Heteroatome enthält, oder $R^3$, $R^4$ und die zwei Kohlenstoffatome, an welchen sie gebunden sind, zusammen einen 3-8 gliedrigen Ring bilden, der 0, 1, 2 oder 3 Heteroatome enthält;

$R^5$ ein Alkyl, Aryl, Heteroaryl, Cyclyl oder Heterocyclyl ist;
A ein H, Alkyl, Aryl, Heteroaryl, Cyclyl, Heterocyclyl, Alkoxy, Amino, Halogen, Mercapto, Thioalkoxy,

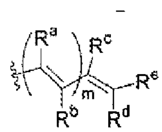

oder

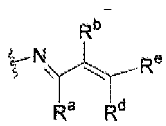

ist; in welchen m gleich 0, 1, 2 oder 3 ist; jedes aus $R^a$, $R^b$, $R^c$, $R^d$ und $R^e$ unabhängig voneinander H, Alkyl, Alkenyl, Alkynyl, Halogen, Aryl, Heteroaryl, Cyclyl, Heterocyclyl, Alkoxy, Mercapto, Thioalkoxy, Amino, Amido ist, oder $R^d$, $R^e$ und das Kohlenstoffatom, an welchem sie gebunden sind, zusammen einen 3-8 gliedrigen Ring bilden, der 0, 1, 2 oder 3 Heteroatome enthält, welcher optional mit Aryl, Heteroaryl, Cyclyl oder Heterocyclyl kondensiert ist; optional A, $R^5$, das N-Atom, an welchem $R^5$ gebunden ist, und das Kohlenstoffatom, an welchem A gebunden ist, zusammen einen 4-8 gliedrigen Ring bilden, der 1, 2 oder 3 Heteroatome enthält; und
X ein Anion oder eine negativ geladene Gruppe ist, die an einem Atom von $R^1$, $R^2$, $R^3$, $R^4$, $R^5$ oder A gebunden ist, zur Verwendung bei der Behandlung koronaviraler Infektionen.

**2.** Verbindung nach Anspruch 1, wobei X eine negativ geladene Gruppe ist, die an einem Atom von $R^1$, $R^2$, $R^3$, $R^4$, $R^5$ oder A gebunden ist, wobei die negativ geladene Gruppe aus $-CO_2^-$, $-SO_3^-$ oder $-PO_3^{2-}$ ausgewählt ist.

**3.** Verbindung nach Anspruch 1, wobei X gleich $I^-$, $Br^-$, $Cl^-$, $ClO_4^-$, $C_2H_5SO_4^-$ oder

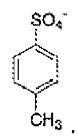

ist.

**4.** Verbindung nach Anspruch 1, wobei A ein H oder Alkyl ist.

**5.** Verbindung nach Anspruch 4, wobei $R^5$ ein Alkyl ist.

**6.** Verbindung nach Anspruch 1, wobei $R^5$ ein Alkyl ist.

**7.** Verbindung nach Anspruch 1, wobei A gleich

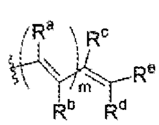

ist.

**8.** Verbindung nach Anspruch 7, wobei eines aus $R^d$ und $R^e$ substituiertes oder unsubstituiertes Phenyl ist.

**9.** Verbindung nach Anspruch 7, wobei eines aus $R^d$ und $R^e$ Thioalkoxy, Amino oder Amido ist.

**10.** Verbindung nach Anspruch 7, wobei $R^d$, $R^e$ und das Kohlenstoffatom, an welchem sie gebunden sind, zusammen einen 3-8 gliedrigen Ring bilden, der 0, 1 oder 2 Heteroatome enthält.

**11.** Verbindung nach Anspruch 10, wobei A gleich

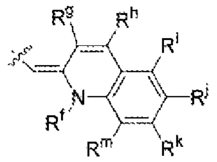

ist,
$R^f$ ein H, Alkyl, Alkenyl, Aryl, Cyclyl,
Heteroaryl oder Heterocyclyl ist; und jedes aus $R^g$, $R^h$, $R^1$, $R^j$, $R^k$ und $R^m$ unabhängig voneinander H, Alkyl, Alkenyl, Aryl, Cyclyl, Heteroaryl, Heterocyclyl, Alkoxyl, Halogen, Nitro oder Cyano sind.

**12.** Verbindung nach Anspruch 11, wobei jedes aus $R^f$ und $R^5$ ein Alkyl ist.

**13.** Verbindung nach Anspruch 10, wobei A gleich

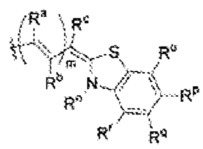

ist,
$R^n$ ein H, Alkyl, Alkenyl, Aryl, Cyclyl, Heteroaryl, Heterocyclyl ist, und jedes aus $R^o$, $R^p$, $R^q$ und $R^r$ unabhängig voneinander H, Alkyl, Alkenyl, Aryl, Cyclyl, Heteroaryl, Heterocyclyl, Alkoxyl, Nitro, Amino, Cyano oder Halogen ist.

**14.** Verbindung nach Anspruch 13, wobei jedes aus $R^n$ und $R^5$ ein Alkyl ist.

**15.** Verbindung nach Anspruch 1, wobei die Verbindung aus denen ausgewählt ist, die in der nachfolgenden Tabelle aufgeführt sind:

| Verbindung Nummer | Struktur |
|---|---|
| 1 | 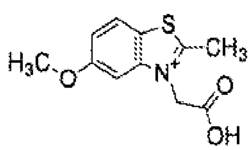 $Br^-$ |
| 2 | 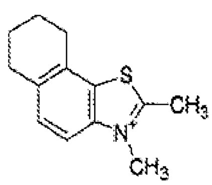 $p$-$CH_3C_6H_4SO_3^-$ |

(fortgesetzt)

| Verbindung Nummer | Struktur |
|---|---|
| 3 | 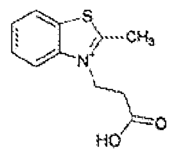 $Br^-$ |
| 4 | 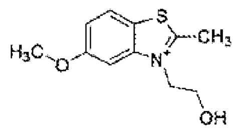 $I^-$ |
| 5 | 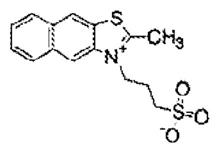 |
| 6 | 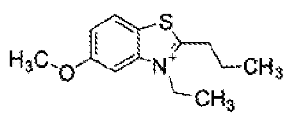 $I^-$ |
| 7 | 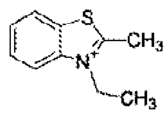 *p*-$CH_3C_6H_4SO_3^-$ |
| 8 | 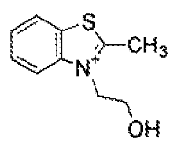 $Cl^-$ |
| 9 | 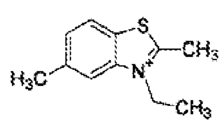 $I^-$ |
| 10 | 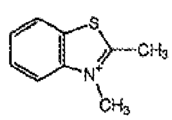 $I^-$ |
| 11 | 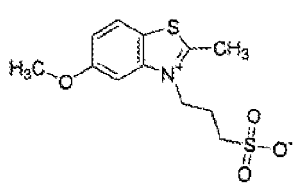 |

(fortgesetzt)

| Verbindung Nummer | Struktur |
|---|---|
| 12 | 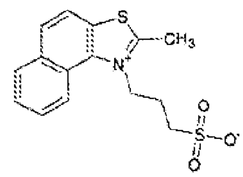 |
| 13 | 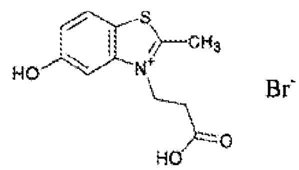 |
| 14 | 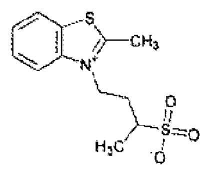 |
| 15 | 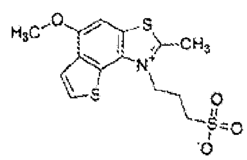 |
| 16 | 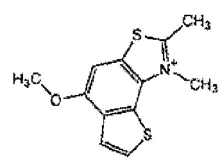 |
| 17 | 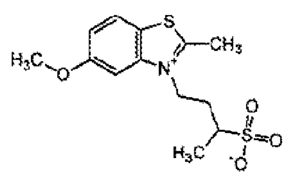 |
| 18 | 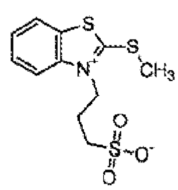 |

(fortgesetzt)

| Verbindung Nummer | Struktur |
|---|---|
| 19 | 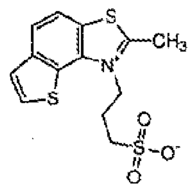 |
| 20 | 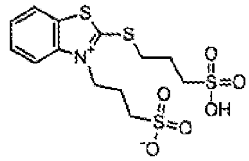 |
| 21 | 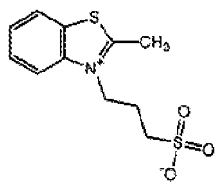 |
| 22 | 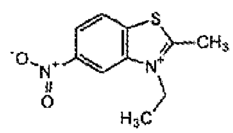 $I^-$ |
| 23 | 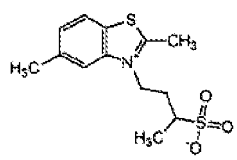 |
| 24 | 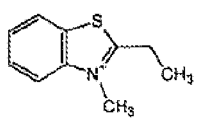 $I^-$ |
| 25 | 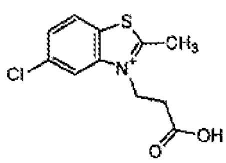 $p$-$CH_3C_6H_4SO_3^-$ |
| 26 | 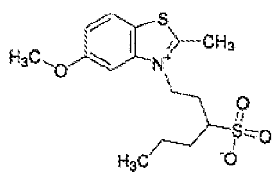 |

(fortgesetzt)

| Verbindung Nummer | Struktur |
|---|---|
| 27 | 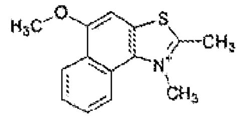 $I^-$ |
| 28 | 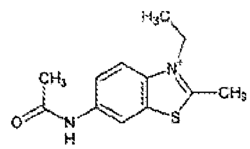 $p$-$CH_3C_6H_4SO_3^-$ |
| 29 | 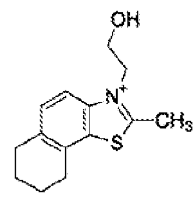 $I^-$ |
| 32 | 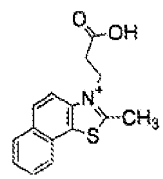 $Br^-$ |
| 33 | 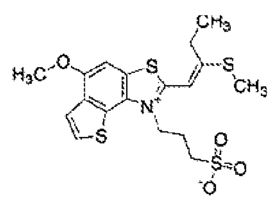 |
| 34 | 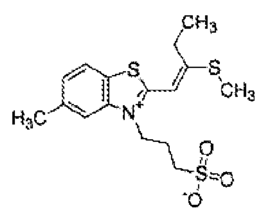 |
| 35 | 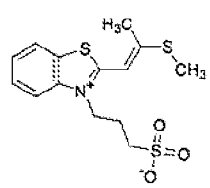 |

(fortgesetzt)

| Verbindung Nummer | Struktur |
|---|---|
| 36 | 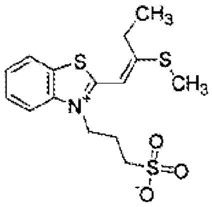 |
| 37 | 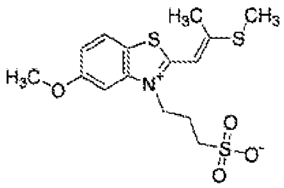 |
| 38 | 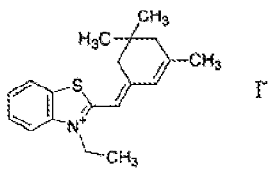 |
| 39 | 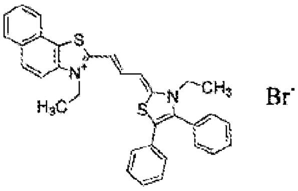 |
| 40 | 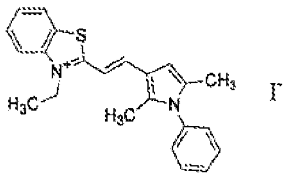 |
| 41 | 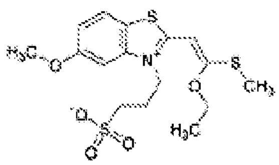 |
| 42 | 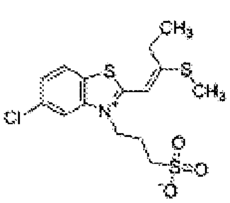 |

(fortgesetzt)

| Verbindung Nummer | Struktur |
|---|---|
| 43 | 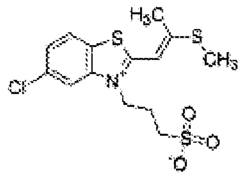 |
| 44 | 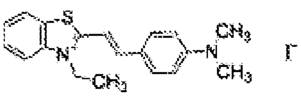 |
| 45 | 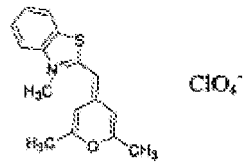 |
| 46 | 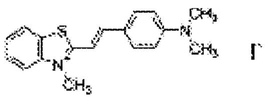 |
| 47 | 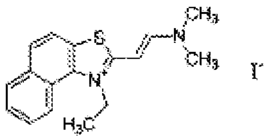 |
| 48 | 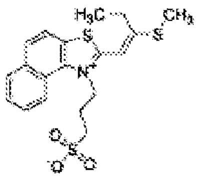 |
| 49 | 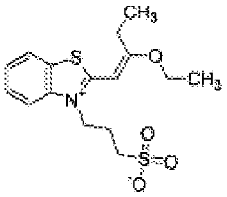 |
| 50 | 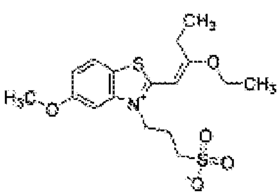 |

(fortgesetzt)

| Verbindung Nummer | Struktur |
|---|---|
| 51 | 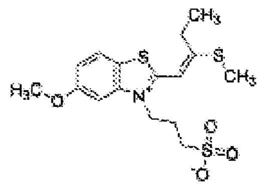 |
| 52 | 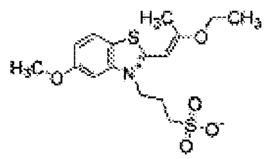 |
| 53 | 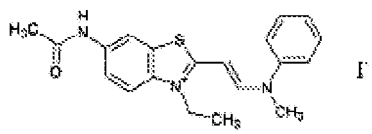 |
| 54 | 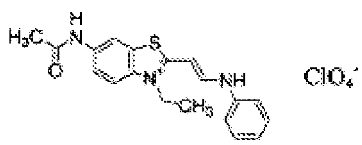 |
| 55 | 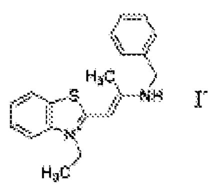 |
| 56 | 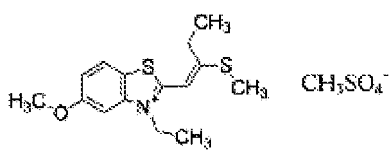 |
| 57 | 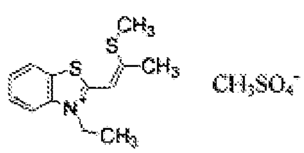 |
| 58 | 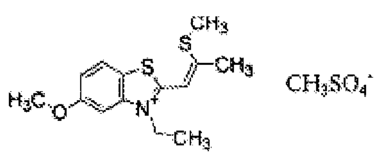 |
| 59 | 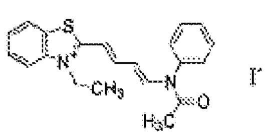 |

(fortgesetzt)

| Verbindung Nummer | Struktur |
|---|---|
| 60 | 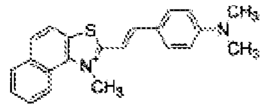 $p$-$CH_3C_6H_4SO_3^-$ |
| 61 | 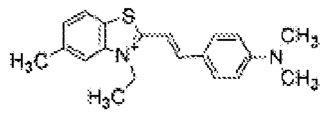 $I^-$ |
| 62 | 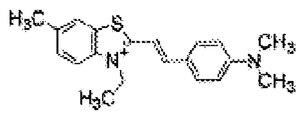 $I^-$ |
| 63 | 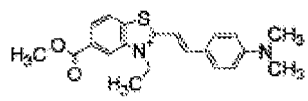 $I^-$ |
| 64 | 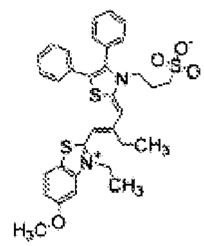 |
| 65 | 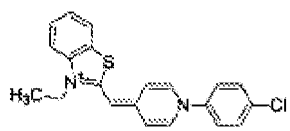 $I^-$ |
| 66 | 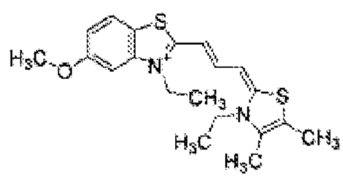 $I^-$ |
| 67 | 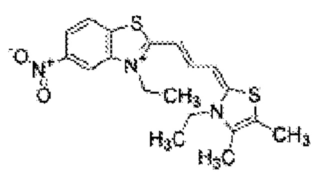 $I^-$ |
| 68 | 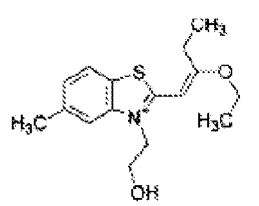 $ClO_4^-$ |

(fortgesetzt)

| Verbindung Nummer | Struktur |
|---|---|
| 69 | 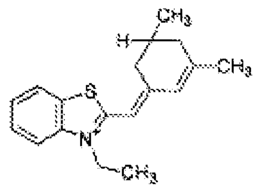 $I^-$ |
| 70 | 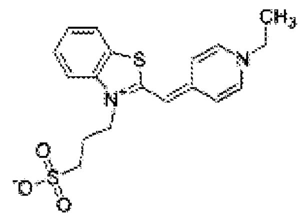 $I^-$ |
| 71 | 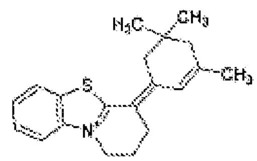 $I^-$ |
| 72 | 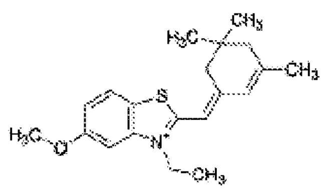 $p$-$CH_3C_6H_4SO_3^-$ |
| 73 | 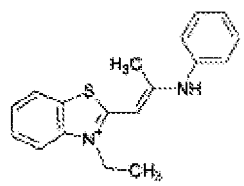 $I^-$ |
| 74 | 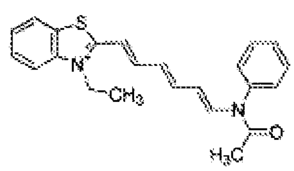 $I^-$ |
| 75 | 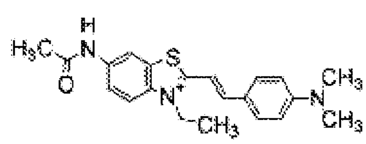 $I^-$ |
| 76 | 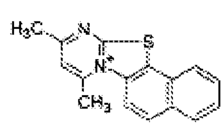 $ClO_4^-$ |

(fortgesetzt)

| Verbindung Nummer | Struktur |
|---|---|
| 77 | 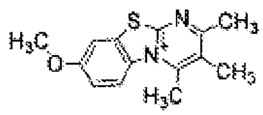 $ClO_4^-$ |
| 78 | 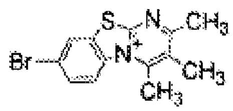 $ClO_4^-$ |
| 79 | 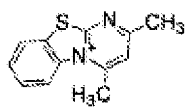 $ClO_4^-$ |
| 80 | 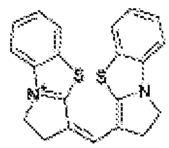 $p$-$CH_3C_6H_4SO_3^-$ |
| 81 | 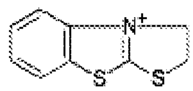 $Br^-$ |
| 82 | 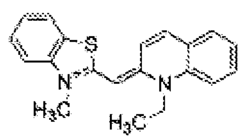 $I^-$ |
| 83 | 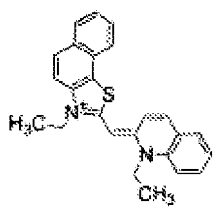 $Cl^-$ |
| 84 | 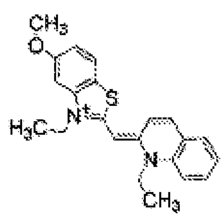 $I^-$ |
| 85 | 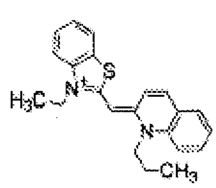 $I^-$ |

(fortgesetzt)

| Verbindung Nummer | Struktur |
|---|---|
| 86 | 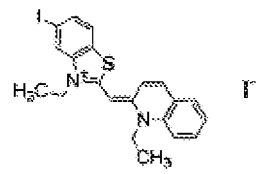 |
| 87 | 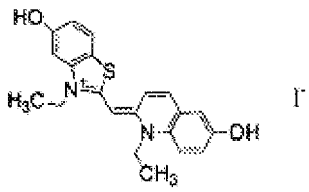 |
| 88 | 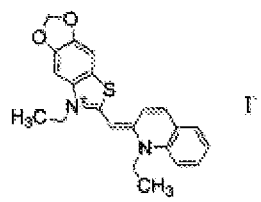 |
| 89 | 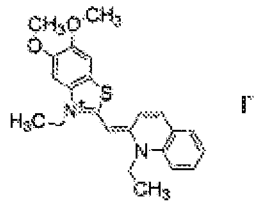 |
| 90 | 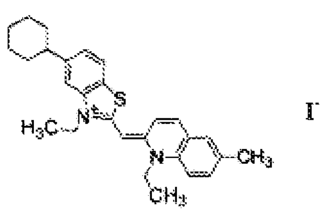 |
| 91 | 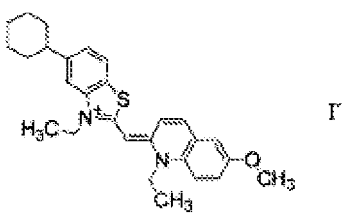 |
| 92 | 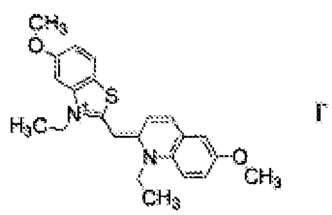 |

(fortgesetzt)

| Verbindung Nummer | Struktur |
|---|---|
| 93 | 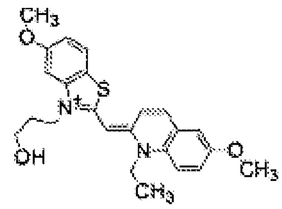 $I^-$ |
| 94 | 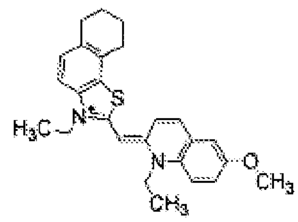 $I^-$ |
| 95 | 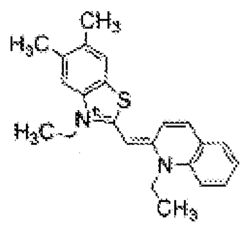 $I^-$ |
| 96 | 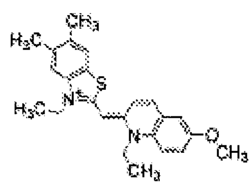 $I^-$ |
| 97 | 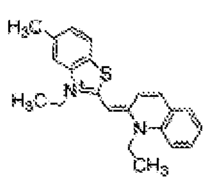 $I^-$ |
| 98 | 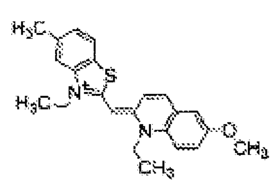 $I^-$ |
| 99 | 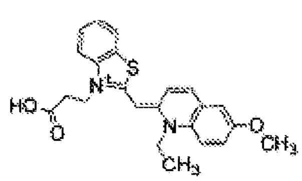 $I^-$ |

(fortgesetzt)

| Verbindung Nummer | Struktur |
|---|---|
| 100 | 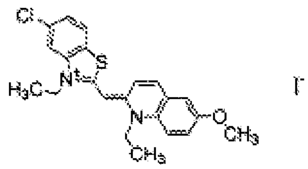 |
| 101 | 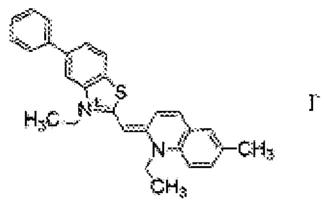 |
| 102 | 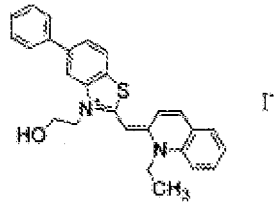 |
| 103 | 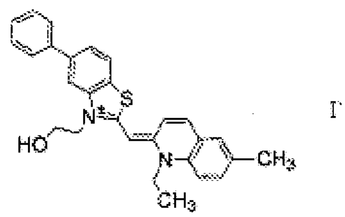 |
| 104 | 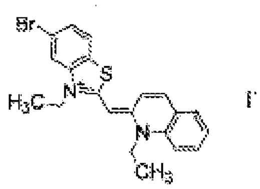 |
| 105 | 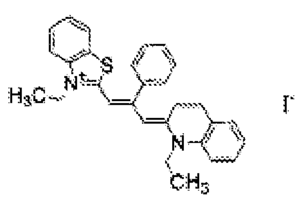 |
| 106 | 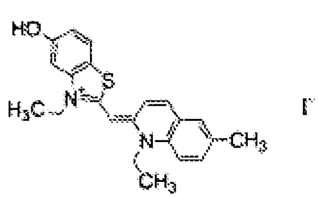 |

(fortgesetzt)

| Verbindung Nummer | Struktur |
|---|---|
| 107 | 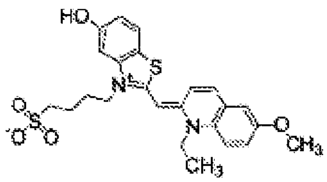 |
| 108 | 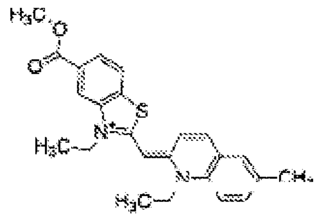 $Br^-$ |
| 109 | 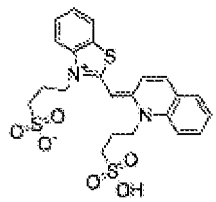 |
| 110 | 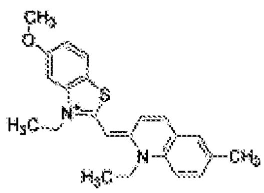 $p$-$CH_3C_6H_4SO_3^-$ |
| 111 | 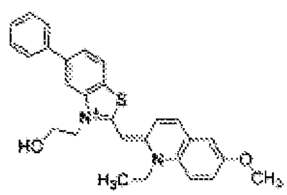 $I^-$ |
| 112 | 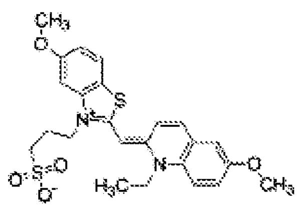 |
| 113 | 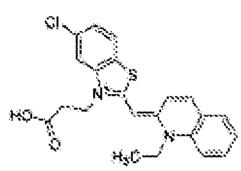 $Cl^-$ |

(fortgesetzt)

| Verbindung Nummer | Struktur |
|---|---|
| 114 | 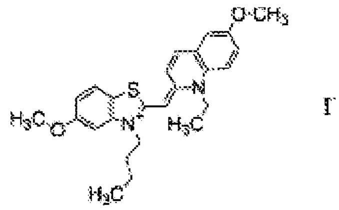 |
| 115 | 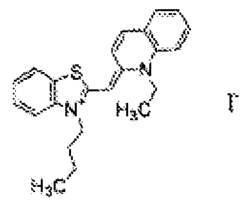 |
| 116 | 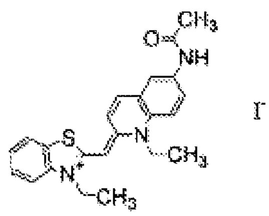 |
| 117 | 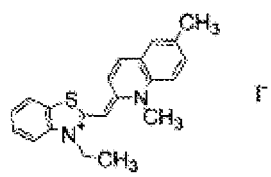 |
| 118 | 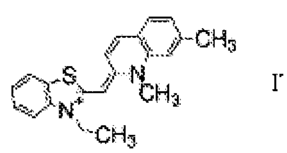 |
| 119 | 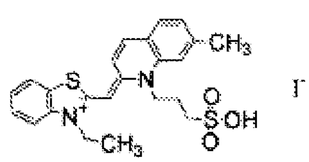 |
| 120 | 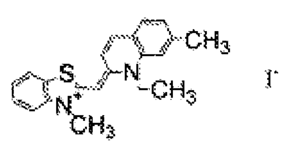 |
| 121 | 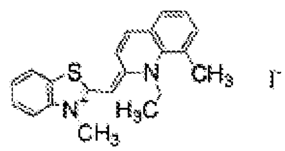 |

(fortgesetzt)

| Verbindung Nummer | Struktur |
|---|---|
| 122 | 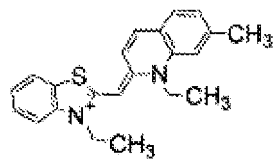 $I^-$ |
| 123 | 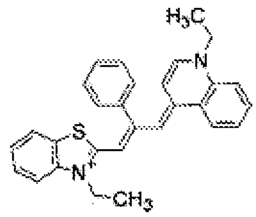 $I^-$ |
| 124 | 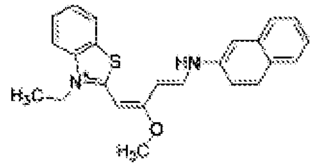 $I^-$ |
| 125 | 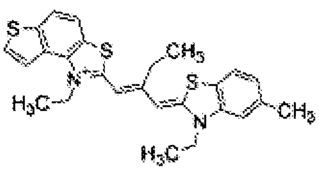 $I^-$ |
| 126 | 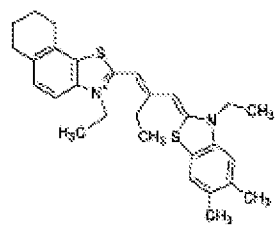 $Br^-$ |
| 127 | 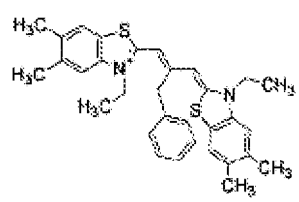 $NCS^-$ |
| 128 | 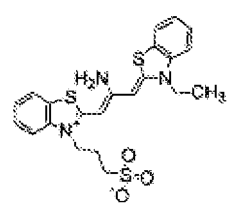 |

(fortgesetzt)

| Verbindung Nummer | Struktur |
|---|---|
| 129 | 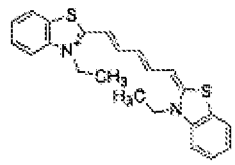 $I^-$ |
| 130 | 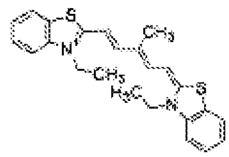 $I^-$ |
| 131 | 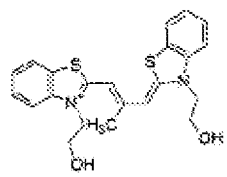 $Cl^-$ |
| 132 | 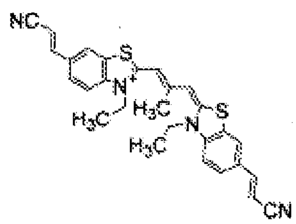 $p\text{-}CH_3C_6H_4SO_3^-$ |
| 133 | 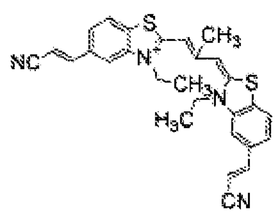 $p\text{-}CH_3C_6H_4SO_3^-$ |
| 134 | 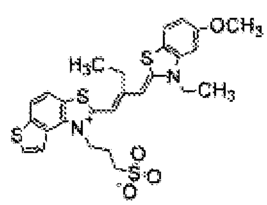 |
| 135 | 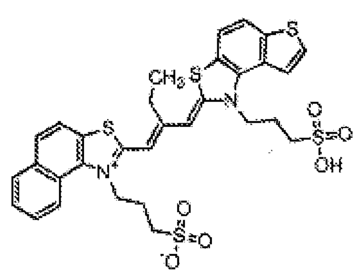 |

(fortgesetzt)

| Verbindung Nummer | Struktur |
|---|---|
| 136 | 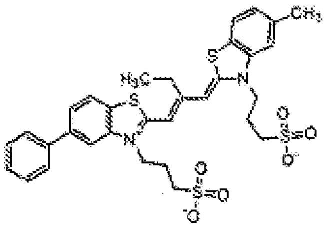 $(C_4H_5)_3NH^+$ |
| 137 | 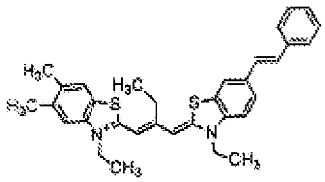 $I^-$ |
| 138 | 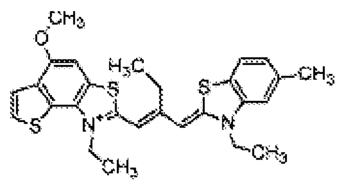 $Br^-$ |
| 139 | 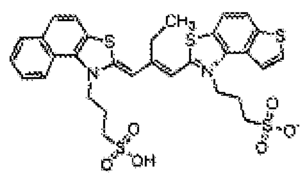 |
| 140 | 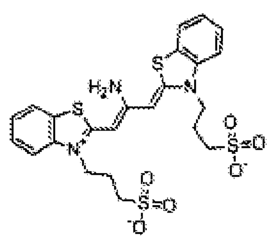 $NH_4^+$ |
| 141 | 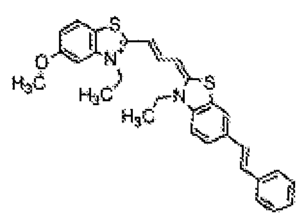 $I^-$ |
| 142 | 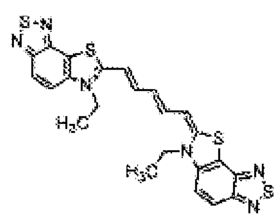 $p\text{-}CH_3C_6H_4SO_3^-$ |

(fortgesetzt)

| Verbindung Nummer | Struktur |
|---|---|
| 143 | 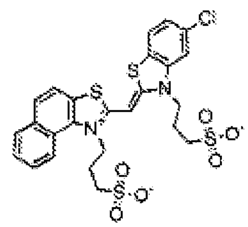 $(C_2H_5)_3NH^+$ |
| 144 | 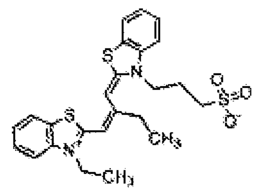 |
| 145 | 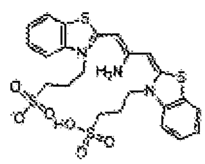 |
| 146 | 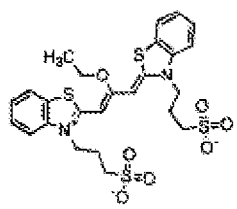 $(C_2H_5)_3NH^+$ |
| 147 | 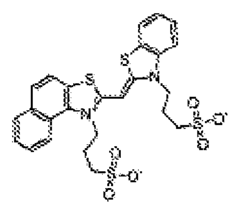 $(C_2H_5)_3NH^+$ |
| 148 | 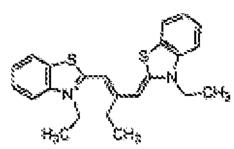 $I^-$ |
| 149 | 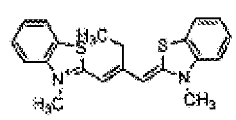 $I^-$ |

(fortgesetzt)

| Verbindung Nummer | Struktur |
|---|---|
| 130 | 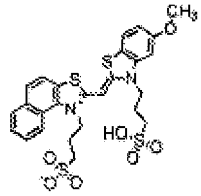 |
| 151 | 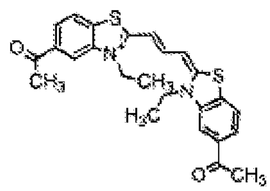 $C_2H_5SO_4^-$ |
| 152 | 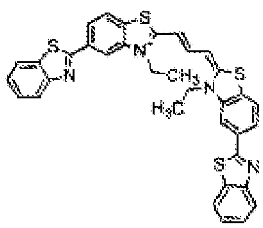 $p$-$CH_3C_6H_4SO_3^-$ |
| 153 | 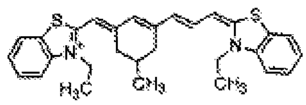 $I^-$ |
| 154 | 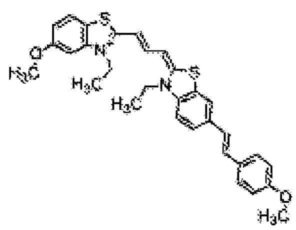 $I^-$ |
| 155 | 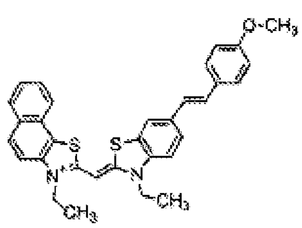 $p$-$CH_3C_6H_4SO_3^-$ |
| 156 | 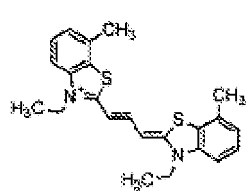 $I^-$ |

(fortgesetzt)

| Verbindung Nummer | Struktur |
|---|---|
| 157 | 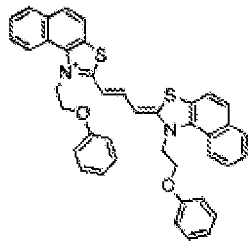 $p$-$CH_3C_6H_4SO_3^-$ |
| 158 | 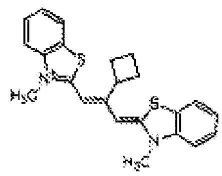 $I^-$ |
| 159 | 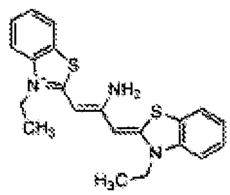 $I^-$ |
| 160 | 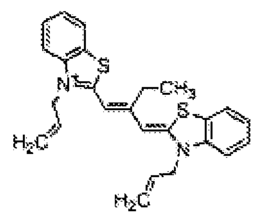 $I^-$ |
| 161 | 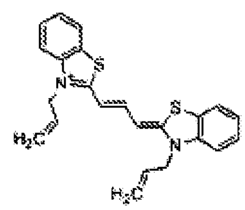 $I^-$ |
| 162 | 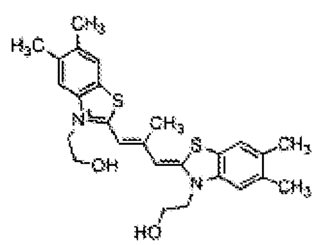 $I^-$ |

(fortgesetzt)

| Verbindung Nummer | Struktur |
|---|---|
| 163 | 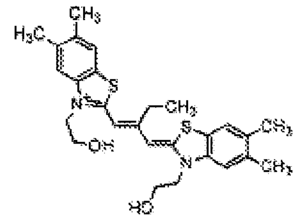 $I^-$ |
| 164 | 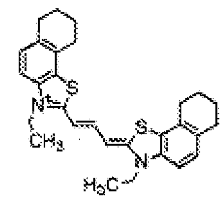 $I^-$ |
| 165 | 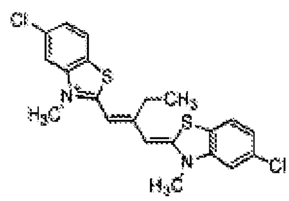 $p$-$CH_3C_6H_4SO_3^-$ |
| 166 | 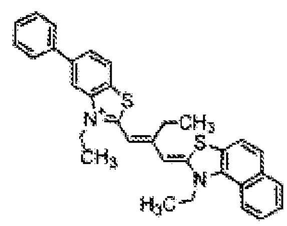 $Br^-$ |
| 167 | 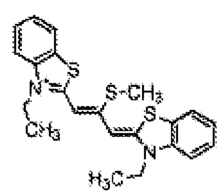 $CH_3SO_4^-$ |
| 168 | 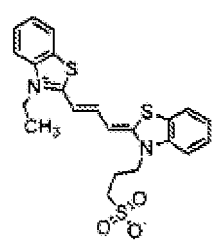 |

(fortgesetzt)

| Verbindung Nummer | Struktur |
|---|---|
| 169 | 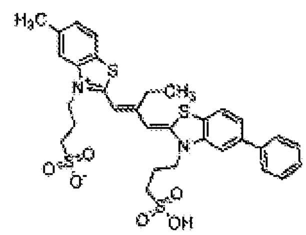 |
| 170 | 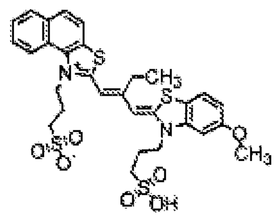 |
| 171 | 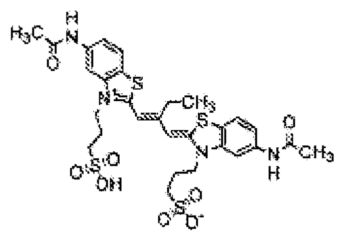 |
| 172 | 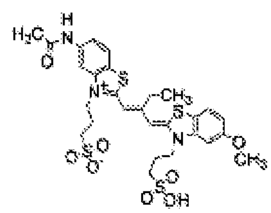 |
| 173 | 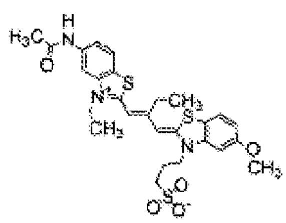 |
| 174 | 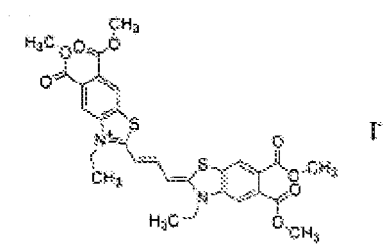 |

(fortgesetzt)

| Verbindung Nummer | Struktur |
|---|---|
| 175 | 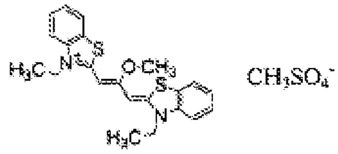 |
| 176 | 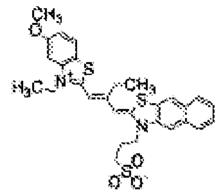 |
| 177 | 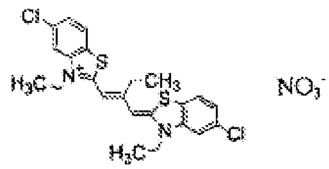 |
| 178 | 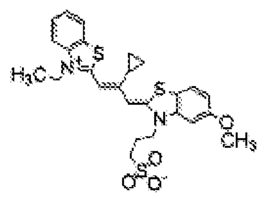 |
| 179 | 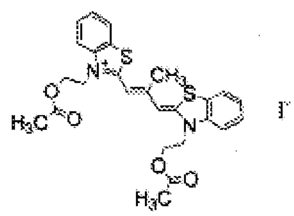 |
| 180 | 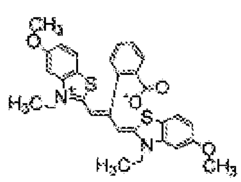 |
| 181 | 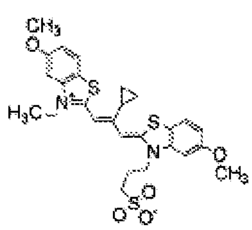 |

(fortgesetzt)

| Verbindung Nummer | Struktur |
|---|---|
| 182 | 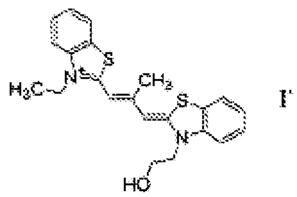 |
| 183 | 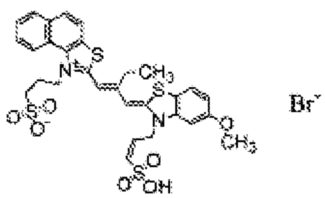 |
| 184 | 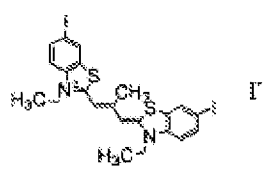 |
| 185 | 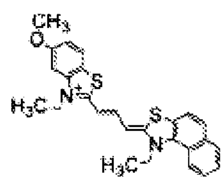 |
| 186 | 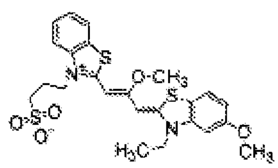 |
| 187 | 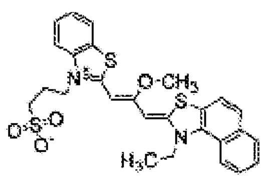 |
| 188 | 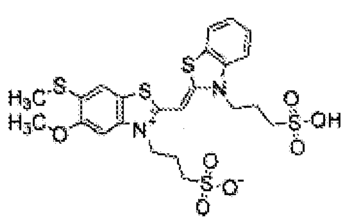 |

(fortgesetzt)

| Verbindung Nummer | Struktur |
|---|---|
| 189 | 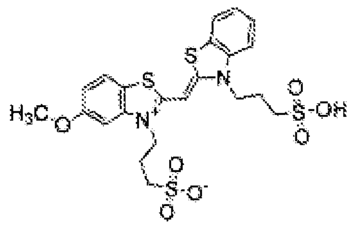 |
| 190 | 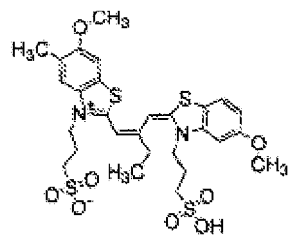 |
| 191 | 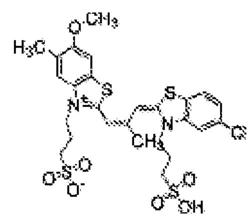 |
| 192 | 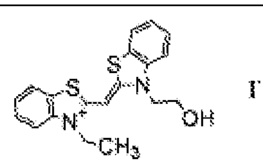 |
| 193 | 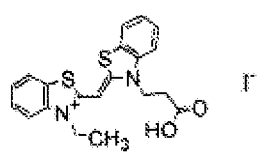 |
| 194 | 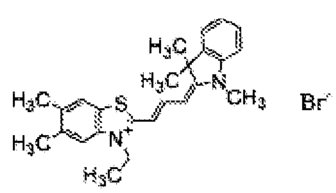 |
| 195 | 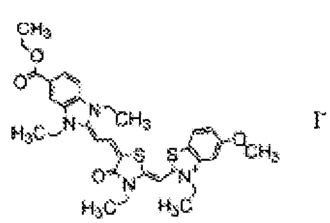 |

(fortgesetzt)

| Verbindung Nummer | Struktur |
|---|---|
| 196 | 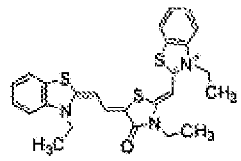 I⁻ |
| 197 | 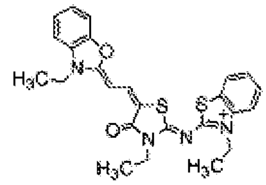 $p\text{-}CH_3C_6H_4SO_3^-$ |
| 198 | 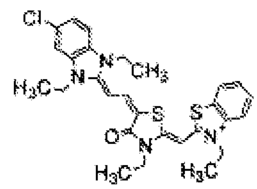 I⁻ |
| 199 | 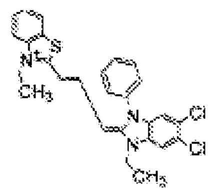 I⁻ |
| 200 | 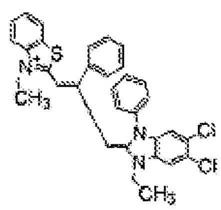 I⁻ |
| 201 | 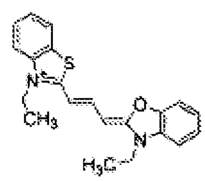 I⁻ |
| 202 | 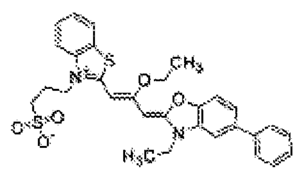 |

(fortgesetzt)

| Verbindung Nummer | Struktur |
|---|---|
| 204 | 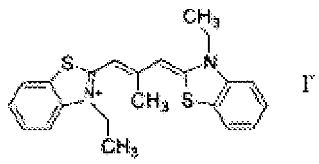 |
| 205 | 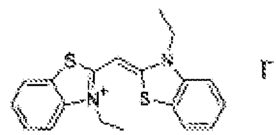 |
| 206 | 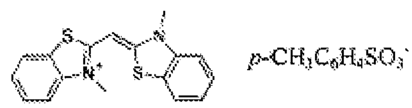 |

**16.** Verbindung nach Anspruch 1, wobei die Verbindung die Produktion von Stickstoffmonoxid durch Unterdrücken einer Expression von induzierbarer Stickstoffmonoxidsynthase inhibiert.

**17.** Verwendung einer wie in einem der Ansprüche 1 - 16 definierten Verbindung bei der Herstellung eines Medikaments zur Behandlung koronaviraler Infektionen.

**Revendications**

**1.** Composé de formule suivante :

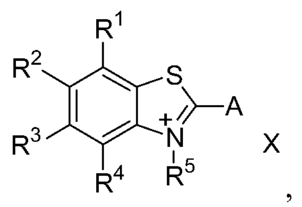

dans laquelle
chacun des $R^1$, $R^2$, $R^3$, et $R^4$, indépendamment, est un H, halo, alkyle, alcényle, alcynyle, alcoxyle, aryle, hétéroaryle, cyclyle, hétérocyclyle, nitro, cyano, amino, amido, ou alcoxycarbonyle, ou $R^1$, $R^2$, et les deux atomes de carbone auxquels ils sont fixés, forment ensemble un cycle à 3-8 éléments contenant 0, 1, 2, ou 3 hétéroatomes ou $R^2$, $R^3$, et les deux atomes de carbone auxquels ils sont fixés, forment ensemble un cycle à 3-8 éléments contenant 0, 1, 2, ou 3 hétéroatomes, ou $R^3$, $R^4$, et les deux atomes de carbone auxquels ils sont fixés, forment ensemble un cycle à 3-8 éléments contenant 0, 1, 2, ou 3 hétéroatomes ;
$R^5$ est un alkyle, aryle, hétéroaryle, cyclyle, ou hétérocyclyle ;
A est un H, alkyle, aryle, hétéroaryle, cyclyle, hétérocyclyle, alcoxy, amino, halo, mercapto, thioalcoxy,

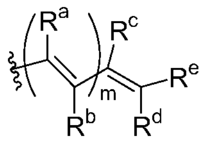

,

ou

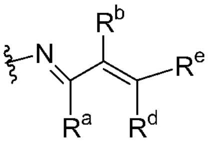

;

où m est égal à 0, 1, 2, ou 3 ; chacun des $R^a$, $R^b$, $R^c$, $R^d$, et $R^e$ est indépendamment un H, un alkyle, alcényle, alcynyle, halo, aryle, hétéroaryle, cyclyle, hétérocyclyle, alcoxy, mercapto, thioalcoxy, amino, amido, ou $R^d$, $R^e$, et l'atome de carbone auquel ils sont fixés, forment ensemble un cycle à 3-8 éléments contenant 0, 1, 2, ou 3 hétéroatomes, qui est éventuellement fusionné avec un aryle, hétéroaryle, cyclyle, ou hétérocyclyle ; éventuellement A, $R^5$ l'atome N auquel $R^5$ est fixé, et l'atome de carbone auquel A est fixé, forment ensemble un cycle à 4-8 éléments contenant 1, 2, ou 3 hétéroatomes ; et
X est un anion ou un groupe chargé négativement fixé à un atome de $R^1$, $R^2$, $R^3$, $R^4$, $R^5$ ou A,
pour utilisation dans le traitement d'infections par coronavirus.

**2.** Composé selon la revendication 1, dans lequel X est un groupe chargé négativement fixé à un atome de $R^1$, $R^2$, $R^3$, $R^4$, $R^5$, ou A, le groupe chargé négativement étant choisi parmi $-CO_2^-$, $-SO_3^-$, ou $-PO_3^{2-}$.

**3.** Composé selon la revendication 1, dans lequel X est un $I^-$, $Br^-$, $Cl^-$, $ClO_4^-$, $C_2H_5SO_4^-$, ou

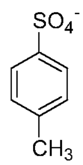

.

**4.** Composé selon la revendication 1, dans lequel A est un H ou un alkyle.

**5.** Composé selon la revendication 4, dans lequel $R^5$ est un alkyle.

**6.** Composé selon la revendication 1, dans lequel $R^5$ est un alkyle.

**7.** Composé selon la revendication 1, dans lequel A est

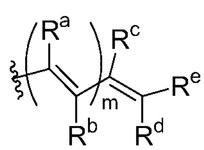

.

**8.** Composé selon la revendication 7, dans lequel un parmi $R^d$ et $R^e$ est un phényle substitué ou non-substitué.

**9.** Composé selon la revendication 7, dans lequel un parmi $R^d$ et $R^e$ est un thioalcoxy, amino, ou amido.

**10.** Composé selon la revendication 7, dans lequel $R^d$, $R^e$, et l'atome de carbone auquel ils sont fixés, forment ensemble

un cycle à 3-8 éléments contenant 0, 1, ou 2 hétéroatomes.

**11.** Composé selon la revendication 10, dans lequel A est

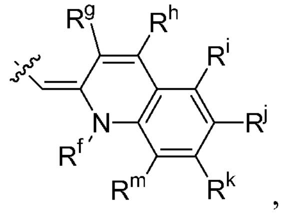

,

$R^f$

étant un H, un alkyle, alcényle, aryle, cyclyle, hétéroaryle, ou hétérocyclyle ; et chacun des $R^g$, $R^h$, $R^i$, $R^j$, $R^k$, et $R^m$, étant indépendamment un H, un alkyle, alcényle, aryle, cyclyle, hétéroaryle, hétérocyclyle, alcoxyle, halogène, nitro, ou cyano.

**12.** Composé selon la revendication 11, dans lequel chacun des $R^f$ et $R^5$ est un alkyle.

**13.** Composé selon la revendication 10, dans lequel A est

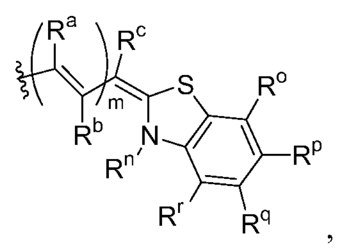

,

$R^n$ étant un H, un alkyle, alcényle, aryle, cyclyle, hétéroaryle, hétérocyclyle, et chacun des $R^o$, $R^p$, $R^q$, et $R^r$, étant indépendamment un H, un alkyle, alcényle, aryle, cyclyle, hétéroaryle, hétérocyclyle, alcoxyle, nitro, amino, cyano, ou halogène.

**14.** Composé selon la revendication 13, dans lequel chacun des $R^n$ et $R^5$ est un alkyle.

**15.** Composé selon la revendication 1, dans lequel le composé est choisi parmi ceux énumérés dans le Tableau ci-dessous :

| Numéro de composé | Structure |
|---|---|
| 1 | 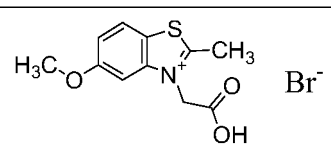 |

(suite)

| Numéro de composé | Structure |
|---|---|
| 2 | 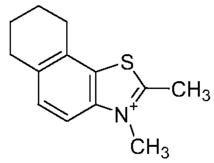 $p$-$CH_3C_6H_4SO_3^-$ |
| 3 | 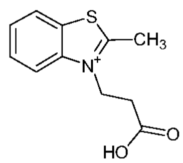 $Br^-$ |
| 4 | 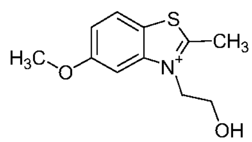 $I^-$ |
| 5 | 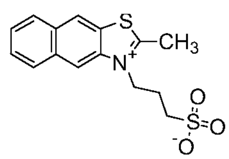 |
| 6 | 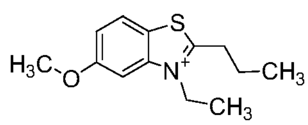 $I^-$ |
| 7 | 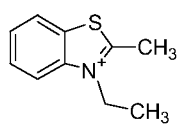 $p$-$CH_3C_6H_4SO_3^-$ |
| 8 | 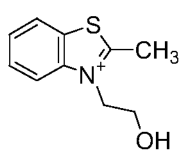 $Cl^-$ |
| 9 | 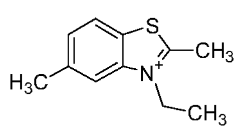 $I^-$ |
| 10 | 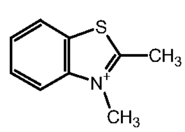 $I^-$ |

(suite)

| Numéro de composé | Structure |
|---|---|
| 11 | 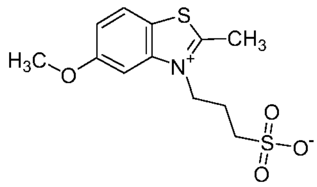 |
| 12 | 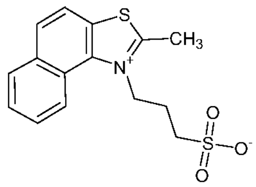 |
| 13 | 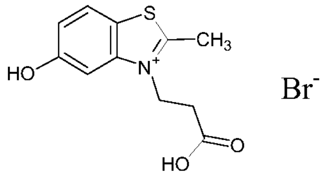 |
| 14 | 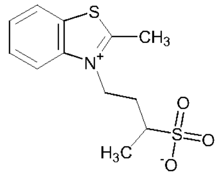 |
| 15 | 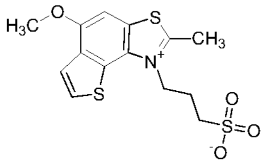 |
| 16 | 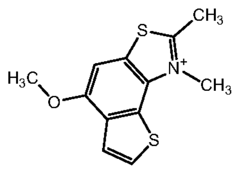 |
| 17 | 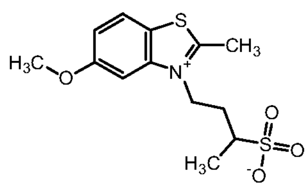 |

(suite)

| Numéro de composé | Structure |
|---|---|
| 18 | 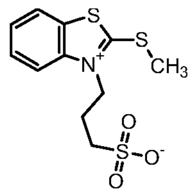 |
| 19 | 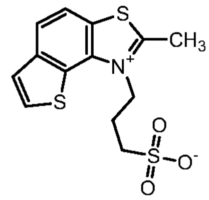 |
| 20 | 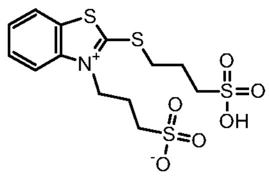 |
| 21 | 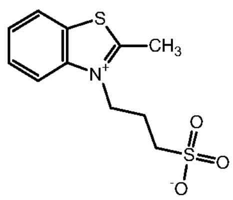 |
| 22 | 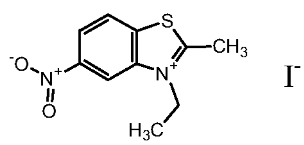 |
| 23 | 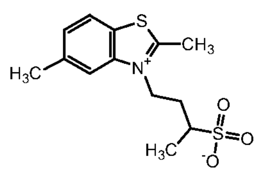 |
| 24 | 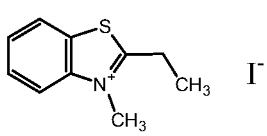 |

(suite)

| Numéro de composé | Structure |
|---|---|
| 25 | 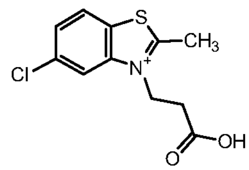 $p$-$CH_3C_6H_4SO_3^-$ |
| 26 | 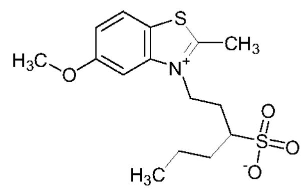 |
| 27 | 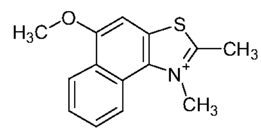 $I^-$ |
| 28 | 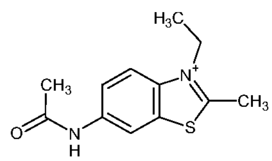 $p$-$CH_3C_6H_4SO_3^-$ |
| 29 | 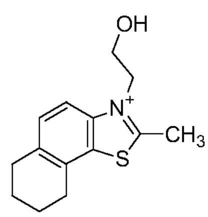 $I^-$ |
| 32 | 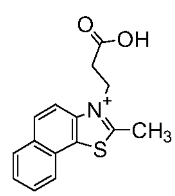 $Br^-$ |
| 33 | 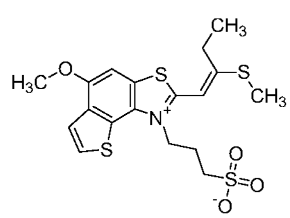 |

(suite)

| Numéro de composé | Structure |
|---|---|
| 34 | 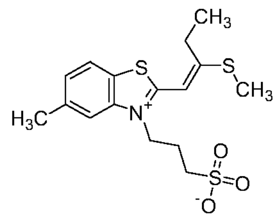 |
| 35 | 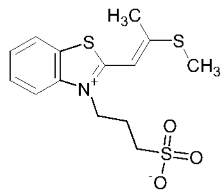 |
| 36 | 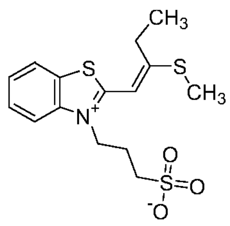 |
| 37 | 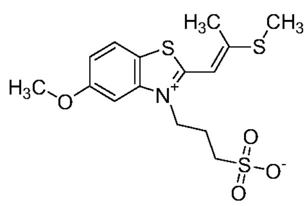 |
| 38 | 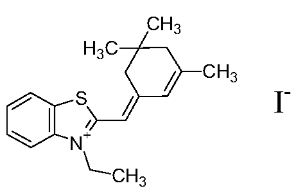 |
| 39 | 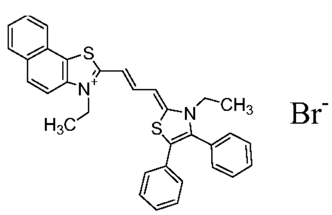 |
| 40 | 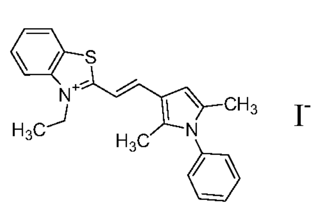 |

(suite)

| Numéro de composé | Structure |
|---|---|
| 41 | 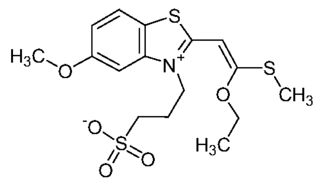 |
| 42 | 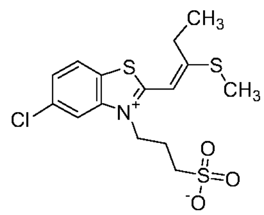 |
| 43 | 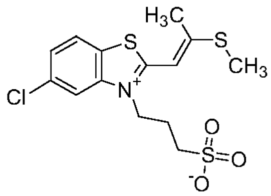 |
| 44 | 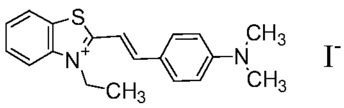 |
| 45 | 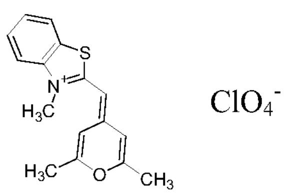 |
| 46 | 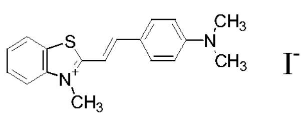 |
| 47 | 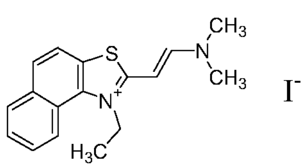 |
| 48 | 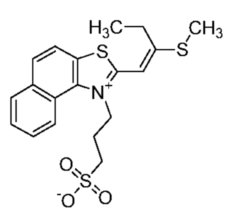 |

(suite)

| Numéro de composé | Structure |
|---|---|
| 49 | 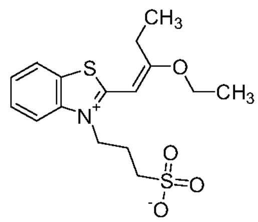 |
| 50 | 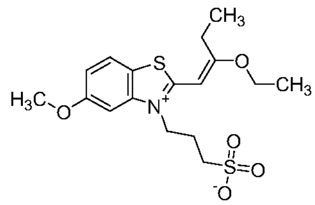 |
| 51 | 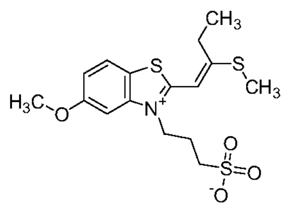 |
| 52 | 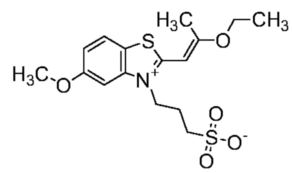 |
| 53 | 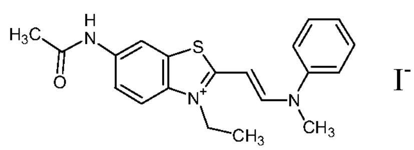 |
| 54 | 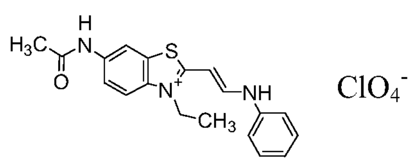 |
| 55 | 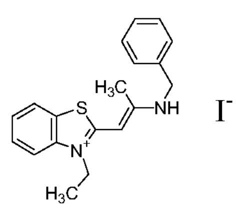 |

(suite)

| Numéro de composé | Structure |
|---|---|
| 56 | 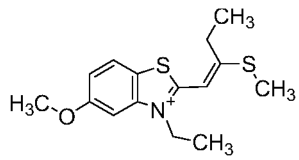 $CH_3SO_4^-$ |
| 57 | 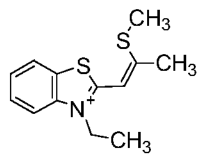 $CH_3SO_4^-$ |
| 58 | 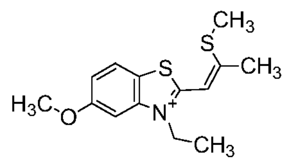 $CH_3SO_4^-$ |
| 59 | 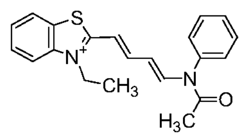 $I^-$ |
| 60 | 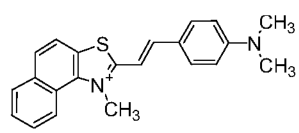 $p$-$CH_3C_6H_4SO_3^-$ |
| 61 | 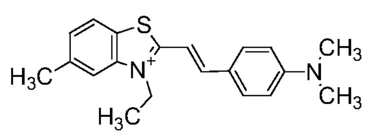 $I^-$ |
| 62 | 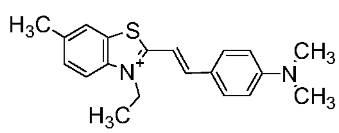 $I^-$ |
| 63 | 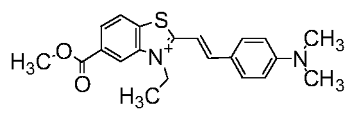 $I^-$ |
| 64 | 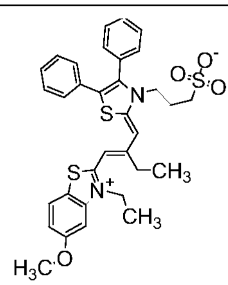 |

(suite)

| Numéro de composé | Structure |
|---|---|
| 65 | 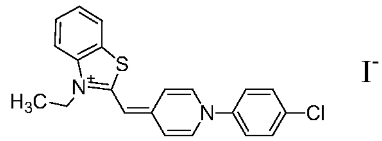 |
| 66 | 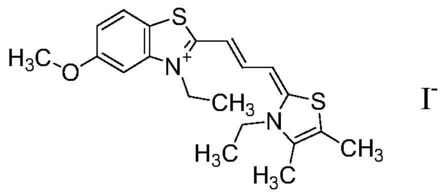 |
| 67 | 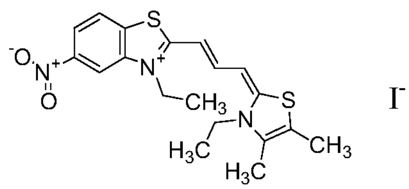 |
| 68 | 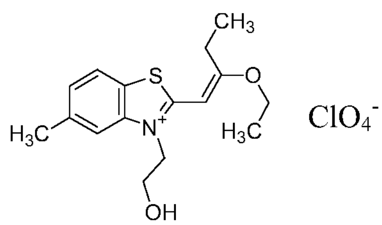 |
| 69 | 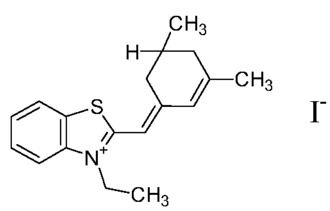 |
| 70 | 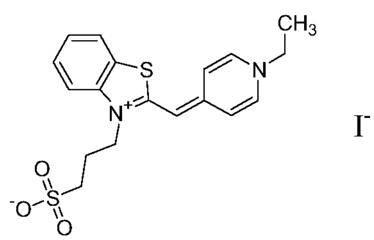 |
| 71 | 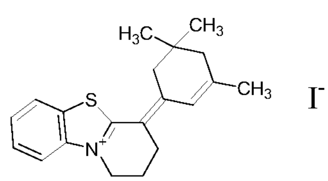 |

(suite)

| Numéro de composé | Structure |
|---|---|
| 72 | 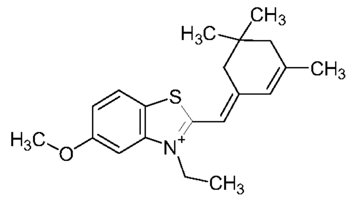 $p$-$CH_3C_6H_4SO_3^-$ |
| 73 | 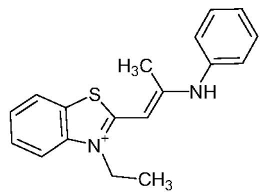 $I^-$ |
| 74 | 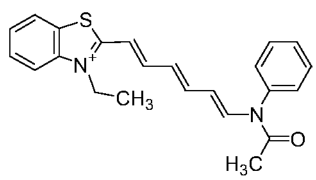 $I^-$ |
| 75 | 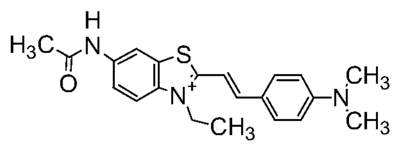 $I^-$ |
| 76 | 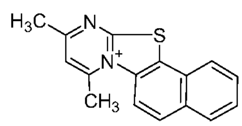 $ClO_4^-$ |
| 77 | 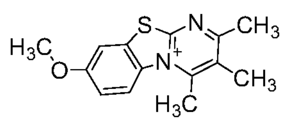 $ClO_4^-$ |
| 78 | 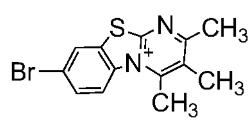 $ClO_4^-$ |
| 79 | 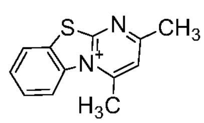 $ClO_4^-$ |
| 80 | 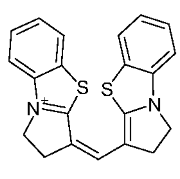 $p$-$CH_3C_6H_4SO_3^-$ |

(suite)

| Numéro de composé | Structure |
|---|---|
| 81 | 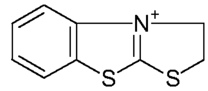 $Br^-$ |
| 82 | 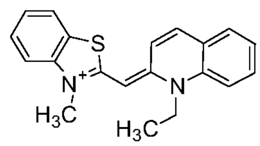 $I^-$ |
| 83 | 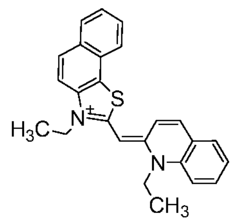 $Cl^-$ |
| 84 | 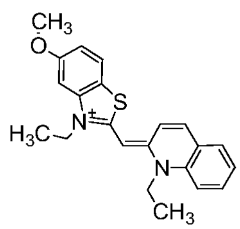 $I^-$ |
| 85 | 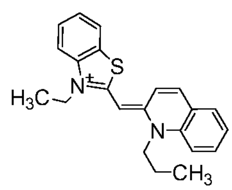 $I^-$ |
| 86 | 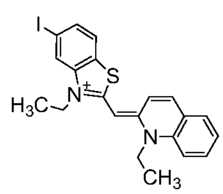 $I^-$ |
| 87 | 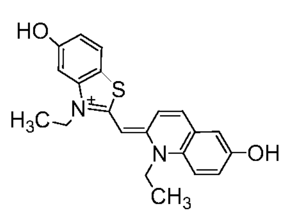 $I^-$ |

(suite)

| Numéro de composé | Structure |
|---|---|
| 88 | 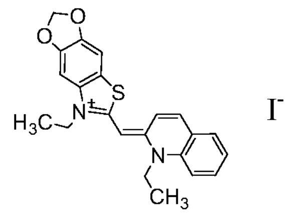 |
| 89 | 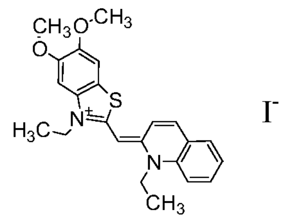 |
| 90 | 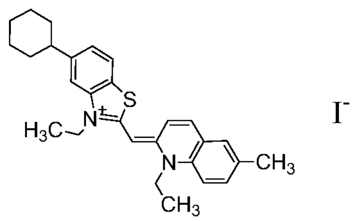 |
| 91 | 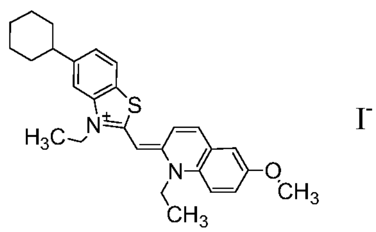 |
| 92 | 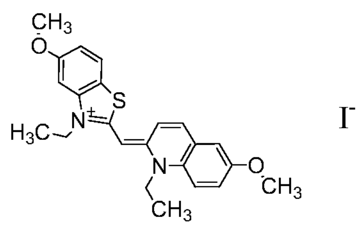 |
| 93 | 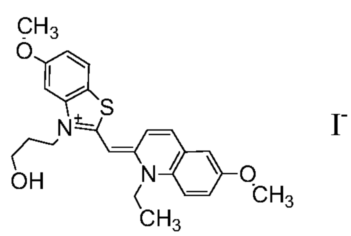 |

(suite)

| Numéro de composé | Structure |
|---|---|
| 94 | 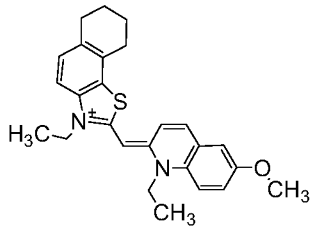 $I^-$ |
| 95 | 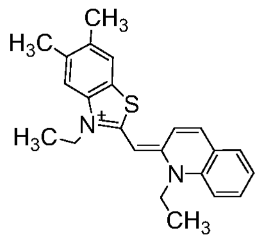 $I^-$ |
| 96 | 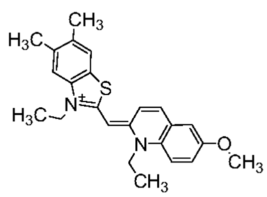 $I^-$ |
| 97 | 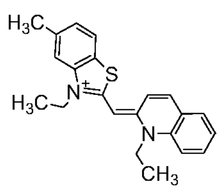 $I^-$ |
| 98 | 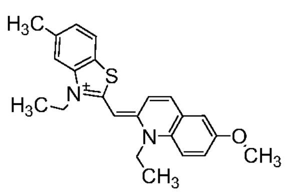 $I^-$ |
| 99 | 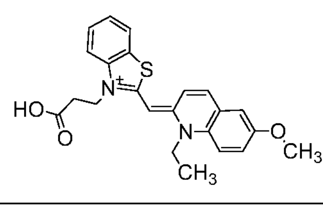 $I^-$ |
| 100 | 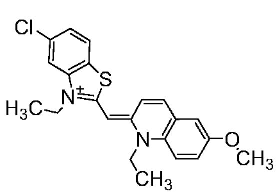 $I^-$ |

(suite)

| Numéro de composé | Structure |
|---|---|
| 101 | 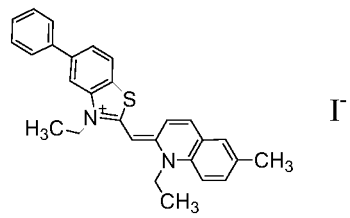 |
| 102 | 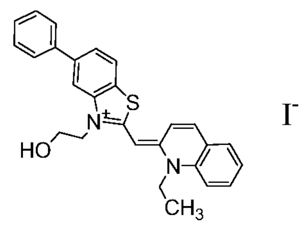 |
| 103 | 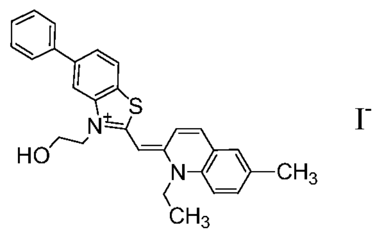 |
| 104 | 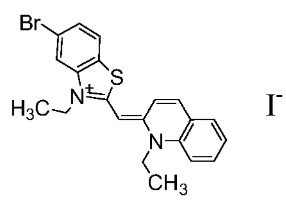 |
| 105 | 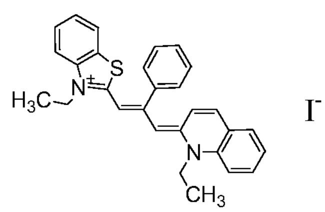 |
| 106 | 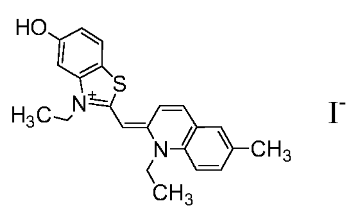 |

(suite)

| Numéro de composé | Structure |
|---|---|
| 107 | 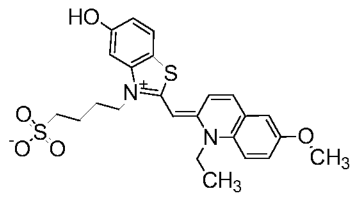 |
| 108 | 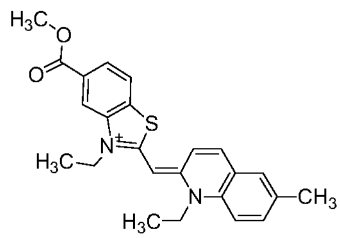 $Br^-$ |
| 109 | 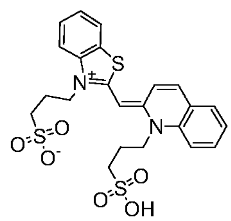 |
| 110 | 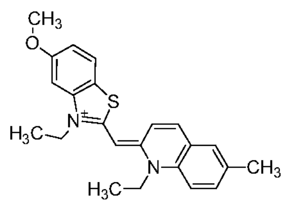 $p\text{-}CH_3C_6H_4SO_3^-$ |
| 111 | 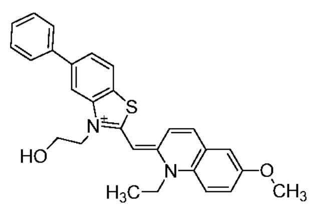 $I^-$ |
| 112 | 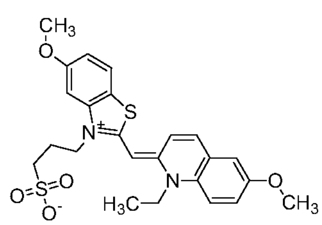 |

(suite)

| Numéro de composé | Structure |
|---|---|
| 113 | 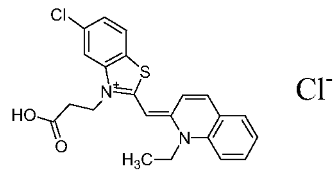 |
| 114 | 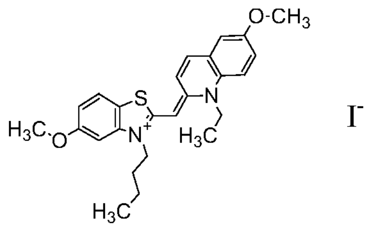 |
| 115 | 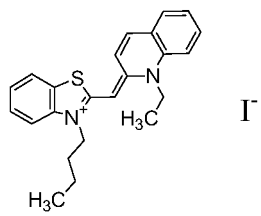 |
| 116 | 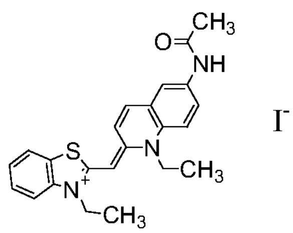 |
| 117 | 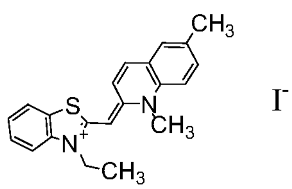 |
| 118 | 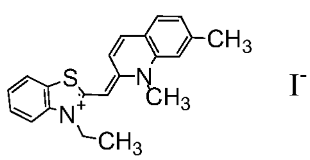 |
| 119 | 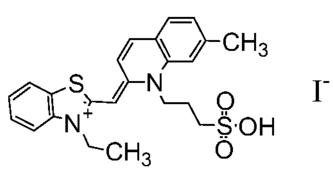 |

(suite)

| Numéro de composé | Structure |
|---|---|
| 120 | 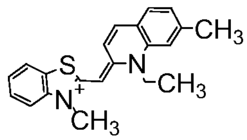 $I^-$ |
| 121 | 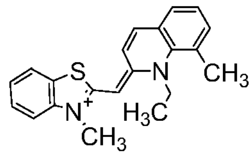 $I^-$ |
| 122 | 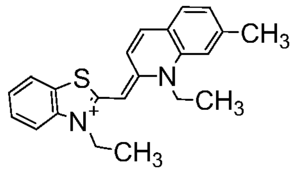 $I^-$ |
| 123 | 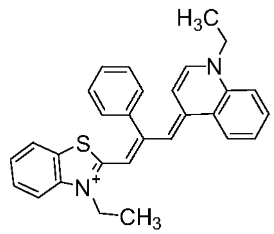 $I^-$ |
| 124 | 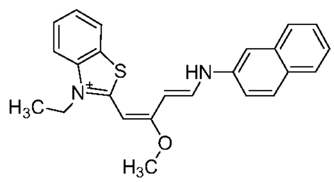 $I^-$ |
| 125 | 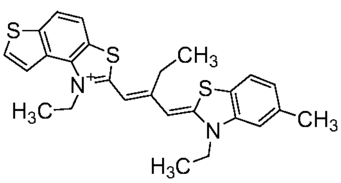 $I^-$ |
| 126 | 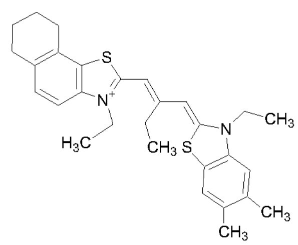 $Br^-$ |

(suite)

| Numéro de composé | Structure |
|---|---|
| 127 | 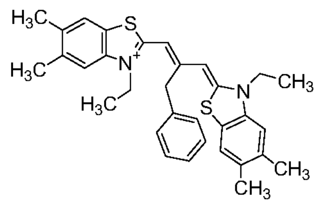 $NCS^-$ |
| 128 | 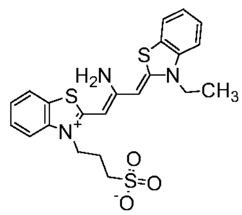 |
| 129 | 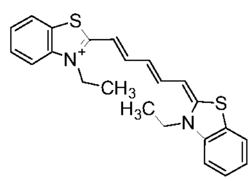 $I^-$ |
| 130 | 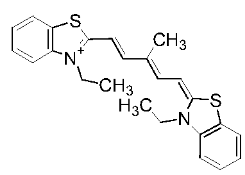 $I^-$ |
| 131 | 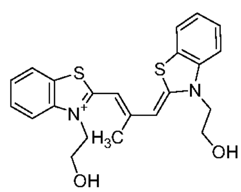 $Cl^-$ |
| 132 | 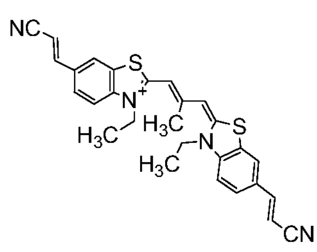 $p$-$CH_3C_6H_4SO_3^-$ |
| 133 | 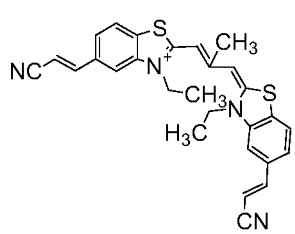 $p$-$CH_3C_6H_4SO_3^-$ |

(suite)

| Numéro de composé | Structure |
|---|---|
| 134 | 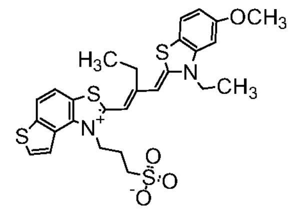 |
| 135 | 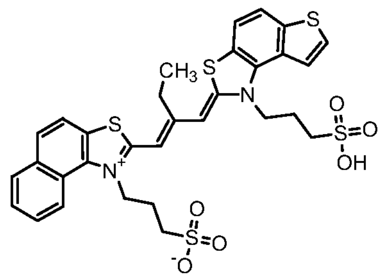 |
| 136 | 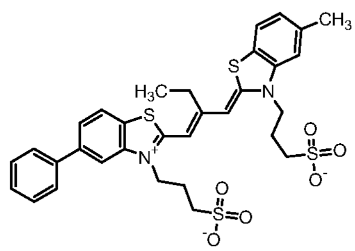 $(C_4H_9)_3NH^+$ |
| 137 | 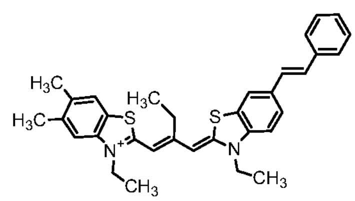 $I^-$ |
| 138 | 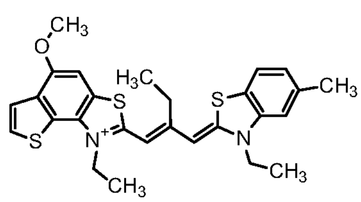 $Br^-$ |
| 139 | 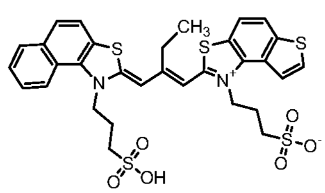 |

(suite)

| Numéro de composé | Structure |
|---|---|
| 140 | 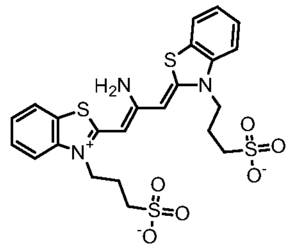 $NH_4^+$ |
| 141 | 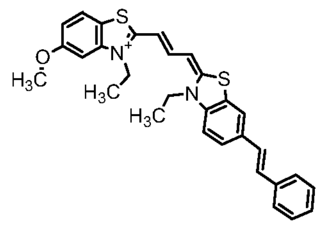 $I^-$ |
| 142 | 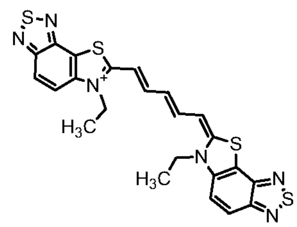 $p$-$CH_3C_6H_4SO_3^-$ |
| 143 | 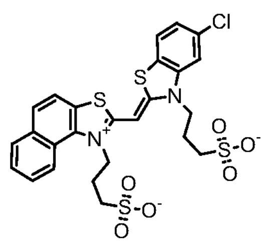 $(C_2H_5)_3NH^+$ |
| 144 | 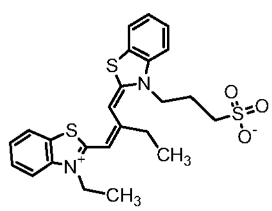 |
| 145 | 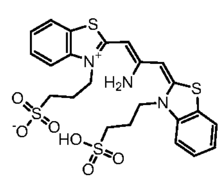 |

(suite)

| Numéro de composé | Structure |
|---|---|
| 146 | 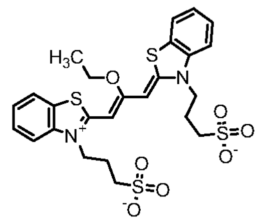 $(C_2H_5)_3NH^+$ |
| 147 | 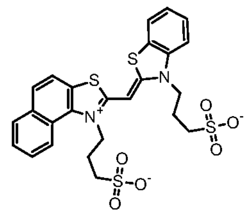 $(C_2H_5)_3NH^+$ |
| 148 | 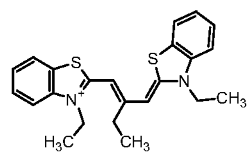 $I^-$ |
| 149 | 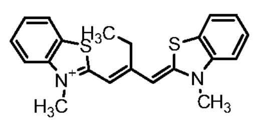 $I^-$ |
| 150 | 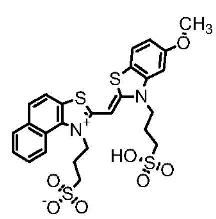 |
| 151 | 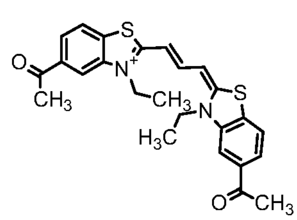 $C_2H_5SO_4^-$ |
| 152 | 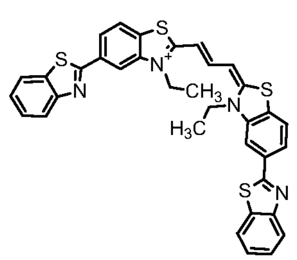 $p$-$CH_3C_6H_4SO_3^-$ |

(suite)

| Numéro de composé | Structure |
| --- | --- |
| 153 | 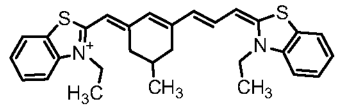 $I^-$ |
| 154 | 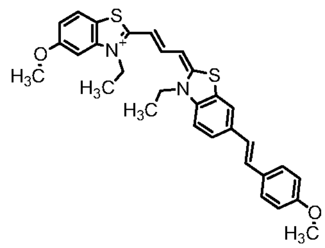 $I^-$ |
| 155 | 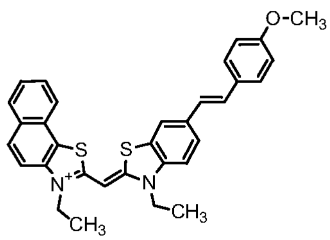 $p$-$CH_3C_6H_4SO_3^-$ |
| 156 | 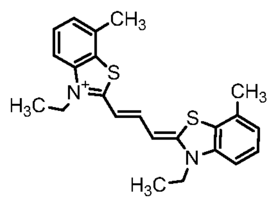 $I^-$ |
| 157 | 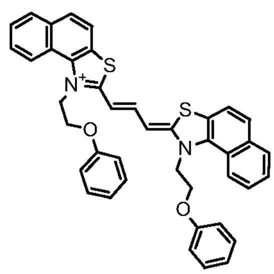 $p$-$CH_3C_6H_4SO_3^-$ |
| 158 | 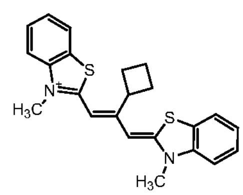 $I^-$ |

(suite)

| Numéro de composé | Structure |
|---|---|
| 159 | 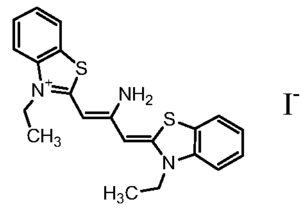 |
| 160 | 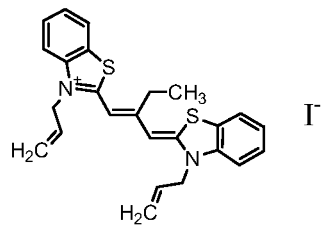 |
| 161 | 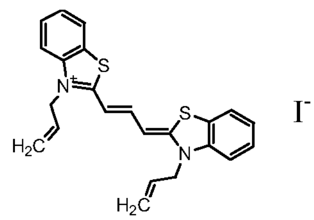 |
| 162 | 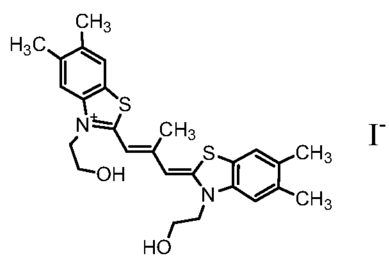 |
| 163 | 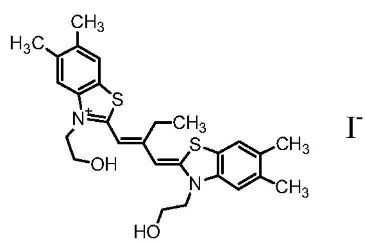 |
| 164 | 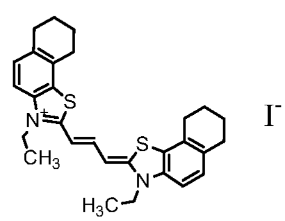 |

(suite)

| Numéro de composé | Structure |
|---|---|
| 165 | 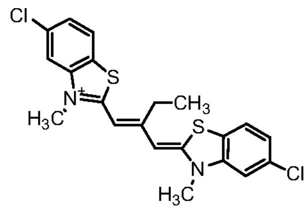 $p\text{-}CH_3C_6H_4SO_3^-$ |
| 166 | 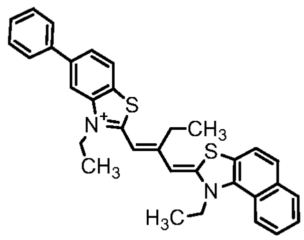 $Br^-$ |
| 167 | 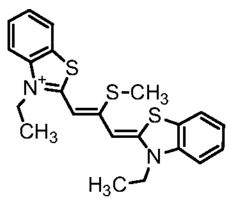 $CH_3SO_4^-$ |
| 168 | 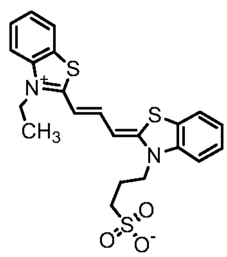 |
| 169 | 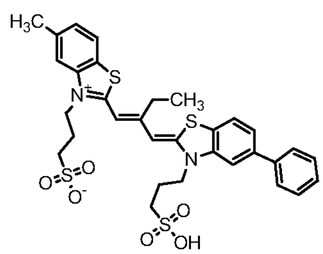 |
| 170 | 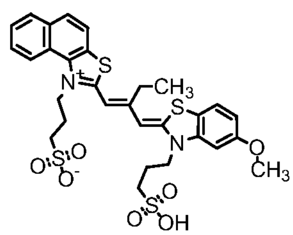 |

(suite)

| Numéro de composé | Structure |
|---|---|
| 171 | 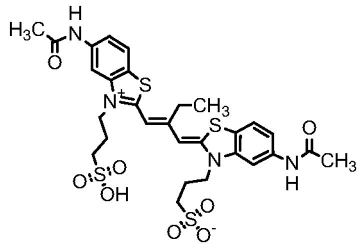 |
| 172 | 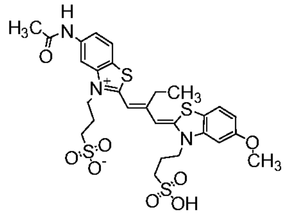 |
| 173 | 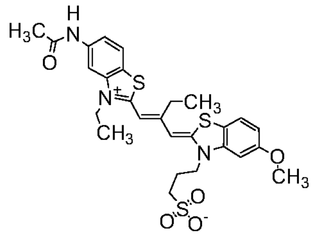 |
| 174 | 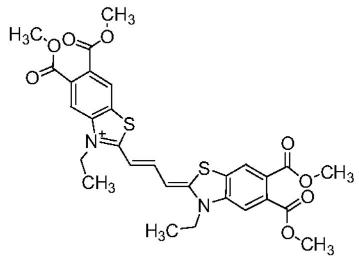 $I^-$ |
| 175 | 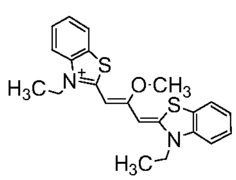 $CH_3SO_4^-$ |
| 176 | 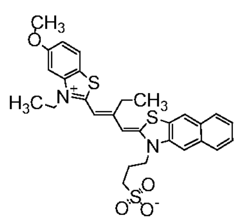 |

(suite)

| Numéro de composé | Structure |
|---|---|
| 177 | 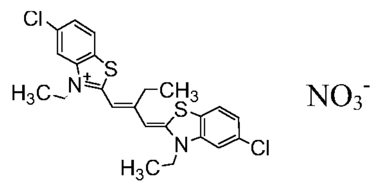 |
| 178 | 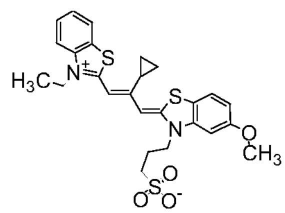 |
| 179 | 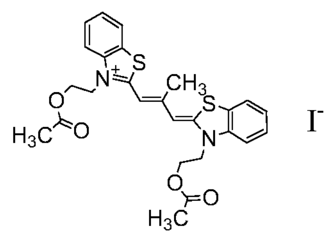 |
| 180 | 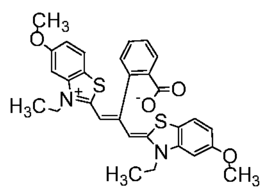 |
| 181 | 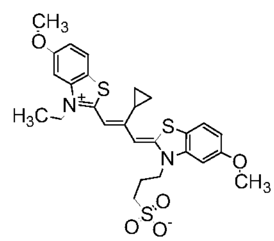 |
| 182 | 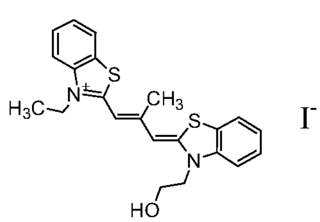 |

(suite)

| Numéro de composé | Structure |
|---|---|
| 183 | 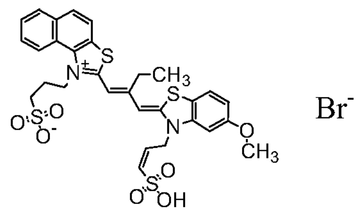 |
| 184 | 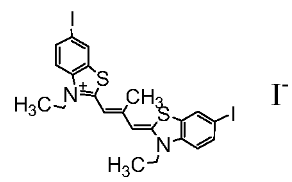 |
| 185 | 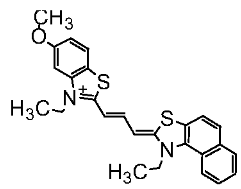 |
| 186 | 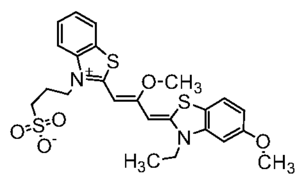 |
| 187 | 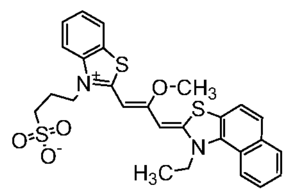 |
| 188 | 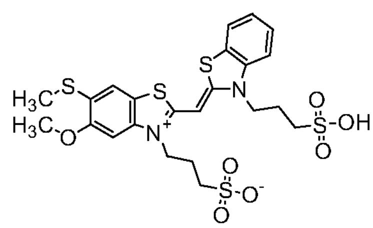 |
| 189 | 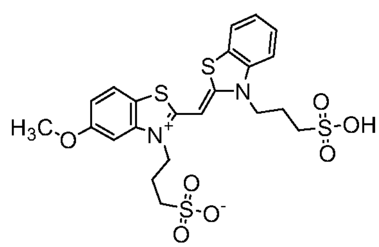 |

(suite)

| Numéro de composé | Structure |
|---|---|
| 190 | 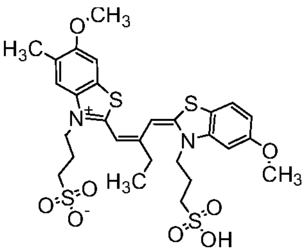 |
| 191 | 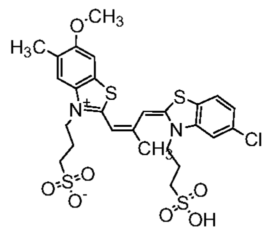 |
| 192 | 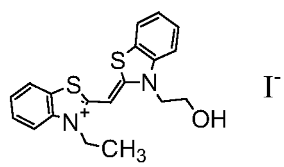 |
| 193 | 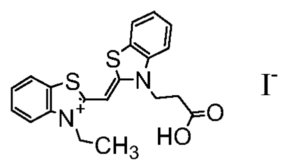 |
| 194 | 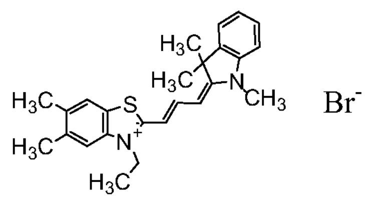 |
| 195 | 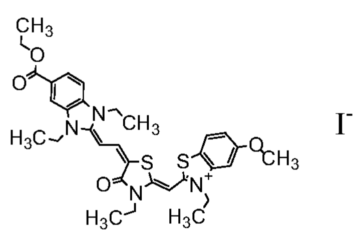 |
| 196 | 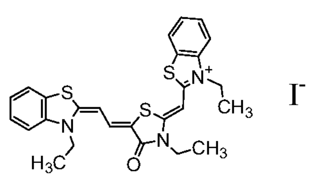 |

(suite)

| Numéro de composé | Structure |
|---|---|
| 197 | 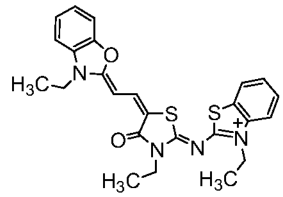 $p$-$CH_3C_6H_4SO_3^-$ |
| 198 | 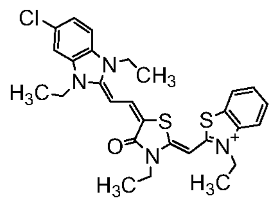 $I^-$ |
| 199 | 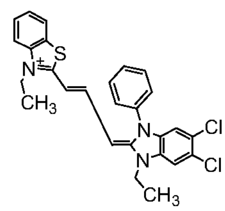 $I^-$ |
| 200 | 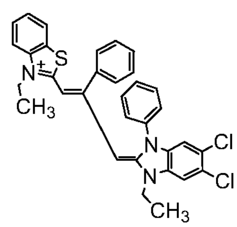 $I^-$ |
| 201 | 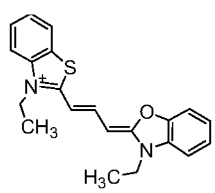 $I^-$ |
| 202 | 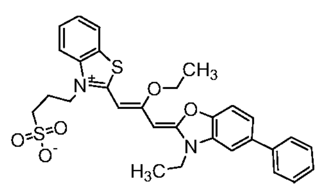 |
| 204 | 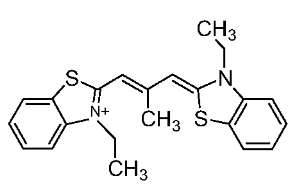 $I^-$ |

(suite)

| Numéro de composé | Structure |
|---|---|
| 205 | 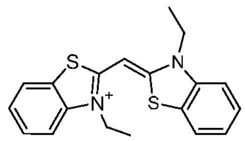 $I^-$ |
| 206 | 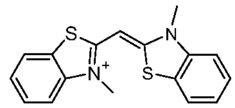 $p$-$CH_3C_6H_4SO_3^-$ |

**16.** Composé selon la revendication 1, dans lequel ledit composé inhibe la production d'oxyde nitrique en supprimant l'expression d'oxyde nitrique synthétase inductible.

**17.** Utilisation d'un composé selon l'une quelconque des revendications 1 à 16 dans la fabrication d'un médicament pour traiter les infections par coronavirus.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0158897 A


### Non-patent literature cited in the description


- **CHEN H-F et al.** DISCOVERY OF ANTI-SARS CORONAVIRUS DRUG BASED ON MOLECULAR DOCKING AND DATABASE SCREENING. *CHINESE JOURNAL OF CHEMISTRY,* 2004, vol. 22 (8), 882-887
- **NG PAK C et al.** Inflammatory cytokine profile in children with severe acute respiratory syndrome. *PEDIATRICS,* January 2004, vol. 113 (1), e7-14
- **R. Larock.** Comprehensive Organic Transformations. VCH Publishers, 1989
- **T.W. Greene ; P.G.M. Wuts.** Protective Groups in Organic Synthesis. John Wiley and Sons, 1999
- **L. Fieser ; M. Fieser.** Fieser and Fieser's Reagents for Organic Synthesis. John Wiley and Sons, 1994
- Encyclopedia of Reagents for Organic Synthesis. John Wiley and Sons, 1995
- **Kuo et al.** *Biochemical and Biophysical Research Communications,* 2004, vol. 318, 862-867